# EUROPEAN PATENT SPECIFICATION

(11) **EP 0 889 881 B1**
(45) Date of publication and mention of the grant of the patent: **23.04.2003**
(21) Application number: 98900110.2
(22) Date of filing: 15.01.1998
(51) Int. Cl.: C07D 249/08, C07D 403/12, C07D 417/12, A61K 31/495, C07D 249/12, C07C 249/14, C07D 257/06, C07D 277/42

(54) **NEW TRIAZOLES AS THERAPEUTIC AGENTS FOR FUNGAL INFECTIONS**
NEUE TRIAZOLE ALS THERAPEUTISCHE MITTEL GEGEN PILZINFEKTIONEN
NOUVEAUX TRIAZOLES, AGENTS THERAPEUTIQUES CONTRE LES MYCOSES

(30) Priority: 17.01.1997 US 786376
(43) Date of publication of application: 13.01.1999
(73) Proprietor: Naeja Pharmaceutical Inc., Edmonton, Alberta T6E 5V2 (CA); TAIHO PHARMACEUTICAL CO., LTD., Tokyo 101-0054 (JP)
(72) Inventor: DANESHTALAB, Mohsen, Edmonton, Alberta T6J 6G2 (CA); BATHINI, Yadagiri, Edmonton, Alberta T6J 6W3 (CA); NGUYEN, Dai, Edmonton, Alberta T5L 6J9 (CA); SIDHU, Inderjit, Edmonton, Alberta T6L 2Y4 (CA); ABEL, Mark, Edmonton, Alberta T6J 1M6 (CA); HA, Chan, Edmonton, Alberta T5P 1A6 (CA); SALAMA, Sameeh, Edmonton, Alberta T6E 2N7 (CA); KHAN, Jehangir, Edmonton, Alberta T6H 4M5 (CA); MICETICH, Ronald, Sherwood Park, Alberta T8A 3V6 (CA); FURUKAWA, Tetsuo, Itano-gun Tokushima-ken 771-15 (JP); UNEMI, Norio, Itano-gun Tokushima-ken 771-02 (JP)
(74) Representative: Walsh, David Patrick
(86) International application number: IB9800046
(87) International publication number: WO98031675

(56) References cited:
- EP-A- 0 321 131
- US-A- 4 738 962
- L. NEUVILLE ET AL.: "Solution phase combinatorial synthesis of arylpiperazines" TETRAHEDRON LETTERS, vol. 38, no. 23, 9 June 1997, OXFORD GB, pages 4091-4094, XP002058738

## Description

### FIELD OF THE INVENTION

The present invention relates to the processes for the preparation of triazole compounds of formula **I**, i.e. 2-aryl-3-(4-substituted piperazin-1-yl)-1-(1H-1,2,4-triazol-1-yl)butan-2-ols, and their use in treating and or preventing the fungal infections in mammals preferably in humans.

### BACKGROUND OF THE INVENTION

Recently the incidence of serious fungal infection has become very prominent in patients undergoing chemotherapy for cancer, organ transplants and patients with AIDS. Most of these infections are caused by opportunistic pathogens like *Candida spp., Aspergillus spp., Pneumocystis carnii* and *Cryptococcus neoformans*. The antifungal agents available in the market suffer with draw backs such as, toxicity, narrow spectrum of activity, fungistatic profile rather fungicidal. Some of them also exhibit drug-drug interactions and, as a result, therapy becomes very complex. In view of the high incidence of fungal infections in immunocompromised patients and the recent trend for the steady increase of the populations of these patients, demands for new antifungal agents with broad spectrum of activity and good pharmacokinetic properties have increased.

Within the available drugs to treat fungal infections, the azole class appears to be more promising. This class of compounds inhibit the biosynthesis of ergosterol in fungi, which is the main constituent of fungal cell membrane. Fluconazole and itraconazole are routinely used for maintenance of fungal infections. Although fluconazole is highly bioavailable, it is not active against filamentous fungi and emergence of fungal resistance has been reported recently (Antimicrob. Agents Chemother. 1995, **39**, 1-8). Itraconazole is active against filamentous fungi, but it shows inconsistent results, maybe due to its high protein binding properties and less bioavailability. During the last few years, several research groups have been actively searching for new azoles with optimum pharmacokinetic properties. As a result, a number of candidate azoles have emerged, and some of them are undergoing preclinical and clinical evaluation. Some of the candidate azoles are disclosed in the following publications:
Sch 51048 (Drugs of the Future, 1995, **20,**241-247).
Sch 56592; Antimicrob. Agents Chemother. (1996, **40,** 1910-1913; 36th Interscience Conference Antimicrob. Agents Chemother. Sept. 1996, New Orleans, Abst. F87-F102).
UK-109,496 (Drugs of the Future, 1996, **21,** 266-271; EP 440372).
TAK-187; 36th Interscience Conference Antimicrob. Agents Chemother. Sept. 1996, New Orleans, Abst. F74; EP 567982).
KP-103 (36th Interscience Conference Antimicrob. Agents Chemother. Sept. 1996, New Orleans, Abst. F78, WO 94126734).
ER-30346 (Drugs of the Future, 1996, **21**, 20-24)

US-A-4738962 discloses azoles as agro-fungicidal agents and EP-A-0321131 discloses azoles as potential antifungal agents but no biological data is disclosed.

In the present invention, we report new triazoles with broad spectrum anti-fungal activity. The triazoles are particularly effective against systemic and lung invasive fungal infections.

### SUMMARY OF THE INVENTION

The present invention relates to new triazole derivatives which can be utilised to treat or prevent fungal infections in animals, preferably in humans.

In accordance to the present invention, there is provided an antifungal triazole of the general formula **I**, i.e. 2-aryl-3-(4-substituted piperazin-1-yl)-1-(1H-1,2,4-triazol-1-yl)-2-ols and pharmaceutically acceptable salts thereof, 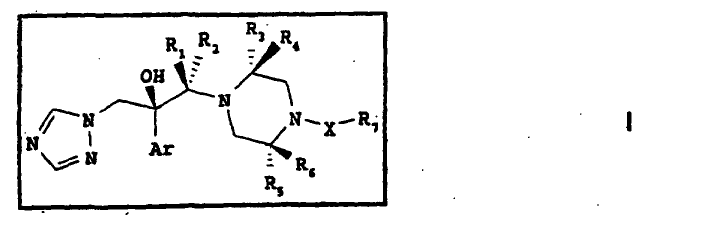 wherein:
Ar is a phenyl group which is unsubstituted or substituted by 1-3 substituents each independently selected from the group consisting of halogen, CF₃ and OCF₃;
R₁ and R₂ are each independently hydrogen or C₁-C₄ alkyl group which is unsubstituted or substituted by 1-3 substituents each independently selected from the group consisting of halogen, hydroxy, C₁-C₄ alkoxy and amino, with the proviso that where R₁ is hydrogen, R₂ is other than hydrogen, and vice versa;
R₃ and R₄ are each independently hydrogen or C₁-C₄ alkyl group which is unsubstituted or substituted by 1-3 substituents each independently selected from the group consisting of halogen, hydroxy, C₁-C₄ alkoxy and amino, or R₃ and R₄ together form =S;
R₅ and R₆ are each independenly hydrogen or C₁-C₄ alkyl group which is unsubstituted or substituted by 1-3 substituents each independently selected from the group consisting of halogen, hydroxy, C₁-C₄ alkoxy and amino, or R₅ and R₆ together form =S;
X is selected from the group consisting of a direct bond, CO, CS, SO₂ and -N=N-;
R₇ is selected from the group consisting of
   (i) phenyl which is unsubstituted or substituted with 1-3 substituents each independently selected from the group consisting of (1) C₁-C₄ alkyl which is unsubstituted or substituted with 1-3 substituents each independently selected from the group consisting of halogen, hydroxy, C₁-C₄ alkoxy and amino, (2) C₁-C₄ alkoxy, (3) halogen, (4) formyl, (5) carboxyl, (6) C₁-C₄ acyloxy, (7) C₁-C₄ alkoxycarbonylamino, (8) phenyl- or naphthyloxycarbonylqmino, (9) semicarbazido, (10) formamido, (11) thioformamido, (12) hydroxy, (13) nitro, (14) amino, (15) furyl, (16) triazolyl, (17) thienyl, (18) oxazolyl, (19) imidazolyl and (20) triazolone-yl,
   (ii) a 5- or 6-membered monocyclic or 8- to 10-membered bicydic heterocycle having 1-4 heteroatoms each independently selected from the group consisting of N. O and S, which heterocycle is unsubstituted or ring-substituted with 1-3 substituents each independently selected from the group consisting of (1) C₁-C₄ alkyl which is unsubstituted or substituted with 1-3 substituents each independently selected from the group consisting of halogen, hydroxy, C₁-C₄ alkoxy and amino, (2) benzyl which is unsubstituted or substituted with 1-3 substituents selected from the group consisting of C,-C₄ alkyl, CF₃, halogen and OCF₃, (3) halogen, (4) hydroxy, (5) nitro, (6) amino, (7) C₁-C₄ acylamino, (8) formyl, (9) formamido, (10) thioformamido, (11) C₁-C₄ alkoxycarbonylamino, (12) phenyl- or naphthyl-oxycarbonylamino and (13) semicarbazido.
   (iii) NHR₈ wherein R₈ is selected from the group consisting of (1) C₁-C₄ alkyl which is unsubstituted or substituted with 1-3 substituents each independently selected from the group consisting of halogen, hydroxy, C₁-C₄ alkoxy and amino, (2) phenyl which is unsubstituted or substituted with 1-3 substituents each independently selected from the group consisting of (a) C₁-C₄ alkyl which is unsubstituted or substituted with 1-3 substituents each independently selected from the group consisting of halogen, hydroxy, C₁-C₄ alkoxy and amino, (b) C₁-C₄ alkoxy, (c) halogen, (d) formyl, (e) carboxyl, (f) C₁-C₄ acyloxy, (g) C₁-C₄ alkoxycarbonylamino, (h) phenyl- or naphthyl-oxycarbonylamino, (i) semicarbazido, (j) formamido, (k) thioformamido, (I) hydroxy, (m) nitro, (n) amino, (o) furyl, (p) triazolyl, (q) thienyl, (r) oxazolyl, (s) imidazolyl and (t) triazolone-yl, and (3) a 5- or 6-membered monocyclic or 8-to 10-membered bicyclic heterocycle having 1-3 heteroatoms each independently selected from the group consisting of N, O and S, which is unsubstituted or substituted with 1-3 substituents each independently selected from the group consisting of hydroxy, halogen, amino and carboxyl,
   (iv) OR₉ wherein R₉ is selected from the group consisting of (1) C₁-C₄ alkyl which is unsubstituted or substituted with 1-3 substituents each independently selected from the group consisting of halogen, hydroxy, C₁-C₄ alkoxy and amino, (2) phenyl which is unsubstituted or substituted with 1-3 substituents each independently selected from the group consisting of (a) C₁-C₄ alkyl which is unsubstituted or substituted with 1-3 substituents each independently selected from the group consisting of halogen, hydroxy, C₁-C₄ alkoxy and amino, (b) C₁-C₄ alkoxy, (c) halogen, (d) formyl, (e) carboxyl, (f) C₁-C₄ acyloxy, (g) C₁-C₄ alkoxycarbonylamino, (h) phenyl- or naphthyl-oxycarbonylamino, (i) semicarbazido, (j) formamido, (k) thioformamido, (I) hydroxy, (m) nitro, (n) amino, (o) furyl, (p) triazolyl, (q) thienyl, (r) oxazolyl, (s) imidazolyl and (t) triazolone-yl and (3) a 5- or 6-membered monocyclic or 8-to 10-membered bicyclic heterocycle having 1-3 heteroatoms each independently selected from the group consisting of N, O and S, which is unsubstituted or substituted with 1-3 substituents each independently selected from the group consisting of (a) C₁-C₄ alkyl which is unsubstituted or substituted with 1-3 substituents each independently selected from the group consisting of halogen, hydroxy, C₁-C₄ alkoxy and amino, (b) phenyl which is unsubstituted or substituted with 1-3 substituents each independently selected from the group consisting of (A) C₁-C₄ alkyl which is unsubstituted or substituted with 1-3 substituents each independently selected from the group consisting of halogen, hydroxy, C₁-C₄ alkoxy and amino, (B) C₁-C₄ alkoxy, (C) halogen, (D) formyl, (E) carboxyl, (F) C₁-C₄ acyloxy, (G) C₁-C₄ alkoxycarbonylamino, (H) phenyl- or naphthyl-oxycarbonylamino, (I) semicarbazido, (J) formamido, (K) thioformamido, (L) hydroxy, (M) nitro, (N) amino, (O) furyl, (P) triazolyl, (Q) thienyl, (R) oxazolyl, (S) imidazolyl and (T) triazolone-yl, (c) naphthyl which is unsubstituted or substituted with 1-3 substituents each independently selected from the group consisting of (A) C₁-C₄ alkyl which is unsubstituted or substituted with 1-3 substituents each independently selected from the group consisting of halogen, hydroxy, C₁-C₄ alkoxy and amino, (B) C₁-C₄ alkoxy, (C) halogen, (D) formyl, (E) carboxyl, (F) C₁-C₄ acyloxy, (G) C₁-C₄ alkoxycarbonylamino, (H) phenyl- or naphthyl-oxycarbonylamino, (I) semicarbazido, (J) formamido, (K) thioformamido, (L) hydroxy, (M) nitro, (N) amino, (O) furyl, (P) triazolyl, (Q) thienyl, (R) oxazolyl, (S) imidazolyl and (T) triazotone-yl, (d) a 5- or 6-membered monocyclic or 8-to 10-membered bicyclic heterocycle having 1-3 heteroatoms each independently selected from the group consisting of N, O and S, (e) (C₁-C₄ alkyl)phenyl, (f) (C₁-C₄ alkyl)naphthyl, (g) hydroxy, (h) halogen, (i) amino and (j) carboxyl, and
   (v) a group of the formula 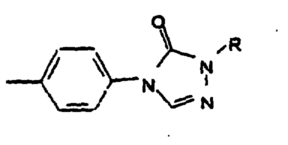 wherein R is selected from the group consisting of (1) hydrogen, (2) C₁-C₁₀ alkyl which is unsubstituted or substituted by 1-5 substituents each independently selected from the group consisting of halogen, hydroxy, C₁-C₄ alkoxy and amino, (3) phenyl which is unsubstituted or substituted with 1-3 substituents each independently selected from the group consisting of (a) C₁-C₄ alkyl which is unsubstituted or substituted with 1-3 substituents each independently selected from the group consisting of halogen, hydroxy, C₁-C₄ alkoxy and amino, (b) C₁-C₄ alkoxy, (c) halogen, (d) forrnyl, (e) carboxyl, (f) C₁-C₄ acyloxy, (g) C₁-C₄ alkoxycarbonylamino, (h) phenyl- or naphthyl-oxycarbonylamino, (i) semicarbazido, (i) formamido, (k) thioformamido, (I) hydroxy, (m) nitro, (n) amino, (o) furyl, (p) triazolyl, (q) thienyl, (r) oxazolyl, (s) imidazolyl, (t) trizolone-yl, (u) CF₃ and (v) OCF₃, (4) a 5- or 6-membered monocyclic or 8- to 10-membered bicyclic heterocycle having 1-3 heteroatoms each independently selected from the group consisting of N, O and S, which is unsubstituted or substituted with 1-3 substituents each independently selected from the group consisting of (a) C₁-C₄ alkyl which is unsubstituted or substituted with 1-3 substituents each independently selected from the group consisting of halogen, hydroxy, C₁-C₄ alkoxy and amino, (b) phenyl which is unsubstituted or substituted with 1-3 substituents each independently selected from the group consisting of (A) C₁-C₄ alkyl which is unsubstituted or substituted with 1-3 substituents each independently selected from the group consisting of halogen, hydroxy, C₁-C₄ alkoxy and amino, (B) C₁-C₄ alkoxy, (C) halogen, (D) formyl, (E) carboxyl, (F) C₁-C₄ acyloxy, (G) C₁-C₄ alkoxycarbonylamino, (H) phenyl- or naphthyl-oxycarbonylamino, (I) semicarbazido, (J) formamido, (K) thioformamido, (L) hydroxy, (M) nitro, (N) amino, (O) furyl, (P) triazolyl, (Q) thienyl, (R) oxazolyl, (S) imidazolyl and (T) triazolone-yl, (c) naphthyl which is unsubstituted or substituted with 1-3 substituents each independently selected from the group consisting of (A) C₁-C₄ alkyl which is unsubstituted or substituted with 1-3 substituents each independently selected from the group consisting of halogen, hydroxy, C₁-C₄ alkoxy and amino, (B) C₁-C₄ alkoxy, (C) halogen, (D) formyl, (E) carboxyl, (F) C₁-C₄ acytoxy, (G) C₁-C₄ alkoxycarbonylamino, (H) phenyl- or naphthyl-oxycarbonylamino, (I) semicarbazido, (J) formamido, (K) thioformamido, (L) hydroxy, (M) nitro, (N) amino, (O) furyl, (P) triazolyl, (Q) thienyl, (R) oxazolyl, (S) imidazolyl and (T) triazolone-yl, (d) a 5- or 6-membered monocyclic or 8- to 10-membered bicyclic heterocycle having 1-3 heteroatoms each independently selected from the group consisting of N, O and S, (e) (C₁-C₄ alkyl)phenyl, (f) (C₁-C₄ alkyl)naphthyl, (g) hydroxy, (h) halogen, (i) amino and (j) carboxyl, (5) phenyl(C₁-C₄ alkyl) which is unsubstituted or ring-substituted with 1-3 substituents each independently selected from the group consisting of (a) C₁-C₅ alkyl which is unsubstituted or substituted with 1-3 substituents each independently selected from the group consisting of halogen, hydroxy, C₁-C₄ alkoxy and amino, (b) halogen, (c) halo(C₁-C₄ alkyl), (d) C₁-C₄ alkoxy, (e) hydroxy, (f) amino, (g) carboxyl, (h) trifluormethoxyl, (i) trifluoromethyl, (j) tetrafluoroethyl, (k) tetrafluoroethoxyl, (l) tetrafluoropropyl and (m) tetrafluoropropoxyl, (6) naphthyl(C₁-C₄ alkyl) which may be substituted with 1-6 substituents selected from (a) C₁-C₅ alkyl which is unsubstituted or substituted with 1-3 substituents each independently selected from the group consisting of halogen, hydroxy, C₁-C₄ alkoxy and amino, (b) halogen, (c) (C₁-C₄ alkyl)halo, (d) C₁-C₄ alkoxy, (e) hydroxy, (f) amino, (g) carboxyl, (h) trifluormethoxyl, (i) trifluoromethyl, (j) tetrafluoroethyl, (k) tetrafluoroethoxyl, (I) tetrafluoropropyl and (m) tetrafluoropropoxyl, (7) methoxyl, (8) trifluormethoxyl, (9) trifluoromethyl, (10) trifluoroethyl, (11) tetrafluoroethyl, (12) tetrafluoroethoxyl, (13) tetrafluoropropyl and (14) tetrafluoropropoxyl.

### DESCRIPTION OF THE PREFERRED EMBODIMENTS

In the above definition of formula **I**, halogen is fluorine, chlorine, bromine or iodine. Preferred halogens are fluorine and chlorine.

C₁-C₄ alkyl is, for example, methyl, ethyl, propyl, 1-methylethyl, n-butyl, 1-methylethyl, isopropyl, 1,1-dimethylethyl, 1-methylpropyl, 2-methylpropyl, cyclopropyl and cyclobutyl. Preferred alkyls are methyl and ethyl.

Examples of **Ar** groups include, 4-fluorophenyl, 2-4-difluorophenyl, 2,4,6-trifluorophenyl, 4-chlorophenyl, 2,4-dichlorophenyl, 2,4,6-trichlorophenyl, 4-trifluoromethylphenyl and 4-trifluoromethoxyphenyl. Preferred **Ar** groups are phenyl group having 1 or 2 substituents each independently selected from fluorine, chlorine, trifluoromethyl and trifluromethoxy. Most preferably, **Ar** is 2,4-difluorophenyl, 2,4-dichlorophenyl, 4-trifluromethylphenyl or 4-trifluoromethoxyphenyl.

Examples of 5-or 6-membered monocyclic rings are 1-pyrrolyl, 2-pyrrolyl, 3-pyrrolyl, 2-furyl, 3-furyl, 2-thienyl, 3-thienyl, 1-imidazolyl, 4-imidazolyl, 1-pyrazolyl, 3-pyrazolyl, 4-pyrazolyl, 5-pyrazolyl, 1-(1H)-1,2,4-triazolyl, 3-(1H)-1,2,4-triazolyl, 3-(4H)-1,2,4-triazolyl, 5-(1H)-1,2,4-triazolyl, 4-(4H)-1,2,4-triazolyl, 1,2,3-triazolyl, 2-oxazolyl, 4-oxazolyl, 5-oxazolyl, 2-thiazolyl, 4-thiazolyl, 5-thiazolyl, 1,2,4-oxadiazol-3-yl, 1,2,4-oxadiazol-5-yl, 1,3,4-oxadiazol-2-yl, 1,2,4-thiadiazol-3-yl, 1,2,4-thiadiazol-5-yl, 1,3,4-thiadiazol-2-yl, 5-(1H)-tetrazolyl, 5-(2H)-tetrazolyl, 2-pyridyl, 3-pyridyl, 4-pyridyl, 2-pyrimidyl, 4-pyrimidyl and 5-pyrimidyl.

Examples of 8- to 10-membered bicyclic heterocycle groups are 2-benzimidazolyl, 5-benzimidazolyl, 2-benzoxazolyl, 5-benzoxazolyl, 6-benzoxazolyl, 2-benzothiazolyl, 5-benzothiazolyl, 6-benzothiazolyl, imidazolo[4,5-b]pyridin-2-yl, imidazolo[4,5-b]pyridin-5-yl, oxazolo[5,4-b]pyridin-2-yl, oxazolo[5,4-b]pyridin-5-yl, oxazolo[5,4-b]pyridin-6-yl, thiazolo[5,4-b]pyridin-2-yl, thiazolo[5,4-b]pyridin-5-yl and thiazolo[5,4-b]pyridin-6-yl.

Triazolone-yl may be 2- or 5-substutitued 1,2,4-triazol-3-one, such as 2H-1,2,4-triazol-3-one-4-yl or 4H-1,2,4-triazol-3-one-2-yl.

Examples of **R**₁ include hydrogen, methyl, ethyl, propyl, hydroxymethyl, alkoxymethyl, fluoromethyl and trifluoromethyl. Preferably, **R**₁ is alkyl. Most preferably, **R**₁ is methyl.

Examples of **R**₂ include hydrogen, methyl, ethyl, propyl, hydroxymethyl, alkoxymethyl, fluromethyl and trifluoromethyl. Preferably, **R**₂ is hydrogen.

Depending on the substituents in formula **I**, the compound may have one or more than one asymmetric centers, resulting in possible stereoisomers. This invention relates to single individual isomers as well as mixture of isomers.

When **R**_{**1**} **and R**_{**2**} are the same, formula **I** has one asymmetric center and there are two possible isomers, i.e. 2R and 2S isomers. This invention relates to mixtures as well as individual isomers. The most preferable isomer is the 2R isomer.

When **R**_{**1**} **and R**_{**2**} are different, formula **I** has two asymmetric centers, and there are four possible isomers, i.e. 2R,3R; 2R,3S; 2S,3R and 2S,3S. This invention relates to the mixture of isomers as well as individual isomers. The most preferred isomer in this situation is 2R,3R.

It is more preferred that **R**_{**7**} is a group 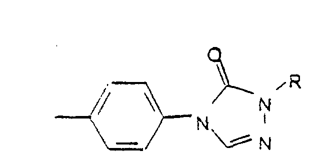 wherein **R** is as described above, and most preferred that the other groups additionally are as follows: **Ar** is 2,4-difluorophenyl, **R**_{**1**} is methyl, and **R**_{**2**} **through R**_{**6**} are each hydrogen.

Specifically, the more preferred embodiments of the present invention include the compounds that are disclosed in Table 3. Those disclosed in Example Nos. 36-51 are more preferred, with Example Nos. 43, 47, 50 and 51 being the most preferred.

The compounds of formula **I** can be prepared by a convergent approach from the epoxide of formula **II** and the piperazino compound of formula **III** (see **Scheme 1**). The epoxides were prepared following the synthetic routes described in the literature (Chem. Pharm. Bull., 1993, **41**, 1035-1042).

The synthetic routes for the preparation of certain piperazino compounds of the formula **III** are disclosed in **Schemes 4-6** and **8**. The **Scheme 7** describes a linear synthetic route for preparing certain compounds of formula **I.** The epoxide of formula **II** and the piperazino compound of formula **III** were reacted in the presence of base such as sodium carbonate, potassium carbonate, cesium carbonate and the like. This reaction was also performed in the presence of lithium perchlorate and sodium perchlorate. The suitable solvents were chosen from acetonitrile, DMF, DMSO, THF, dichloromethane, chloroform, methanol, ethanol, isopropanol and *tert*-butanol. The most preferred solvents were DMF and acetonitrile. The temperature of the reaction varied from 60-180 °C, depending on the solvent and reactants. The most preferred temperature was 80-140 °C. The reactants were allowed to react to the completion or near to the completion of the reaction. The length of reaction time varied from few hours to several hours depending on the reactants, temperature, and the solvent.

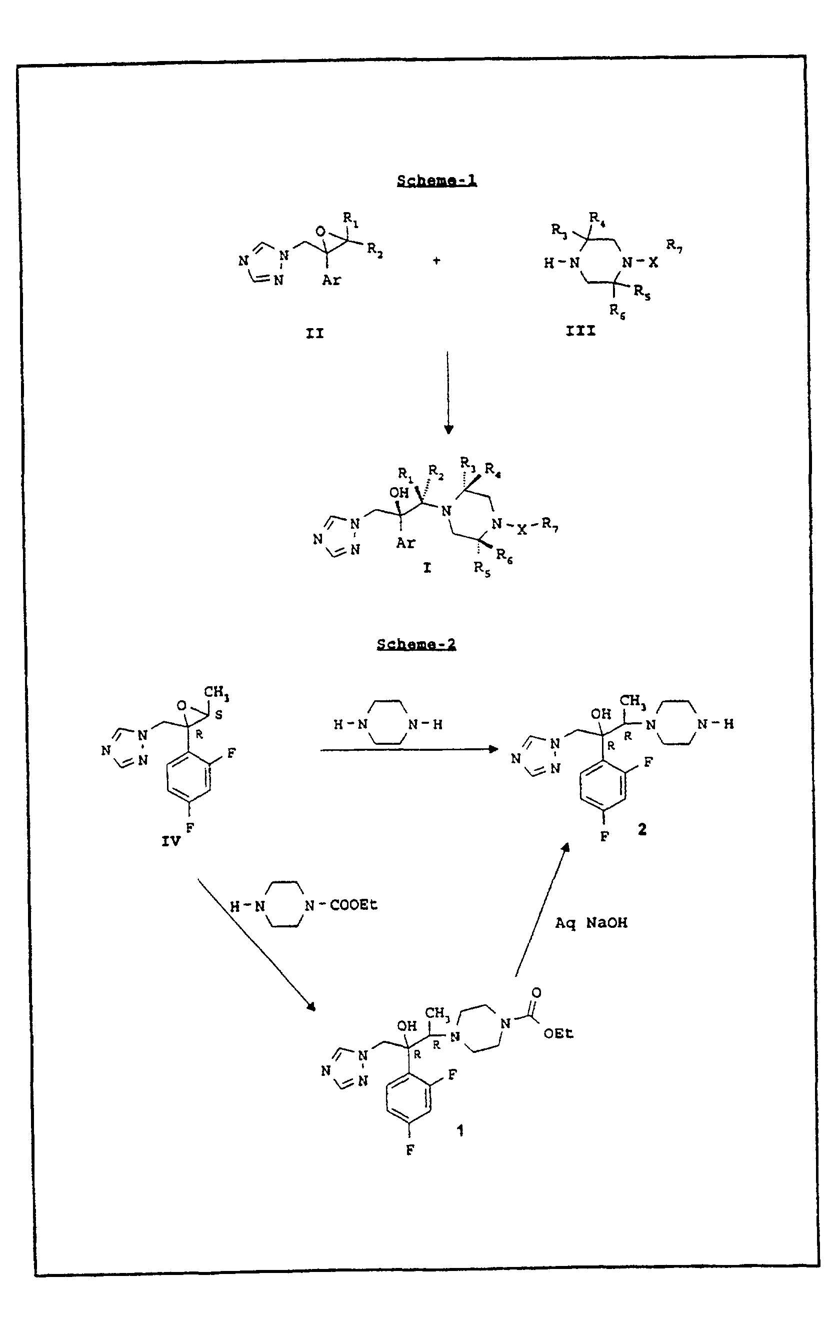

Certain compounds of formula **I** were prepared from a common intermediate, which forms Example **2** of this invention as described in **Scheme 3.** Example **2** was prepared from epoxide **IV** by two synthetic routes as depicted in **Scheme 2**. In the first route, a 1:1 molar ratio of epoxide and ethyl piperazine-1-carboxylate were reacted in the presence of potassium carbonate or lithium perchlorate in a suitable solvent. The obtained ester was hydrolysed with strong base such as sodium hydroxide or potassium hydroxide to give piperazinyl compound **2.** In this reaction the resulting N-carboxylic acid undergoes in-situ decarboxylation to give the piperazino compound **2**. In an alternate route, the piperazino compound was obtained in a single step from the reaction of epoxide **IV** with piperazine. In a typical procedure, the epoxide and excess piperazine were reacted in the presence of lithium perchlorate in a suitable solvent and at a desired temperature.

Certain compound of formula **I** wherein X is CO, SO₂, CS, or -N=N- were prepared as described in **Scheme 3.**

The compounds of formula **I** wherein X is CO were produced from the reaction of piperazinyl compound **2** with acid chloride (R₇COCl) in the presence of a base such as triethylamine, N-methylmorpholine, N,N-diisopropylethylamine. The reaction was carried out in an inert solvent at -20 °C to 80 °C. The most preferred solvents were dichloromethane, chloroform, tetrahydrofuran and acetonitrile. Most preferably the reaction was carried out at 0-25 °C. Certain compounds of formula **I** wherein X is SO₂ were prepared from the reaction of **2** and R₇-SO₂-Cl. In these reactions, R₇ is as described above.

Certain compounds of formula **I** wherein X is CO or CS and R₇ is OR were prepared from the reaction of piperazinyl compound **2** and isocyanates and isothiocyanates. In a typical reaction, a 1:1 molar ratio of compound and isocyanate or isothiocyanate were reacted in a suitable solvent at -15 to 45 °C. The preferred solvents were acetonitrile, ethyl acetate and dichloromethane. The preferred temperature was 0 to 25 °C. The reaction time varied from few hours to several hours depending on the reactants, solvent and temperature.

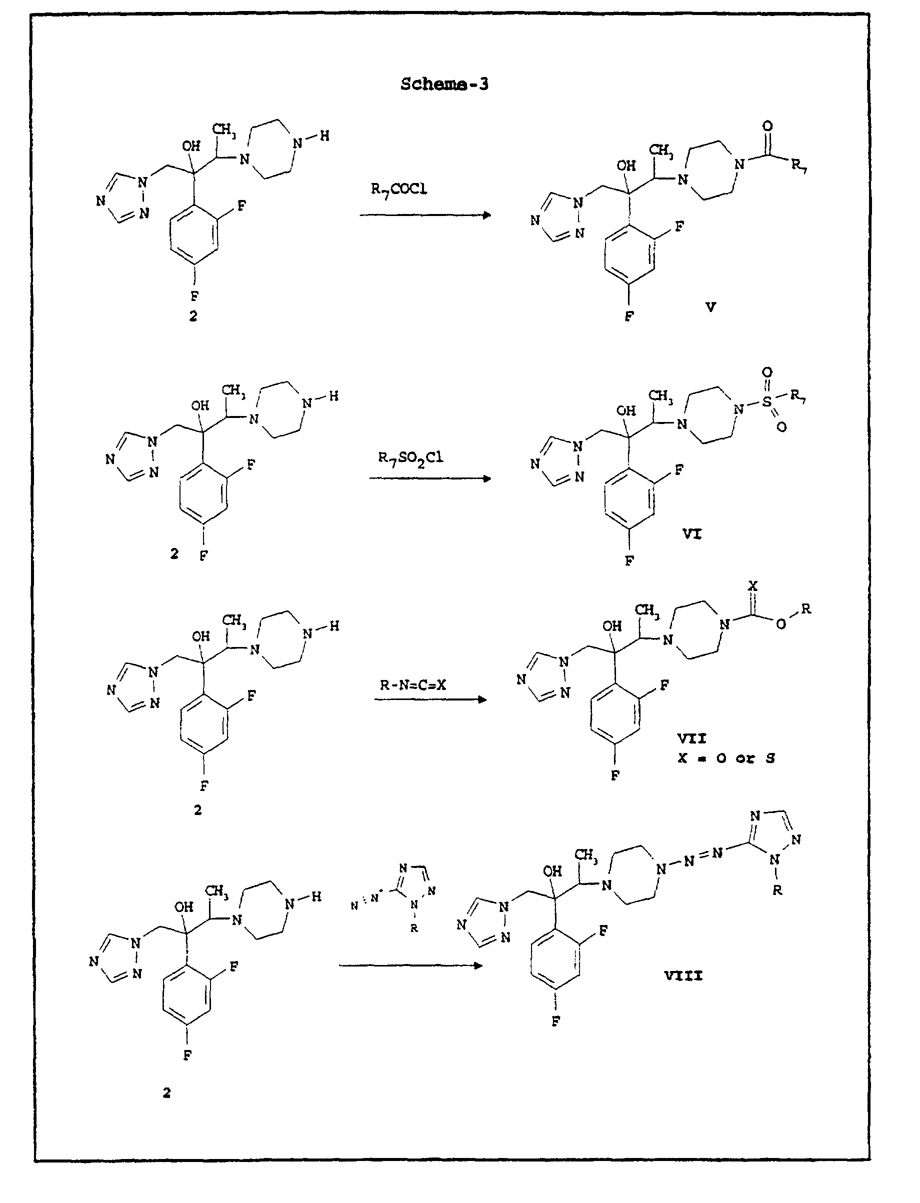

The reaction of 1H or 2H-1,2,4-triazol-3-diazonium salt with the piperazinyl compound **2** gave the N-azo compound of formula **VIII**. This reaction was conducted at suitable temperature in suitable solvent in the presence of base like sodium hydroxide, potassium hydroxide and potassium carbonate.

Certain compounds of formula **I** wherein X is bond and R₇ is unsubstituted or substituted azole were prepared according to **Scheme 1** The synthetic routes followed for the preparation of piperazinyl azoles **X, XI, XII, XIII** and **XVI** are described in **Schemes 4-6.** In a typical procedure, piperazinyl azole and epoxide were heated in a suitable solvent in the presence of lithium perchlorate or potassium carbonate for 24-48 h. The molar ratio of the reactants varied from 1:1 to 1:5. After usual workup the product was purified by column chromatography.

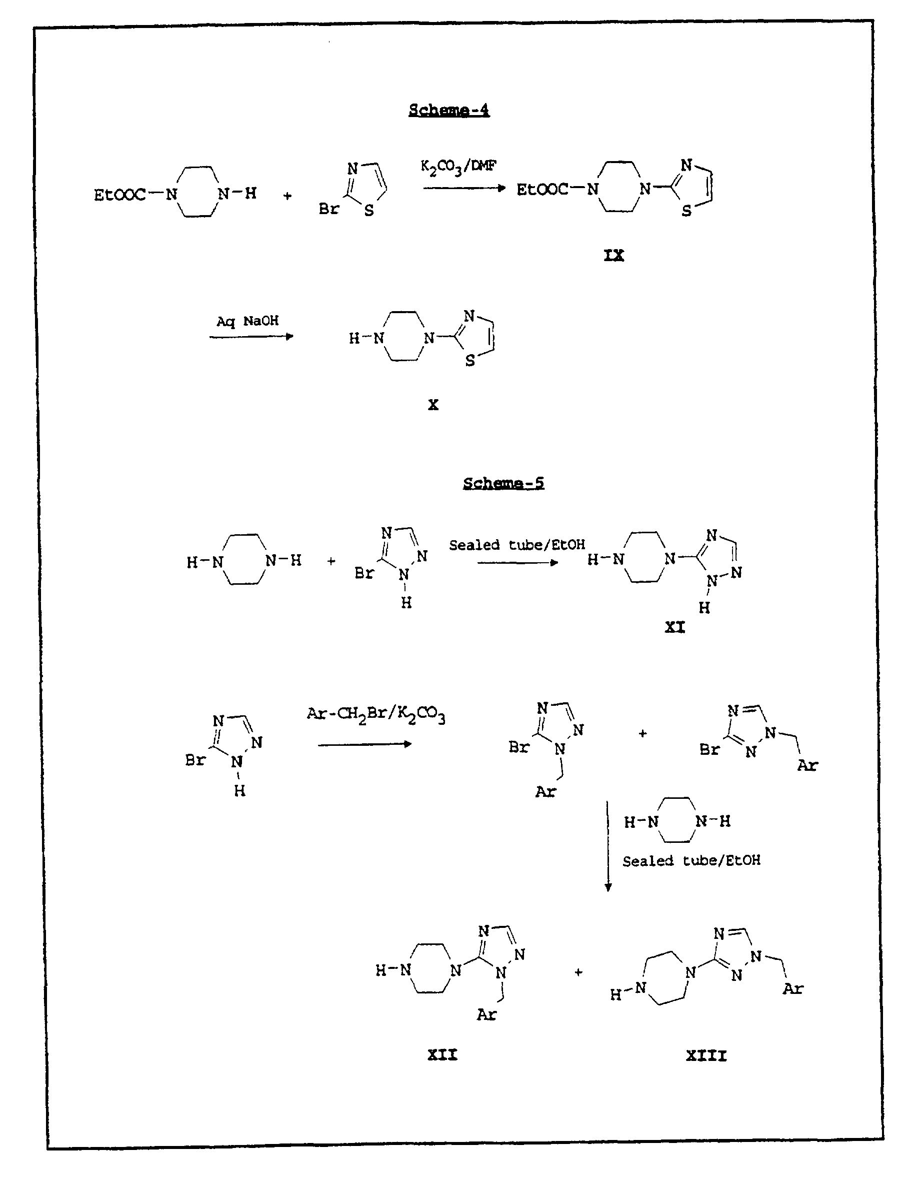

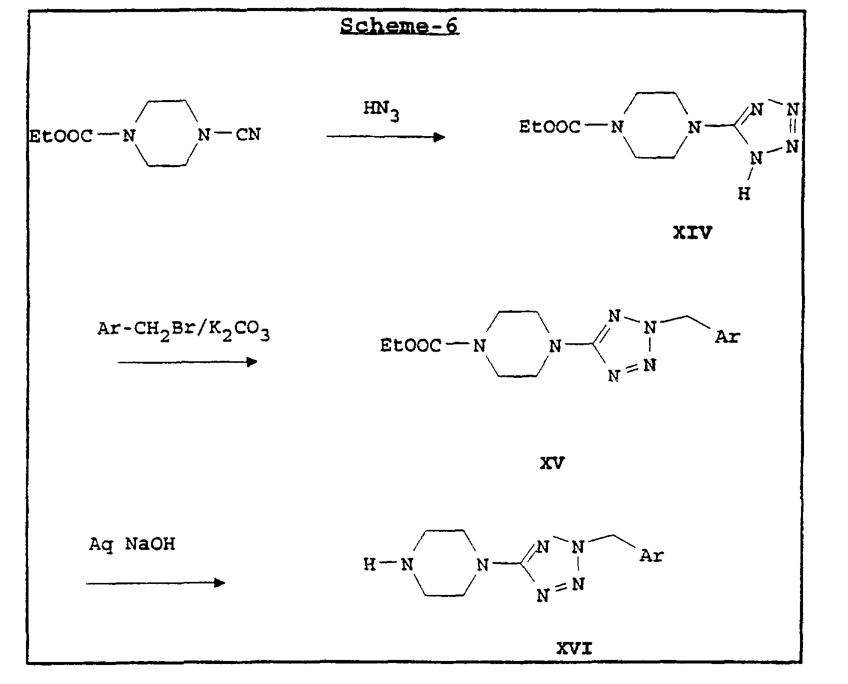

Certain compounds of formula **I** wherein X is a bond and R₇ is a 4-[2-substituted-1,2,4-triazol-3-one-4-yl]phenyl group were prepared in two independent synthetic routes. The first route involves the linear approach as described in **Scheme 7.** The intermediates formed during this synthesis are compounds of formula I, wherein X is bond and R₇ is phenyl group with substitution.

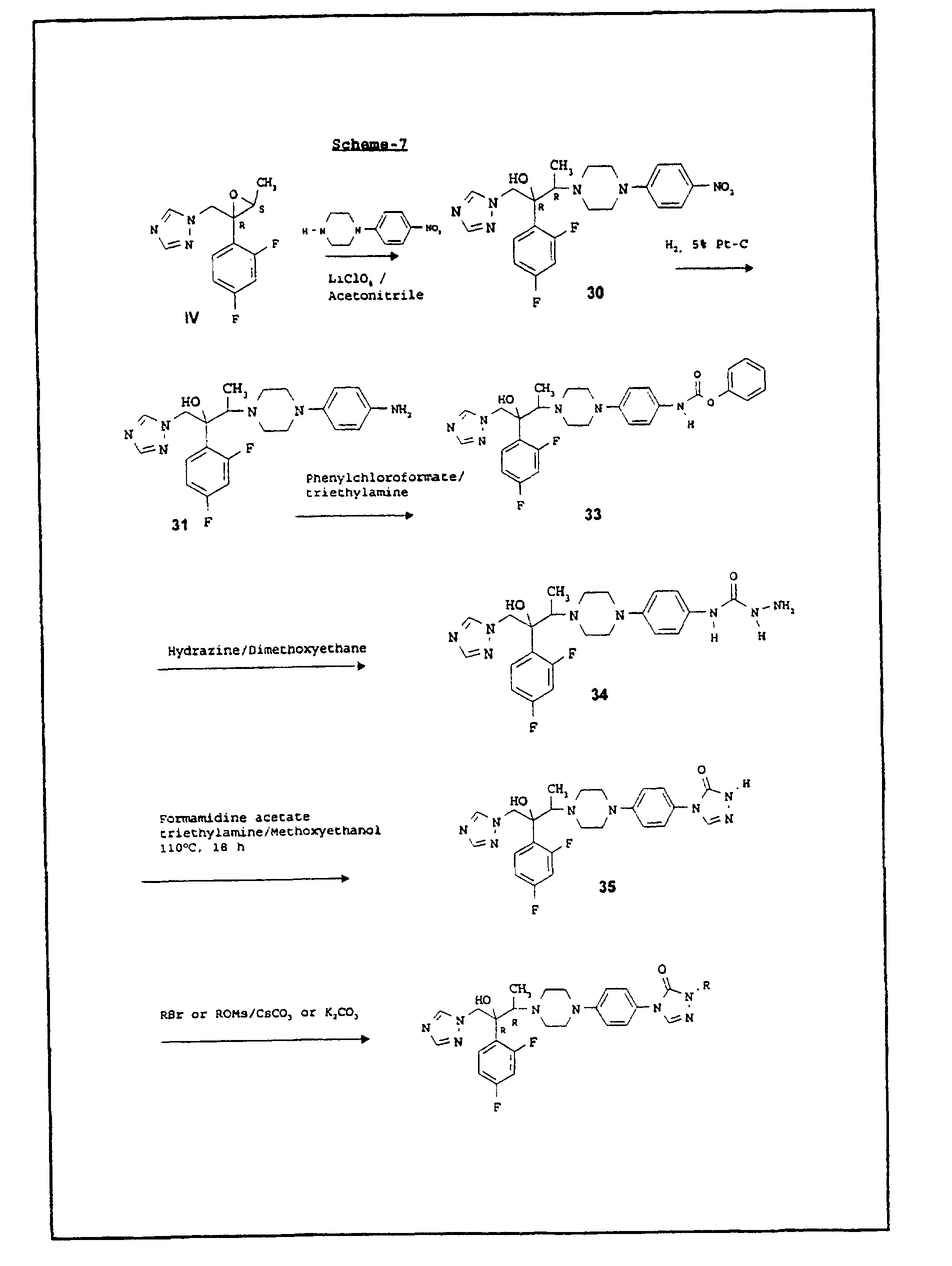

The second synthetic route involves a more efficient convergent approach (**Scheme 1**). In a typical procedure, an appropriate epoxide of formula **II** and an appropriate piperazinyl derivative **XXIII** were reacted in the presence of lithium perchlorate in a suitable solvent and at a suitable temperature. The reactants were allowed to react to the completion of the reaction. The reaction time varied from few hours to several hours depending on the reactants. The epoxides used in this invention are known and were prepared by following the reported procedures. The piperazino compounds of formula **XXIII** were prepared as described in **Scheme 8.**

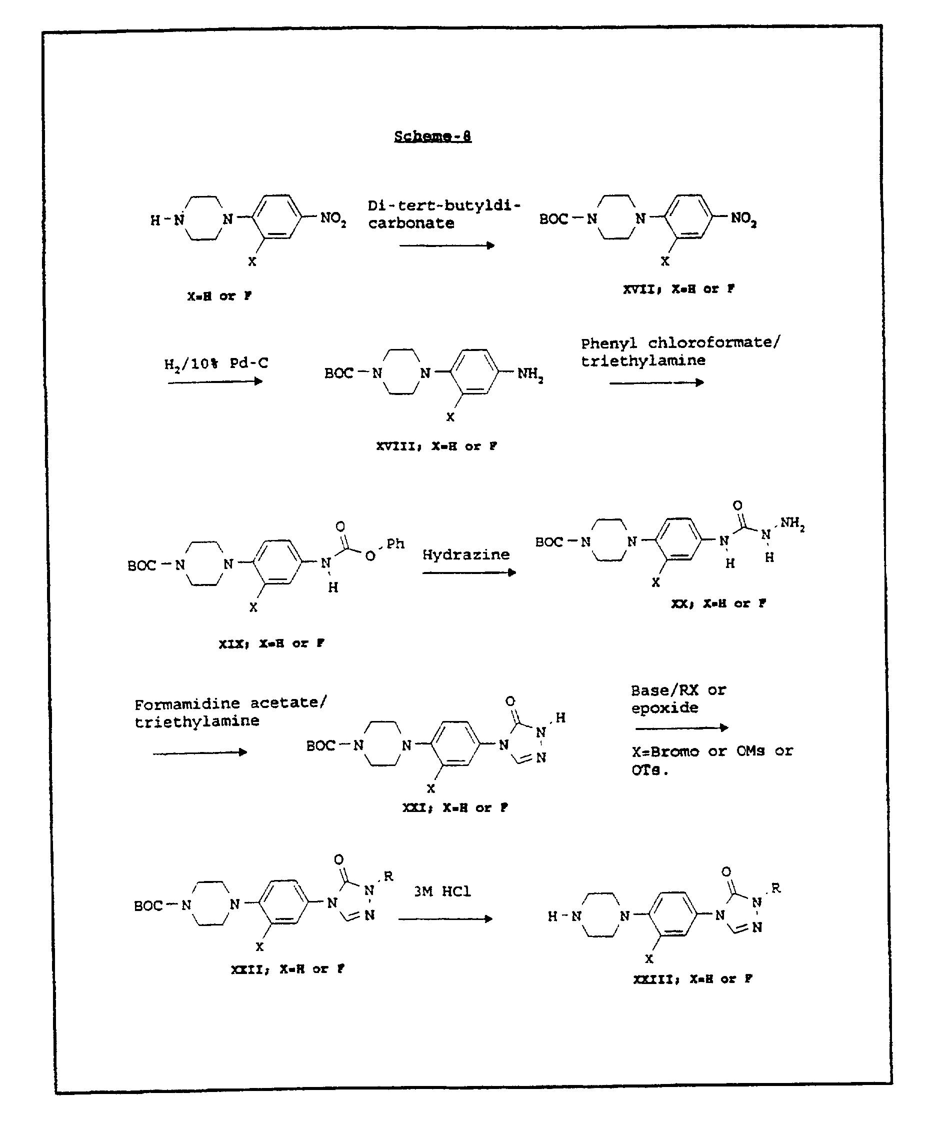

Pharmaceutically acceptable salts of formula **I** were prepared by mixing the solution of the free base with excess of acid at 0-25 °C. The precipitated salt was collected by filtration. In some cases the solvent was evaporated to dryness and the resulting crude salt was recrystallized in a suitable solvent. For the preparation of these acid addition salts, acids were selected from hydrochloric acid, hydrobromic acid, sulphuric acid, tartaric acid, succinic acid, fumaric acid, methanesulphonic acid or *p*-toluenesulphonic acid.

The compounds of the present invention were evaluated *in vitro* for their antifungal activity and were assessed based on minimum inhibitory concentration of the test compound, i.e. the concentration of the test compound at which growth of particular organism fails to occur. The MIC was determined after incubating the test compound with the strain for 48 h at 37 °C in an appropriate medium. The microorganisms used in this test include *Candida albicans, C. tropicalis, C. kefyr, C. krusei, C. guilliermondii, C. glabrata, Cryptococcus neoformans, Aspergillus niger* and *A. fumigatus. In vitro* data of certain representative compounds is provided in the following Table.

**Table 1**

| ***In vitro* activity of compounds of formula I** | | | | | | | | | |
|---|---|---|---|---|---|---|---|---|---|
| Fungal strains | MIC of compounds of formula **I**, µg/ml | | | | | | | | |
| | **# 36** | **# 40** | **# 41** | **# 42** | **# 43** | **# 44** | **# 46** | Fluconazole | **Itraconazole** |
| *Candida* | 0.09 | 0.09 | 0.09 | 0.09 | 0.048 | 0.048 | 0.048 | 0.78 | 0.09 |
| *albicans* | | | | | | | | | |
| *C. tropicalis* | 0.09 | 0.048 | 0.09 | 0.09 | 0.048 | 0.048 | 0.048 | 0.39 | 0.09 |
| *C. kefyr* | 0.048 | 0.048 | 0.48 | 0.048 | 0.048 | 0.048 | 0.048 | 0.39 | 0.09 |
| *C. krusei* | 0.09 | 0.19 | 1.56 | 1.56 | 0.09 | 0.09 | 0.19 | 50 | 0.39 |
| *C. guilliermondii* | 0.048 | 0.048 | 0.048 | 0.09 | 0.09 | 0.048 | 0.048 | 1.56 | 0.19 |
| *C. glabrata* | 0.09 | 0.39 | 0.78 | 12.5 | 0.78 | 1.56 | 0.39 | 12.5 | 0.19 |
| *Cryptococcu s neoformans* | 0.048 | 0.48 | 0.48 | 0.19 | 0.048 | 0.048 | 0.048 | 1.56 | 0.39 |
| *Saccheromyces cerevisiae* | 0.19 | 0.09 | 0.19 | 0.39 | 0.09 | 0.048 | 0.09 | 6.25 | 0.39 |
| *A. niger* | 0.78 | 0.78 | 3.12 | 3.12 | 0.19 | 0.19 | 0.09 | >100 | 0.39 |
| *Aspergillus fumigatus* | 0.39 | 0.39 | 0.78 | 0.39 | 0.19 | 0.048 | 0.048 | >100 | 0.19 |

Certain selected compounds were evaluated *in-vivo* for their antifungal efficacy. Series of doses of test compounds were administered by oral, i.v and s.c. routes to infected mice, i.e mice that are inoculated with a strain of *Candida albicans* or *Aspergillits fumigatus.* Efficacy of test compound was determined based on survival of treated mice compared to control. The *in vivo* efficacy was assessed based on ED₅₀ of the test compound. The following table provides ED₅₀ of certain compounds for systemic infections of *C. albicans* and *A. fumigatus* in mice models.

**Table 2**

| ***In-vivo* activity of compounds of formula I** | | |
|---|---|---|
| | Therapeutic efficacy (ED₅₀ mg/kg) in mice systemic infectinons | |
| Example # | *C. albicans* | *A. fumigatus* |
| **36** | 8.01 | >45 |
| **40** | 4.68 | 67.05 |
| **41** | 8.01 | 89.28 |
| **42** | >90 | 71.14 |
| **43** | 1.57 | 42.3 |
| **46** | 56.02 | >45 |

The compounds of formula **I** and their salts are anti-fungal agents, useful to treat or prevent topical, lung invasive, as well as systemic fungal infections in mammals including humans. For example they are useful in treating topical infections in man caused by species of *Candida, Trichophyton*, *Microsporum*, mucosal infections caused by species of *Candida,* and systemic infections caused by species of *Candida, Aspergillus, Cryptococcus, Pneumocystis, Histoplasma or Blastomyces*. The above compounds have shown impressive *in vivo* efficacy against mice systemic candidiosis, systemic aspergillosis and lung invasive aspergillosis.

When the compounds of the present invention or their pharmaceutically acceptable salts are used for treatment or prophylaxis of fungal infections in mammals including humans, they can be administered alone, but generally it is more preferred to administer the compounds in a pharmaceutical formulation. This formulation varies with intended route of administration. For example the compounds can be administered orally in the form of tablets, coated tablets, capsules, suspensions, solutions and the like. These oral preparations which contain the present compounds are prepared with excipients, binders, coloring agents, flavors, etc. which can be formulated in a manner known in the art.

The present compounds can be injected parenterally, for example intravenously, intramuscularly or subcutaneously. These injections are sterile aqueous solutions which contain the antifungal agent with other substances, such as salts, glucose or an isotonic agent, and can be formulated in a conventional manner.

The amount of the present compounds incorporated into the pharmaceutical composition varies depending on the physical and chemical properties of the drug, dosage and route of administration. Preferably the oral formulations are prepared with 1 to 25% (w/w) antifungal agent and the injection formulation is prepared with 0.1 to 5% (w/w) of antifungal agent.

Alternatively, the antifungal agents also can be administered in the form of a suppository or pessary, or they may be applied topically in the form of lotion, solution, ointment, cream or dusting powder. Suppositories and ointments which contain 1-10% of active ingredient with a base, stabilizer or surfactant can be prepared in a conventional manner.

The dosage of the compound of formula I or its pharmaceutically acceptable salt can be suitably determined depending on the indivdual cases, taking symptoms, age, sex, disease status, patient condition, route of administration and the like into consideration. Usually the dosage can be 0.01-20 mg /kg in single or divided daily doses.

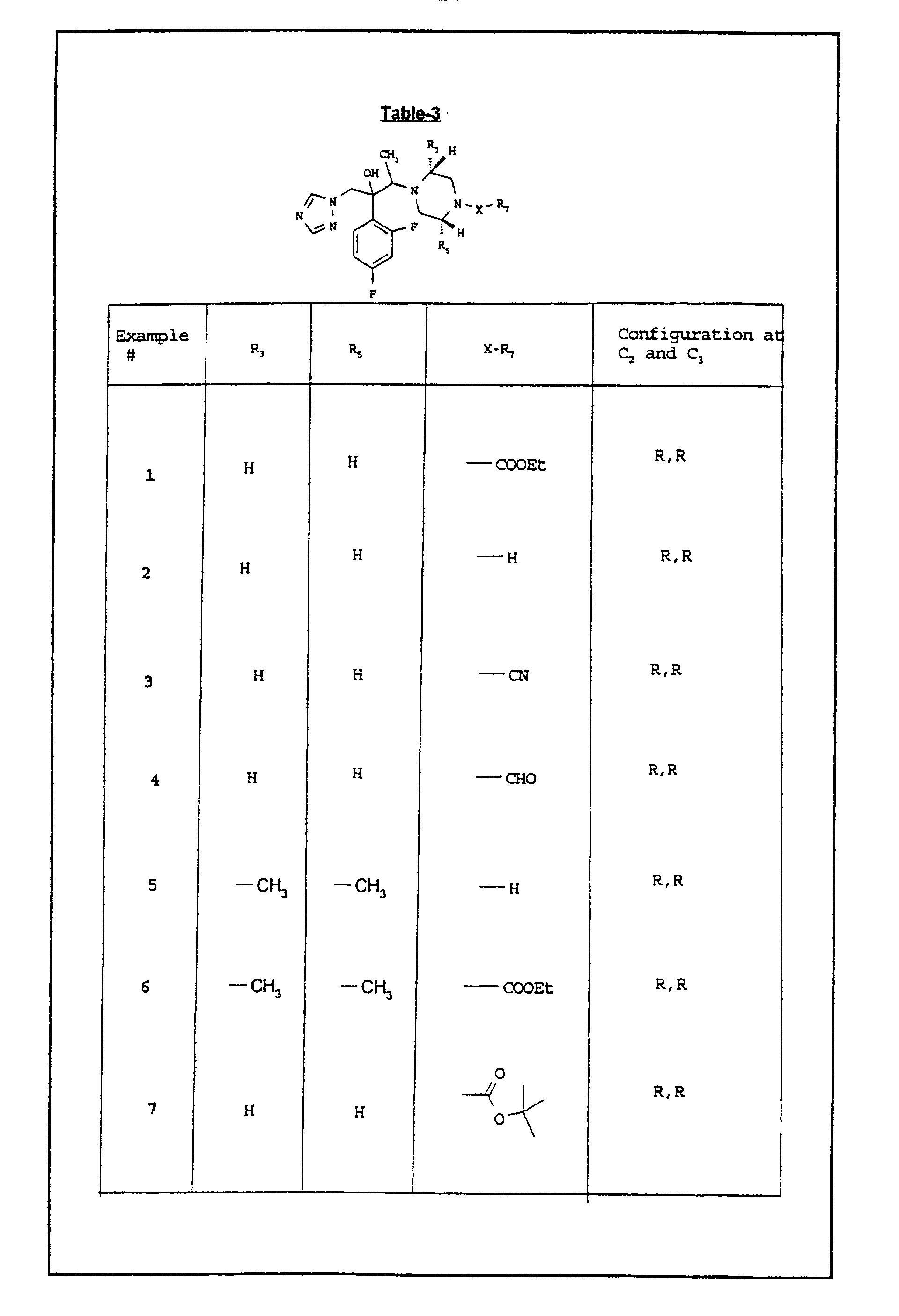

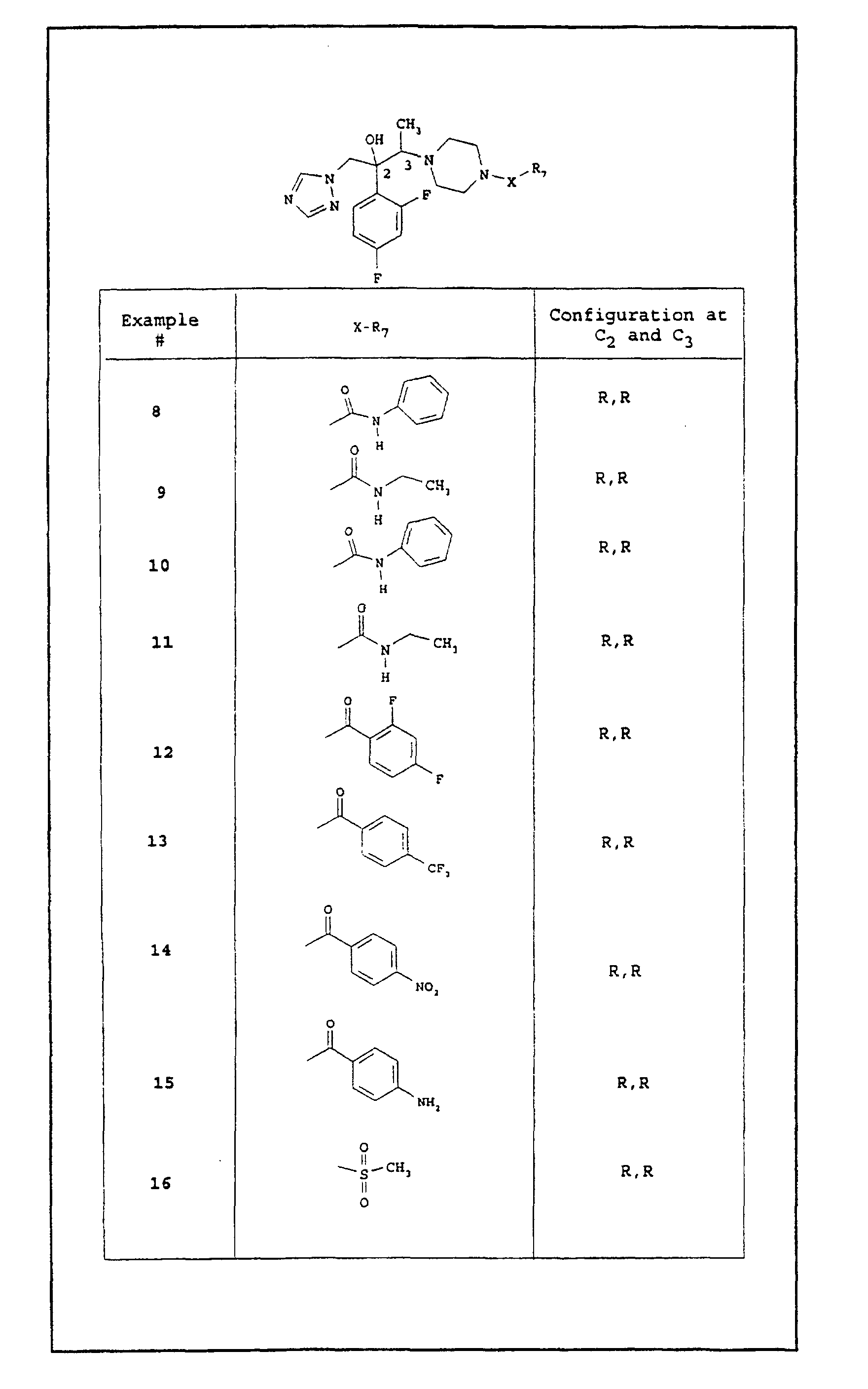

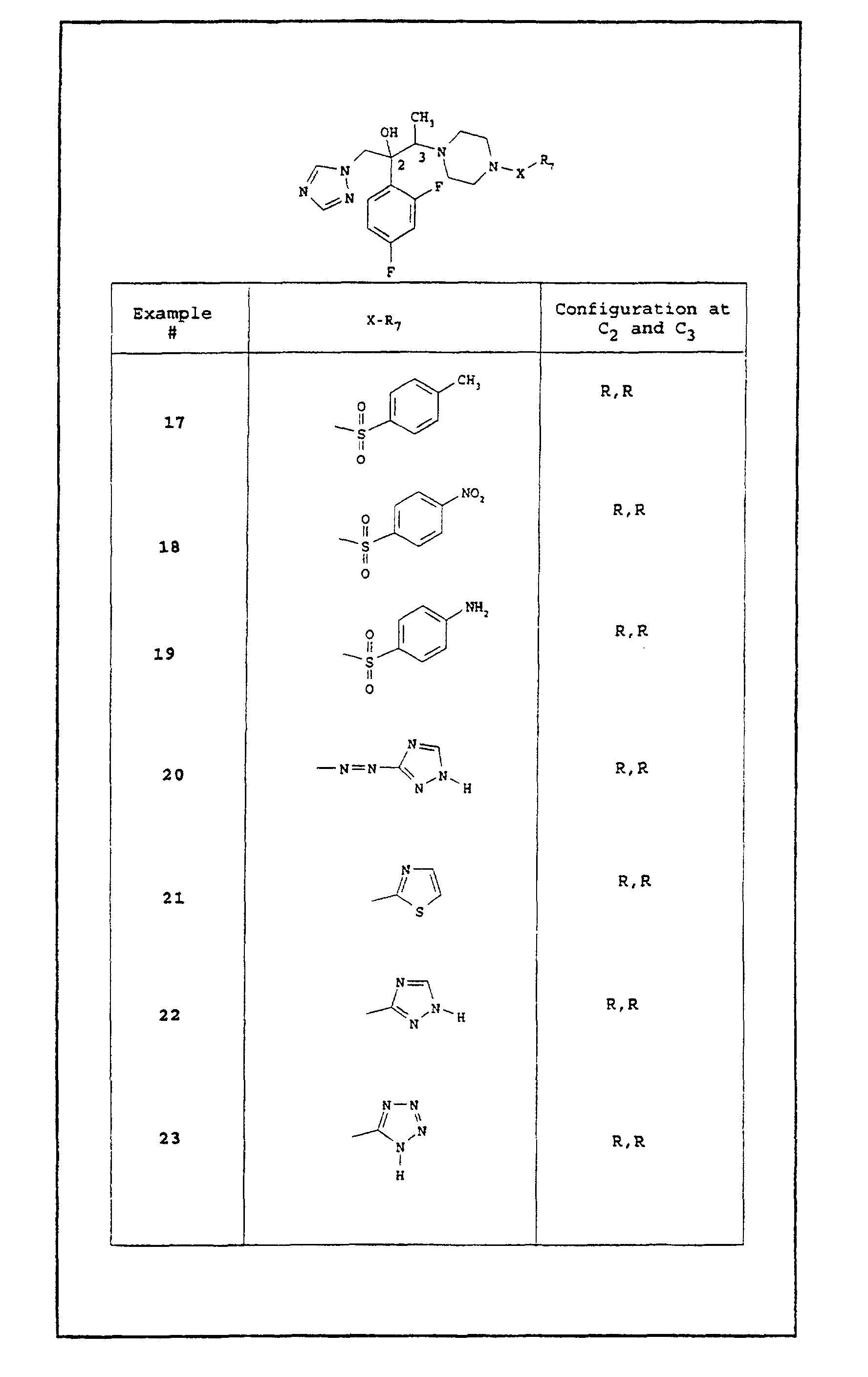

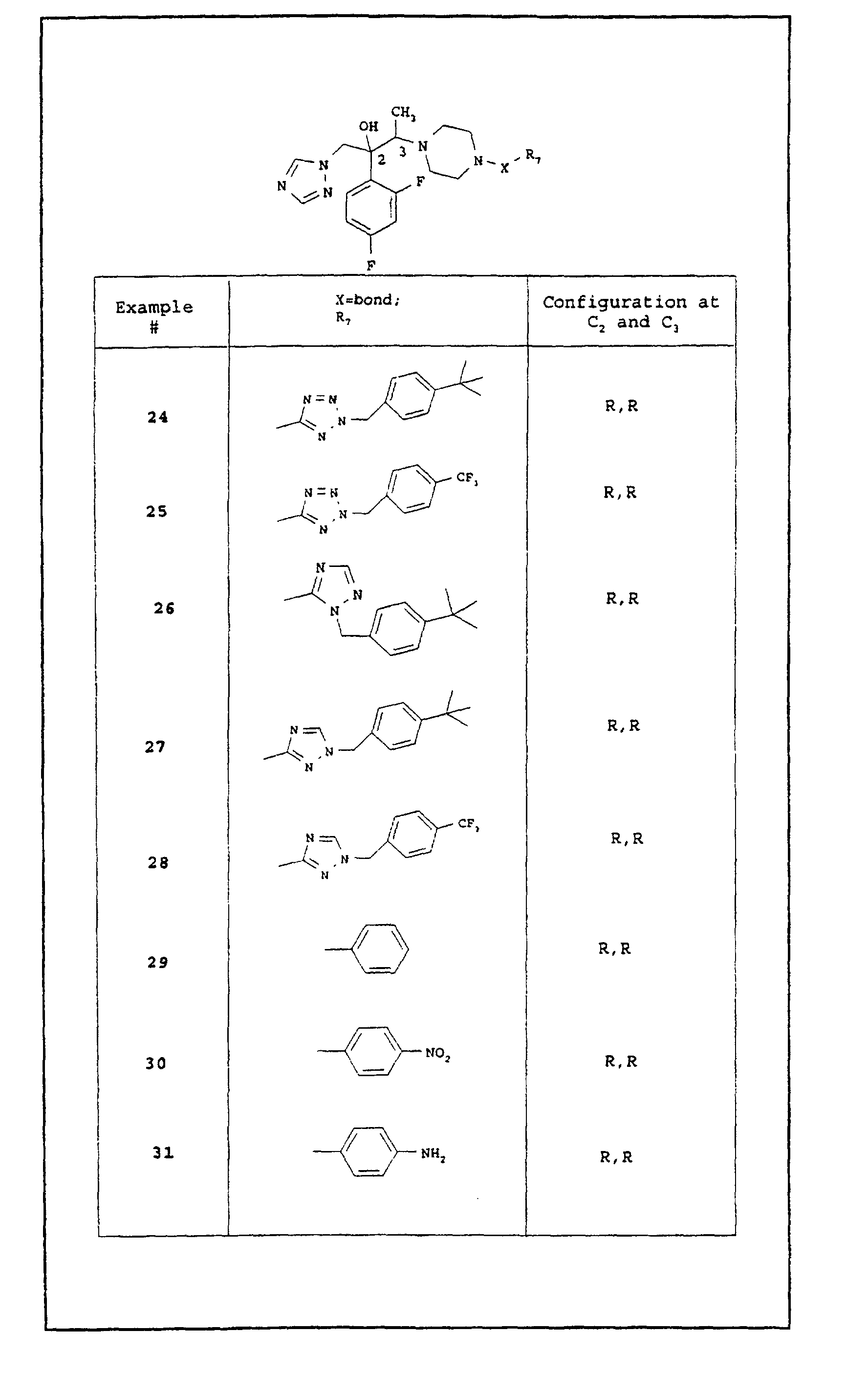

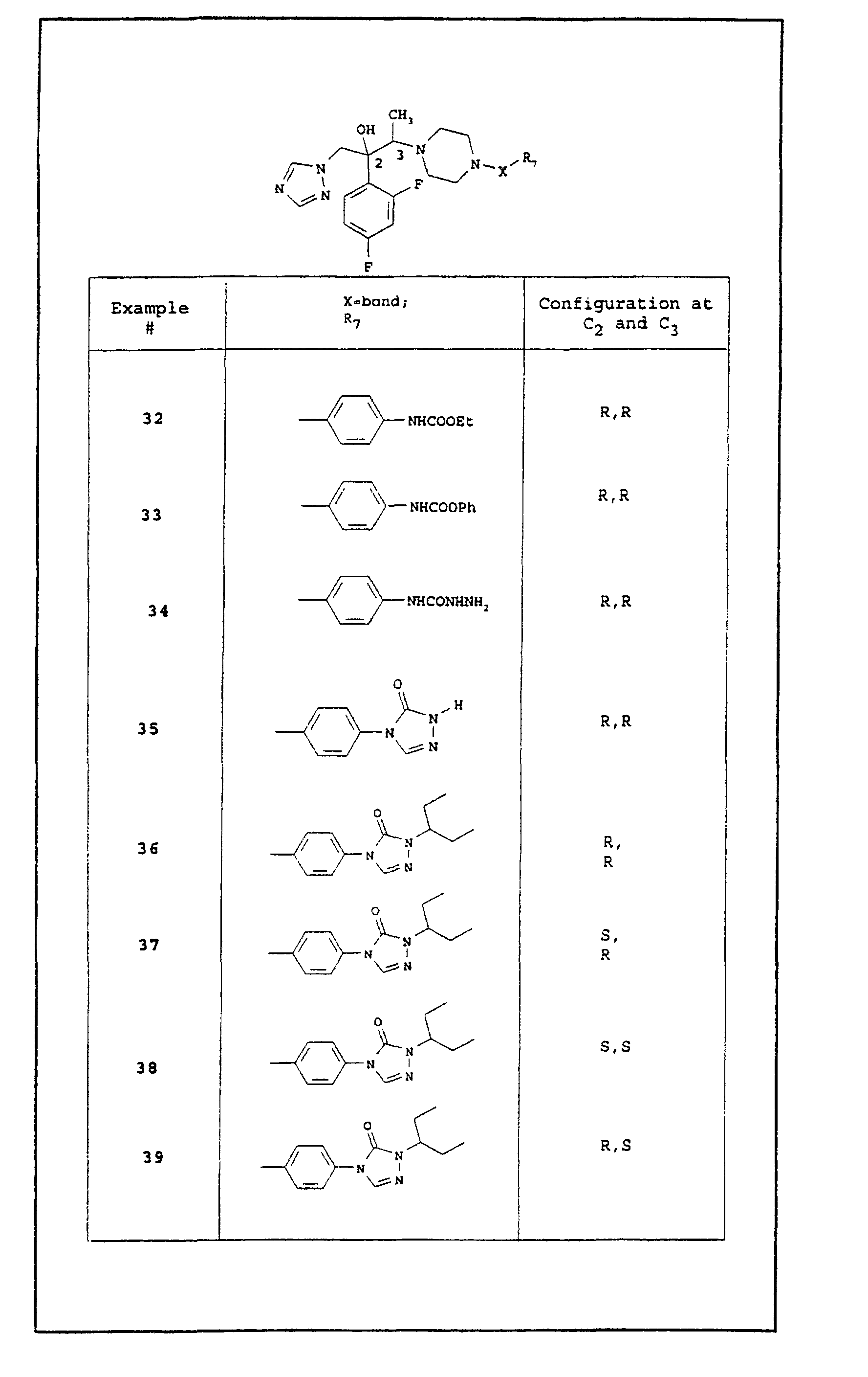

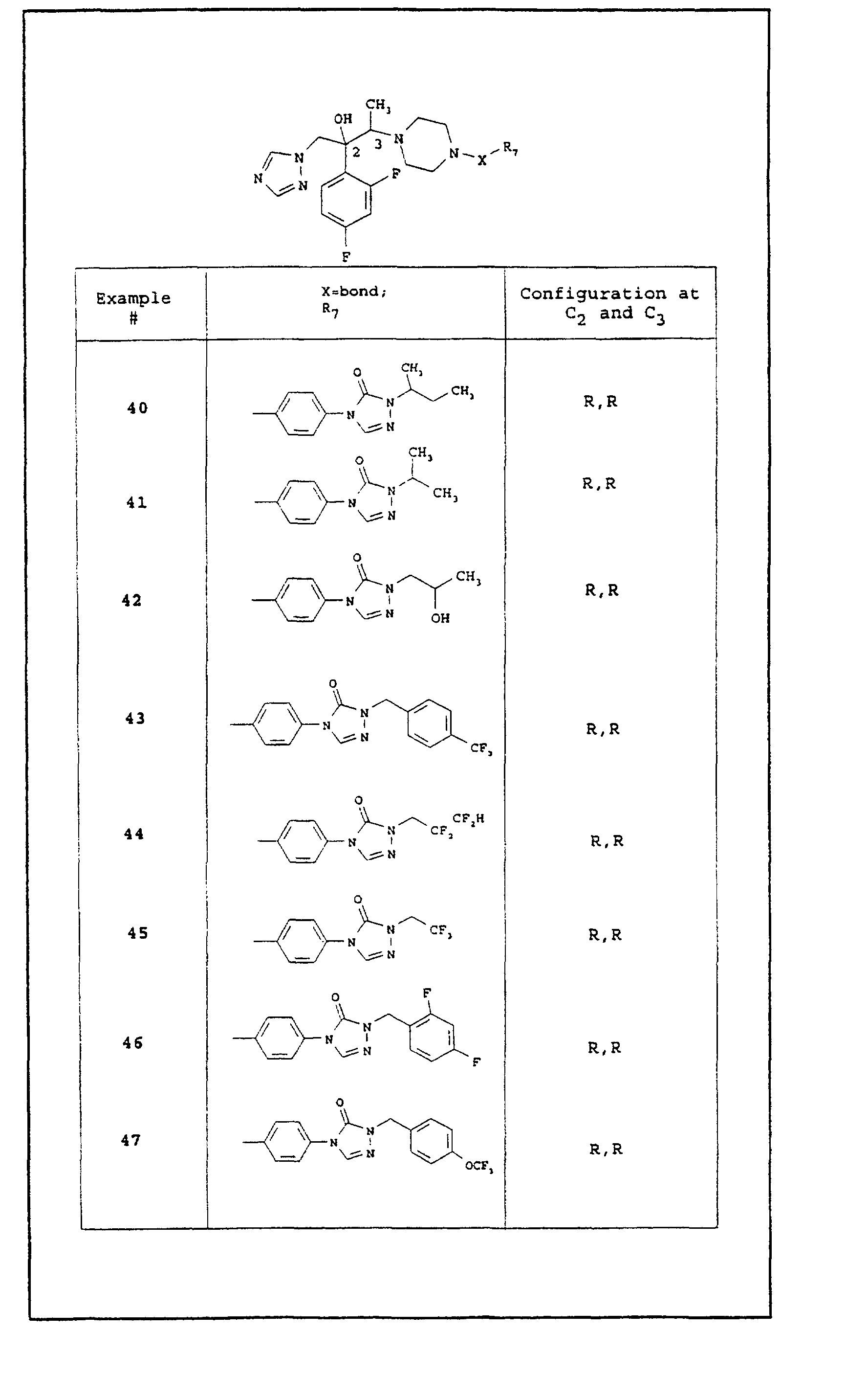

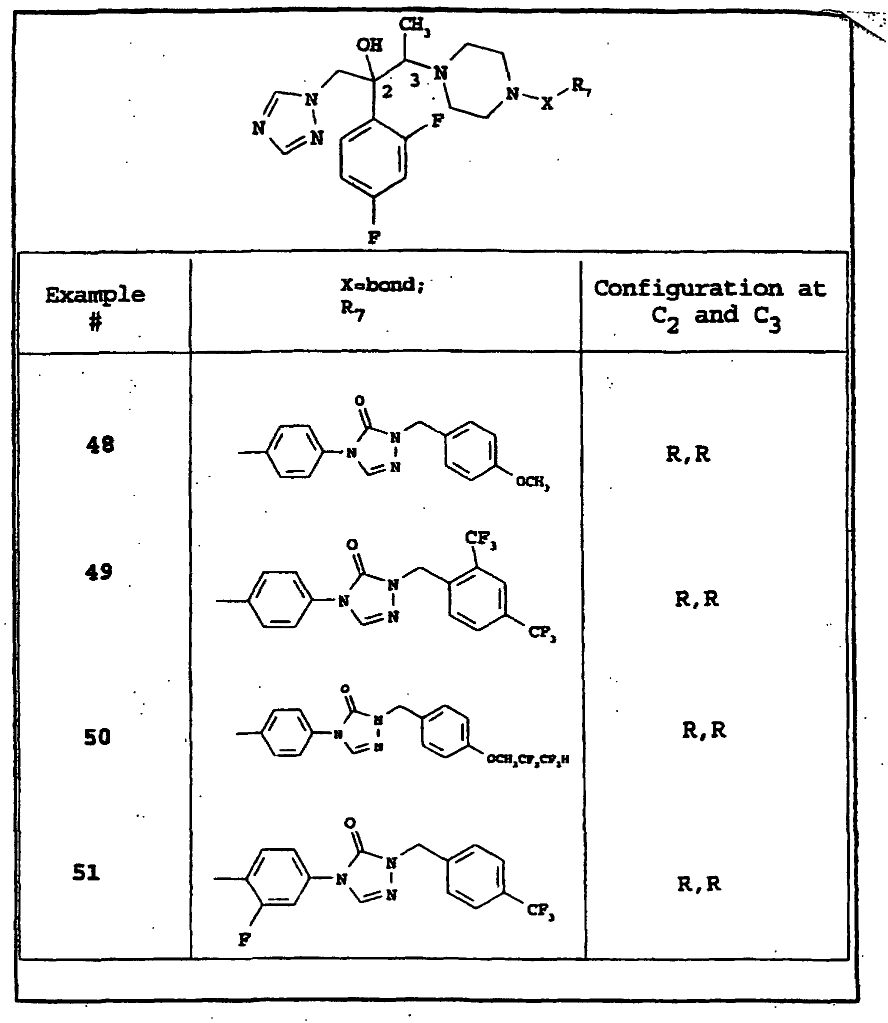

Examples 1-4 & 7 should be regarded as comparative.

### EXAMPLES:

### Example 1

### (2R,3R)-2-(2,4-Difluorophenyl)-3-(4-ethoxycarbonylpiperazin-1-yl)-1-(1H-1,2,4-triazol-1-yl)butan-2-ol:

A mixture of (2R,3S)-2-(2,4-difluorophenyl)-3-methyl-2-(1 H-1,2,4-triazol-1-yl)methyloxirane **IV** (1.0 g, 4.0 mmol), ethyl piperazine-1-carboxylate (1.2 ml, 8 mmol) and potassium carbonate in DMF (5 ml) was heated at 120 °C for 18 h. After cooling the reaction mixture was poured onto crushed ice and extracted with ethyl acetate (3X30 ml). The combined extract was washed with water, brine, dried (MgSO₄) and concentrated and the resulting product was purified on a column of silica gel (hexane/EtOAc, 1:1) to give the title compound as off-white solid (400 mg, 24%).
m.p.: 182-183 °C.
¹H NMR (CDCl₃) δ: 0.90 (d, 3H, CH₃), 1.26 (t, 3H, CH₃), 2.31-2.49 (m, 2H, CH₂), 2.78-3.07 (m, 3H, CH₂ and CH), 3.40-3.59 (m, 4H, 2XCH₂), 4.15 (q, 2H, OCH₂), 4.9 (AB q, 2H, CH₂), 5.05 (s, 1H, OH), 6.66-6.84 (m, 2H, Ar-H), 7.36-7.51 (m, 1H, Ar-H), 7.78 (s, 1H, Het-H), 7.92 (s, 1H, Het-H).
FAB-MS: 410.2 (MH⁺), calcd. C₁₉H₂₅F₂N₅O₃ 409.44.

### Example 2

### (2R,3R)-2-(2,4-Diflurophenyl)-3-(piperazin-1-yl)-1-(1H-1,2,4-triazole-1-yl)butan-2-ol:

A mixture of (2R,3S)-2-(2,4-difluorophenyl)-3-methyl-2-(1H-1,2,4-triazol-1-yl)methyloxirane (1.0 g, 4.0 mmol), piperazine (860 mg, 10 mmol) and lithium perchlorate (625 mg, 6 mmol) in acetonitrile (15 ml) was heated under reflux for 48 h. The solvent was removed under reduced pressure, the residue was treated with crushed ice and extracted with ethylacetate (3X30 ml). The combined organic extract was washed with water, brine, dried (Na₂SO₄) and concentrated to give the title compound as thick viscous gum (1.5 g, 78%). The title compound was also prepared by hydrolysis of 2R,3R-2-(2,4-difluorophenyl)-3-(4-ethoxycarbonylpiperazin-1-yl)-1-(1H-1,2,4-triazol-1-yl)butan-2-ol **1** with 2M sodium hydroxide solution.
¹H NMR (CDCl₃) δ: 0.97 (d, 3H, CH₃), 2.32-2.42 (m, 2H, CH₂), 2.66-2.90 (complex, 8H, 3XCH₂, CH and NH), 4.85 (AB q, 2H, CH₂), 6.68-6.83 (m, 2H, Ar-H), 7.42-7.55 (m, 1H, Ar-H), 7.78 (s, 1H, Het-H), 8.0 (s, 1H, Het-H).
FAB-MS: 338.1 (MH⁺), calcd. C₁₆H₂₁F₂N₅O 337.38.

### Example 3

### (2R,3R)-3-(4-Cyanopiperazin-1-yl)-2-(2,4-diflurophenyl)-1-(1H-1,2,4-triazol-1-yl)butan-2-ol:

To a cooled (0 °C) mixture of (2R,3R)-2-(2,4-difluorophenyl)-3-{piperazin-1-yl)-1-(1H-1,2,4-triazol-1-yl)butan-2-ol **2** (250 mg, 0.74 mmol) and triethylamine (0.42 ml, 3 mmol) in acetonitrile (15 ml) was added cyanogen bromide (160 mg, 1.5 mmol) in acetonitrile (0.5 ml). The reaction mixture was stirred at room temperature for 18 h. The solvent was removed under reduced pressure. To the resulting residue water was added and extracted with ethyl acetate (3X20 ml). The combined organic extract was washed with water, brine and dried over magnesium sulphate. The solvent was removed and the product was purified on a column of silica gel (hexane/EtOAc, 1:1) to give the title compound as a colorless solid (150 mg, 56%).
m.p: 220 °C (decomp).
IR (Nujol) Vmax: 2210 cm⁻¹.
¹H NMR (CDCl₃) δ: 0.89 (d, 3H, CH₃), 2.52-2.61 (m, 2H, CH₂), 3.0-3.31 (m, 7H, 3XCH₂ and CH), 4.88-4.91 (m, 3H, CH₂ and OH), 6.66-6.78 (m, 2H, Ar-H), 7.3-7.5 (m, 1H, Ar-H), 7.78 (s, 1H, Het-H), 7.87 (s, 1H, Het-H).
FAB-MS: 363.0 (MH⁺), calcd. C₁₇H₂₀F₂N₆O 362.39.

### Example 4

### (2R,3R)-2-(2,4-Diflurophenyl)-3-(4-formylpiperazin-1-yl)-1-(1H-1,2,4-triazol-1-yl)butan-2-ol:

A mixture of compound **2** (200 mg, 0.6 mmol) and potassium carbonate (248 mg, 1.8 mmol) in DMF (5 ml) was heated at 120 °C for 24 h. The contents were poured into cold water and extracted with ethyl acetate (3X20 ml). The combined organic extract was washed with water, brine and dried over sodium suphate. The solvent was removed under reduced pressure and the resulting product was purified on a column of silica gel (EtOAc/MeOH; 98:2) to give the title compound as colorless solid (80 mg, 36%).
m.p: 118-120 °C.
¹H NMR (CDCl₃) δ: 0.90 (d, 3H, CH₃), 2.44-2.52 (m, 2H, CH₂), 2.93-3.11 (m, 3H, CH₂ and CH), 3.39-3.59 (m, 4H, 2XCH₂), 4.93-4.97 (m, 3H, CH₂ and OH), 6.66-6.81 (m, 2H, Ar-H), 7.36-7.48 (m, 1H, Ar-H), 7.79 (s, 1H, Het-H), 7.91 (s, 1H, Het-H), 8.02 (s, 1H, CHO).
FAB-MS: 366.1 (MH⁺), calcd. C₁₇H₂₁F₂N₅O₂ 365.39.

### Example 5

### (2R,3R)-2-{2,4-Diflurophenyl)-3-(2,5-dimethylpiperazin-1-yl)-1-(1H-1,2,4-triazol-1-yl)butan-2-ol:

The title compound **5** was obtained as a thick viscous oil in 58% yield from the reacton of (2R,3S)-2-(2,4-difluorophenyl)-3-methyl-2-(1H-1,2,4-triazol-1-yl)methyloxirane **IV** and 2,5-dimethylpiperazine, by a similar method described in example **2**.
¹H NMR (CDCl₃) δ: 0.85-0.96 (3 d merged, 9H, 3XCH₃), 1.85-1.96 (m, 2H), 2.4-2.59 (m, 2H), 2.64-2.96 (m, 3H), 3.27-3.38 (m, 1H), 4.81 (AB q, 2H, CH₂), 5.50 (brs, 1H, OH), 6.69- 6.84 (m, 2H, Ar-H), 7.45-7.58 (m, 1H, Ar-H), 7.81 (s, 1H, Het-H), 8.02 (s, 1H, Het-H).
FAB-MS: 366.1 (MH⁺), calcd. C₁₈H₂₅F₂N₅O 365.43.

### Example 6

### (2R,3R)-2-(2,4-Difiurophenyl)-3-(2.5-dimethyl-4-ethoxycarbonylpiperazin-1-yl)-1-(1H-1,2,4-triazol-1-yl)butan-2-ol:

To a mixture of compound **5** (228 mg, 0.62 mmol) and triethylamine (0.18 ml, 1.24 mmol) in dichloromethane (10 ml) at 0 °C was added dropwise ethyl chloroformate (135 mg, 1.24 mmol) in dichloromethane (3 ml). The reaction mixture was stirred at 0 °C for 30 minutes and 1 h at room temperature. The contents were diluted with 30 ml of dichloromethane, washed with water, brine and dried over magnesium sulphate. The solvent was removed under reduced pressure and the resulting oil was chromatographed on a column of silica gel. Elution with hexane/EtOAc (1:1) gave the title compound as an off-white solid (210 mg, 80%).
m.p: 57-59 °C.
¹H NMR (CDCl₃) δ: 0.94 (d, 3H, CH₃), 1.07-1.10 (m, 6H, 2XCH₃), 1.28 (t, 3H, CH₃), 2.34-2.46 (m, 1H), 2.76-2.82 (m, 1H), 2.96-3.09 (m, 2H), 3.21-3.31 (m, 1H), 3.67 (m, 1H), 3.98-4.27 (m, 3H), 4.80 (AB q, 2H), 5.13 (s, 1H, OH), 6.67-6.82 (m, 2H, Ar-H), 7.39-7.52 (m, 1H, Ar-H), 7.78 (s, 1H, Het-H), 7.94 (s, 1H, Het-H).
FAB-MS: 438.3 (MH⁺), calcd. C₂₁H₂₉F₂N₅O₃ 437.49.

### Example 7

### (2R,3R)-3-(4-tert-BOC-Piperazin-1-yl)-2-(2,4-diflurophenyl)-1-(1H-1,2,4-triazol-1-yl)butan-2-ol:

To a mixture of compound **2** (150 mg, 0.44 mmol) and triethylamine (1.0 ml, 0.72 mmol) in dichloromehane (15 ml) was added di*-tert*-butyldicarbonate (110 mg, 0.48 mmol) at °C. The reaction mixture was stirred for 1h at 0 °C and 3 h at room temperature. Then diluted with chloroform (20 ml), washed with water, brine and dried over sodium sulphate. The solvent was removed under reduced pressure and the resulting product was purified on a column of silica gel (EtOAc/hexane) to give the title compound as a colorless solid (160 mg, 82%).
m.p.: 138-139 °C.
¹H NMR (CDCl₃) δ: 0.92 (d, 3H, CH₃), 1.46 (s, 9H, 3XCH₃), 2.34-2.40 (m, 2H, CH₂), 2.79-2.92 (m, 2H, CH₂), 2.97 (m, 1H, CH), 3.43 (m, 4H, 2XCH₂), 4.88 (AB, q, 2H, CH₂), 5.11 (s, 1H, OH), 6.66-6.80 (m, 2H, Ar-H), 7.38-7.51 (m, 1H, Ar-H), 7.78 (s, 1H, Het-H), 7.94 (s, 1H, Het-H).
FAB-MS 438.2 (MH⁺); calcd. C₂₁H₂₉F₂N₅O₃ 437.49.

### Example 8

### (2R,3R)-3-(4-Anilinocarbonylpiperazin-1-yl)-2-(2,4-difluorophenyl)-1-(1H-1,2,4-triazol-1-yl)butan-2-ol:

To a ice cooled solution of (2R,3R)-2-(2,4-diflurophenyl)-3-(piperazin-1-yl)-1-(1H-1,2,4-triazole-1-yl)butan-2-ol **2** (150 mg, 0.44 mmol) in acetonitrile (10 ml) was added phenylisocyanate (60 mg, 0.5 mmol) in acetonitrile (4 ml). The reaction mixture was stirred at 0 °C for 2h, diluted with 20 ml of ethyl acetate and successively washed with water, brine and dried over sodium sulphate. This organic extract was concentrated and the residue was purified by passing through a column of silica gel (hexane/EtOAc) to give the title compound as a colorless solid (170 mg, 84%).
m.p.: 98-100 °C (decomp).
¹H NMR (CDCl₃) δ: 0.92 (d, 3H, CH₃), 2.5 (m, 2H, CH₂), 3.02 (m, 3H, CH₂ and CH), 3.52 (m, 4H, 2XCH₂), 4.8-5.1 (AB q and S merged, 3H, CH₂ and OH), 6.31 (br s, 1H, NH), 6.65-6.85 (m, 2H, Ar-H), 7.0-7.1 (m, 1H, Ar-H), 7.25-7.5 (m, 5H, Ar-H), 7.78 (s, 1H, Het-H), 7.92 (s, 1H, Het-H).
MS (FAB): 457.0 (MH⁺), calcd. C₂₃H₂₆F₂N₆O₂ 456.49.

### Example 9

### (2R,3R)-2-(2,4-Difluorophenyl)-3-(4-ethylaminocarbonylpiperazin-1-yl)-1-(1H-1,2,4-triazol-1-yl)butan-2-ol:

The title compound was prepared similarly to example **8** starting from same piperazine derivative and ethylisocyanate. The product was obtained as a colorless solid in 75 % yield.
m.p.: 103-105 °C.
¹H NMR (CDCl₃) δ: 0.9 (d, 3H, CH₃), 1.14 (t, 3H, CH₃), 2.42 (m, 2H, CH₂), 2.8-3.1 (m and q merged, 3H, CH₂ and CH), 3.2-3.5 (m, 6H, 3XCH₂), (4.4 brs, 1H, NH), 4.88 (AB q, 2H, CH₂), 5.06 (s, 1H, OH), 6.7-6.9 (m, 2H, Ar-H), 7.45 (m, 1H, Ar-H), 7.78 (s, 1H, Het-H), 7.93 (s, 1H, Het-H).
FAB-MS: 409.1 (MH⁺), calcd. C₁₉H₂₆F₂N₆O₂ 408.45.

### Example 10

### (2R,3R)-3-(4-Anilinothiocarbonylpiperazin-1-yl)-2-(2,4-difluorophenyl)-1-(1H-1,2,4-triazol-1-yl)butan-2-ol:

The title compound was prepared similarly to example **8** starting from same piperazine derivative **2** and phenylisothiocyanate. The product was obtained as a colorless solid in 80% yield.
m.p.: 106-108 °C.
¹H NMR (CDCl₃) δ: 0.88 (d, 3H, CH₃), 2.5 (m, 2H, CH₂), 2.9-3.1 (m, 3H, CH₂ and H), 3.84 (m, 4H, 2XCH₂), 4.8-5.1 (AB q and s merged, CH₂ and OH), 6.7-6.85 (m, 2H, Ar-H), 7.1-7.5 (m, 7H, 6 Ar-H and 1 NH), 7.77 (s, 1H, Het-H), 7.88 (s, 1H, Het-H).
FAB-MS: 473.3 (MH⁺), calcd. C₂₃H₂₆F₂N₆OS 472.56.

### Example 11

### (2R,3R)-2-(2.4-Difluorophenyl)-3-(4-ethylaminothiocarbonylpiperazin-1-yl)-1-(1H-1,2,4-triazol-1-yl)butan-2-ol:

The title compound was prepared similarly to example **8** starting from same piperazine derivative **2** and ethylthioisocyanate. The product was obtained as a colorless solid in 85% yield.
m.p.: 96-98 °C.
1H NMR (CDCl₃) δ: 0.88 (d, 3H, CH₃), 1.25 (t, 3H, CH₃), 2.5 (m, 2H, CH₂), 3.06 (m, 3H, CH₂ and CH), 3.6-4.0 (q and m merged, 6H, 3XCH₂), 4.8-5.0 (AB q and s merged, 3H, CH₂ and OH), 5.39 (s, 1H, NH), 6.7-6.9 (m, 2H, Ar-H), 7.4- 7.5 (m, 1H, Ar-H), 7.78 (s, 1H, Het-H), 7.89 (s, 1H, Het-H).
FAB-MS: 425.1 (MH⁺), calcd. C₁₉H₂₆F₂N₆OS 424.51.

### Example 12

### (2R,3R)-3-[4-(2,4-Difluorobenzoyl)piperazin-1-yl]-2-(2,4-difluorophenyl)-1-(1H-1,2,4-triazol-1-yl)butan-2-ol.

To a mixture of compound **2** (674 mg, 2 mmol) and triethylamine (0.55 ml, 4 mmol) in tetrahydrofuran (10 ml) was added dropwise 2,4-difluorobezoyl chloride (440 mg, 2.5 mmol) in 5 ml tetrahydrofuran at 0 °C. The reaction mixture was stirred at 0 °C for 30 minutes and at room temperature for 18 h. The solvent was removed under reduced pressure, the residue was dissolved in dichloromethane (50 ml). The organic phase was washed with brine, dried over sodium sulphate. The solvent was removed under reduced pressure and the resulting product was purified on a column of silica gel (EtOAc/hexane, 9:1) to give the title compound as colorless prisms ( 448 mg, 47%).
m.p. = 63-65°C.
1H NMR (CDCl₃) δ: 0.88 (d, J = 6.1 Hz, 3H), 2.04-2.59 (m, 2H); 2.99-3.09 (m, 4H); 3.35 (s, 2H); 3.72-4.10 (m, 1H) 4.91 (s, 2H); 5.01 (s, 1H); 6.65-7.00 (m, 5H); 7.34-7.48 (m, 1H); 7.77 (s, 1H); 7.9 (s, 1H).
FAB-MS: 478.3 (MH⁺), calcd. C₂₃H₂₃F₄N₅O₂ 477.46.

### Example 13

### (2R,3R)-2-(2,4-Difluorophenyl)-1(1H-1,2,4-triazol-1-yl)-3-[4-(4-trifluoromethylbenzoyl)piperazin-1-yl]butan-2-ol.

The example **13** was prepared form the reaction of piperazino derivative **2** and 4-(trifluoromethyl)benzoyl chloride by following the similar procedure described for the example **12.**
Colorless prisms, m.p.: 88-90 °C, Yield 87%.
¹H NMR (CDCl₃) δ: 0.88 (d, J = 6.5 Hz, 3H); 2.39-2.59 (m, 2H) 3.00-3.10 (m, 4H); 3.41-3.73 (m, 4H); 4.83 (AB q, 2H) 6.65-6.79 (m, 2H); 7.35-7.47 (m, 1H); 7.49 (d, J=8.0 Hz, 2H) 7.66 (d, J=8.0 Hz, 2H); 7.78 (s, 1H), 7.91 (s, 1H).
FAB-MS: 510.1 (MH⁺), Calcd. C₂₄H₂₄F₅N₅O₂ 509.48.

### Example 14

### (2R,3R)-2-(2,4-Difluorophenyl)-3-[4-(4-nitrobenzoyl)piperazin-1-yl]-1-(1H-1,2,4-triazol-1-yl)butan-2-ol

The example **14** was prepared form the reaction of piperazino derivative **2** and 4-nitrobenzoyl chloride by following the similar procedure described for the example **12.** After column purification the product was obtained as brown prisms in 92% yield.
m.p. 90-92 °C.
¹H NMR (CDCl₃) δ: 0.88 (d, J = 6.5 Hz, 3H), 2.42-2.61 (m, 2H) 3.05-3.08 (m, 4H); 3.39-3.73 (m, 3H); 4.92 (s, 3H) 6.66-6.78 (m, 2H); 7.34-7.47 (m, 1H); 7.55 (d, J = 8.6 Hz, 2H) 7.77 (s, 1H); 7.88 (s, 1H); 8.26 (d, J = 8.6 Hz, 2H).
FAB-MS: 487.0 (MH⁺),C₂₃H₂₄F₂N₆O₄ 486.42.

### Example 15

### (2R,3R)-3-[4-(4-Aminobenzoyl)piperazin-1-yl]-2-(2,4-difluorophenyl)-1-(1H-1,2,4-triazol-1-yl)butan-2-ol

A solution of nitrocompound **14** (487 mg, 1.0 mmol) in ethyl acetate (95 ml) was hydrogenated over 10% Pd-C (50 mg) at 45 psi pressure and room temperature for 6 h. The catalyst was removed by filtration and the filtrate was concentrated to give the amino compound **15** as a colorless solid (330 mg, 72%).
m.p.: 100-102 °C.
¹H NMR (CDCl₃) δ : 0.89 (d, J = 5.8 Hz, 3H); 2.42-2.47 (m, 2H) 2.88-3.06 (m, 3H); 3.63 (br, 4H); 3.09 (br, 2H) 4.82 (t, J = 16.3 Hz, 2H); 5.02 (br, 1H); 6.61 (d, J = 8.5 Hz, 2H) 6.73-6.79 (m, 2H); 7.22 (d, J = 8.5 Hz, 2H); 7.30-7.48 (m, 1H) 7.77 (s, 1H); 7.92 (s, 1H).
FAB-MS: 457.1 (MH⁺), calcd. C₂₃H₂₆F₂N₆O₂ 456.46.

### Example 16

### (2R,3R)-2-(2,4-Difluorophenyl)-3-[4-(p-toluenesulphonyl)piperazin-1-yl]-1-(1H-1,2,4-triazol-1-yl)butan-2-ol:

To a mixture of piperazinyl compound **2** (146 mg, 0.43 mmol) and triethylamine in dichloromethane (5 ml) was added p-toluenesulphonyl chloride (51 mg, 0.45 mmol) in dichloromethane (1 ml) at 0 °C. The reaction mixture was stirred at 0 °C for 15 minutes and 2 h at room temperature. Then the reaction mixture was diluted with chloroform (15 ml), washed with water, brine and dried over sodium sulphate. The solvent was removed under reduced pressure and the resulting product was purified on a column of silica gel (CHCl₃/MeOH; 98:2 ) to give the title compound as a colorless solid (75 mg, 16%).
m.p.: 81-83 °C.
¹H NMR (CDCl₃) δ : 0.88 (d, J = 6.9 Hz, 3H), 2.44 (s, 3H), 2.48-2.57 (m, 2H), 2.92-3.08 (m, 7H), 4.74 (s, 2H), 4.83 (s, 1H), 6.64-6.76 (m, 2H), 7.29-7.36 (m, 3H), 7.63 (s, 1H), 7.68 (s, 1H), 7.74 (s, 1H), 7.80 (s, 1H).
FAB-MS: 491.9 (MH⁺), calcd. C₂₃H₂₇F₂N₅O₃S 491.55.

### Example 17

### (2R,3R)-2-(2,4-Difluorophenyl)-3-[4-(methanesulphonyl)piperazin-1-yl]-1-(1H-1,2,4-triazol-1-yl)butan-2-ol:

The epoxide **IV** (300 mg, 1.19 mmol) and LiClO₄ (235 mg, 1.43 mmol) were dissolved in 12 mL dry acetonitrile and 1-methanesulfonylpiperazine (190 mg, 1.79 mmol) was added. The mixture was heated to reflux for 4 days, cooled and the solvent evaporated. The residue was dissolved in dichloromethane and then washed with water and brine. The solution was dried over Na₂SO₄ and the solvent evaporated. The crude reaction products were eluted through a silica gel column using 3% MeOH/97% EtOAc as eluent to give the title compound (180 mg, 36%) as a colorless solid.
m.p.: 83-85 °C.
¹H NMR (CDCl₃) δ: 0.91 (d, J = 6.4 Hz, 3H), 2.56-2.61 (m, 2H), 2.80 (s, 3H), 3.02-3.18 (m, 3H), 3.22-3.30 (m, 4H), 4.89 (s, 2H), 4.93 (s, 1H), 6.66-6.79 (m, 2H), 7.35-7.48 (m, 1H), 7.79 (s, 1H), 7.88 (s, 1H).
FAB-MS: 415.9 (MH⁺), C₁₇H₂₃F₂N₅O₃S 415.45

### Example 18

### (2R,3R)-2-(2,4-Difluorophenyl)-3-[4-(4-nitrophenylsulphonyl)piperazin-1-yl]-1-(1H-1,2,4-triazol-1-yl)butan-2-ol.:

The title compound was prepared from piperazinyl compound **2** and p-nitrobenzenesulphonyl chloride by following the similar procedure described for example **12.** The product was obtained as tan needles in 38% yield after purification on a column of silica gel (CHCl₃/MeOH; 96:4) followed by recrystalization in ether.
m.p.: 160-162 °C.
¹H NMR (CDCl₃) δ : 0.87 (d, J = 6.7 Hz, 3H), 2.51-2.66 (m, 2H), 2.96-3.20 (m, 7H), 4.74 (s, 2H), 4.81 (s, 1H), 6.62-6.76 (m, 2H), 7.29-7.41 (m, 1H), 7.72 (s, 1H), 7.77 (s, 1H), 7.94-7.98 (m, 2H), 8.39-8.43 (m, 2H).
FAB-MS: 523.2 (MH⁺), calcd. C₂₂H₂₄F₂N₆O₅S 522.52.

### Example 19

### (2R,3R)-3-[4-(4-aminophenylsulphonyl)piperazin-1-yl]-2-(2,4-difluorophenyl)-1-(1H-1,2,4-triazol-1-yl)butan-2-ol:

A solution of nitrocompound **18** (230 mg, 0.44 mmol) in ethanol (10 ml) was hydrogenated over 10% Pd-C (25 mg) at 45 psi pressure and room temperature for 18 h. The catalyst was removed by filtration and the filtrate was concentrated and the resulting product was purified on a column of silica gel (CHCl₃/MeOH; 57:43) to give the amino compound **19** as a colorless solid (165 mg, 76%).
m.p.: 95-97 °C.
¹H NMR (CDCl₃) δ : 0.88 (2, J = 6.1 Hz, 3H), 2.48-2.58 (m, 2H), 2.96-3.09 (m, 7H), 4.14 (br, 2H), 4.75 (s, 2H), 4.86 (s, 1H), 6.63-6.77 (m, 4H), 7.31-7.43 (m, 1H), 7.52-7.57 (m, 2H), 7.74 (s, 1H), 7.83 (s, 1H).
FAB-MS: 493.5 (MH⁺), calcd. C₂₂H₂₆F₂N₆O₃S 492.54.

### Example 20

### (2R,3R)-2-{2,4-Difluorophenyl)-3-[4-(3-azo-1H-1,2,4-triazolyl)piperazin-1-yl]-1-(1H-1,2,4-triazol-1-yl)butan-2-ol:

To a cooled solution of 3-amino-1H-1,2,4-triazole (378 mg, 4.5 mmol) in concentrated hydrochloric acid (1 ml) was added sodium nitrite (310 mg, 4.5 mmol) in portions. The resulting diazonium salt was transferred to a flask containing compound **2** (1 g, 3.0 mmol) in 20 ml of 3:1 mixture of 10% sodium hydroxide solution and tetrahydrofuran. The resulting reaction mixture was stirred at room temperature for 2 h and extracted with chloroform (3X25 ml). The combined organic extract was washed with water, brine and dried over sodium sulphate. The solvent was removed under reduced pressure and the resulting product was purified on a column of silica gel to give the title compound as thick viscous gum (100 mg, 8%).
¹H NMR (CDCl₃) δ : 0.92 (d, 3H, CH₃), 2.65 (m, 2H, CH₂), 3.1-3.3 (m, 3H, CH₂ and CH), 4.0 (m, 4H, 2XCH₂), 4.95 (m, 3H, CH2 and OH), 6.72-6.81 (m, 2H, Ar-H), 7.4-7.49 (m, 1H, Ar-H), 7.79 (s, 1H, Het-H), 7.91 (s, 1H, Het-H), 7.97 (s, 1H, Het-H).
FAB-MS: 433.2 (MH⁺), calcd. C₁₈H₂₂F₂N₁₀O 432.43.

### Example 21

### Ethyl 4-(2-thiazolyl)piperazine-1-carboxylate:

A mixture of 2-bromothiazole (1.64 g, 10 mmol), ethyl 1-piperazinecarboxylate (1.896, 12 mmol) and sodium iodide (1.498, 10 mmol) in N,N-dimethylformamide (10 ml) was heated at 120°C for 18 h. After cooling, the solvent was removed under reduced pressure and the residue was treated with crushed ice. The desired product was precipitated as colorless solid, was isolated by filtration (2.2 g, 91%).
¹H NMR (CDCl₃) δ: 1.28 (t, 3H, CH₃), 3.48 (m, 4H, 2XCH₂), 3.62 (m, 4H, 2XCH₂), 4.17 (q, 2H, OCH₂), 6.60 (d, 1H, Het-H, J=3.5 Hz), 7.20 (d, 1H, Het-H, J=3.5 Hz)

### 2-(Piperazin-1-ylthiazole:

To a methanolic solution of ethyl 4-(2-thiazolyl)piperazine-1-carboxylate (2 g, 8 mmol), 20 ml of 10% sodium hydroxide solution was added. The resulting mixture was heated under reflux for 5 h. After cooling, the reaction mixture was concentrated under reduced pressure, the residue was diluted with water and extracted with chloroform (3 X 30 ml). The combined extract was washed with water, brine, dried (Na₂SO₄) and concentrated to give the title compound as a thick viscous liquid (1.2 g, 86%).
1H NMR (CDCl₃) δ: 1.68 (br s, 1H, NH), 2.98 (m, 4H, 2XCH₂), 3.46 (m, 2XCH₂), 6.56 (d, 1H, Het-H, J=3.5 Hz), 7.2 (d, 1H, Het-H, J=3.5 Hz).

### (2R,3R)-2-(2,4-Difluorophenyl)-3-[4-(2-thiazolyl)piperazin-1-yl]-1-(1H-1,2,4-triazol-1-yl)butan-2-ol:

To a mixture of (2R,3S)-2-(2,4-difluorophenyl)-3-methyl-2-(1H-1,2,4-triazol-1-yl)methyloxirane **IV** (502 mg, 2 mmol) and lithium perchlorate (320 mg, 3 mmol) in acetonitrile (10 ml), 2-(piperazin-1-yl)thiazole (**X**)(507 mg, 3 mmol) was added. The resulting mixture was heated under reflux for 48 h. The reaction mixture was cooled, concentrated under reduced pressure. The residue was disolved in chloroform, washed with water, brine, dried (Na₂SO₄) and concentrated. The resulting product was purified on a column of silica gel using ethyl acetate and hexane (4:1) as eluent to give the title compound as colorless solid (650 mg, 77%).
m.p.: 180-181 °C.
¹H NMR (CDCl₃) δ: 0.93 (d, 3H, CH₃), 2.57 (m, 2H, CH₂), 3.05 (m, 3H, CH₂ and CH) 3.51 (m, 4H, 2XCH₂), 4.92 (AB q, 2H, CH₂), 5.05 (s, 1H, OH), 6.58 (d, 1H, Het-H, J=3.5 Hz), 6.7-6.8 (m, 2H, Ar-H), 7.2 (d, 1H, Het-H, J=3.5 Hz), 7.4-7.5 (m, 1H, Ar-H), 7.78 (s, 1H, Het-H), 7.93 (s, 1-H, Het-H).
FAB-MS: 421.0 (MH⁺), calcd. C₁₉H₂₂F₂N₆OS 420.482.

### Example 22

### (2R,3R)-2-(2,4-Difluorophenyl)-3-[4-(1H-1,2,4-triazol-3-yl)piperazin-1-yl]-1-(1H-1,2,4,triazol-1-yl)butan-2-ol:

The title compound was prepared in 48% yield from (2R,3S)-2-(2,4-difluorophenyl)-3-methyl-2-(1 H-1,2,4-triazol-1-yl)methyloxirane and 3-(piperazin-1-yl)-1H-1,2,4-triazole (XI) in the presence of lithium perchlorate following the similar procedure described for example **21.**
m.p.: 71-73 °C.
¹H NMR (CDCl₃) δ: 0.91 (d, 3H, CH₃), 2.48-2.60 (m, 2H, CH2), 2.90 -3.03 (m, 3H, CH₂ and CH), 3.32-3.51 (m, 4H, 2XCH₂), 4.81-5.04 (m, 2H, CH₂), 5.20 (br, 2H), 6.67-6.86 (m, 2H, Ar-H), 7.35-7.58 (m, 1H, Ar-H), 7.72 (s, 1H, Het-H), 7.78 (s, 1H, Het-H), 8.01 (s, 1H, Het-H)
FAB-MS: 405.0 (MH⁺), calcd. C₁₈H₂₂F₂N₈O 404.36.

### Example 23

### (2R,3R)-2-(2,4-difluorophenyl)-3-[4-(1H-5-tetrazolyl)piperazin-1-yl]-1-(1H-1,2,4,triazol-1-yl)butan-2-ol:

A mixture of compound 3 (140 mg, 0.39 mmol), sodium azide (31 mg, 0.47 mmol) and ammonium chloride (25 mg, 0.47 mmol) in DMF (5 ml) was heated at 90 °C for 48 h. Solvent was removed under reduced pressure and the residue was extracted with ethyl acetate (3X10 ml). Combined organic extract was washed with water, brine and dried over sodium sulphate. The solvent was removed under reduced pressure and the resulting product was purified on a column of silica gel to give the title compound as a off-white solid (30 mg, 19%).
m.p.: 104-105 °C.
¹H NMR (CDCl₃) δ: 0.93 (d, 3H, CH₃), 2.63 (m, 2H, CH₂), 3.07 (m, 3H, CH₂ and CH), 3.56 (m, 4H, 2XCH₂), 4.93 (AB q and s merged, 3H, CH₂ and OH), 6.70-6.77 (m, 2H, Ar-H), 7.42-7.45 (m, 1H, Ar-H), 7.80 (s, 1H, Het-H), 7.96 (s, 1H, Het-H).
FAB-MS: 406.3 (MH⁺), calcd. C₁₇H₂₁F₂N₉O 405.42

### Example 24

### (2R,3R)-2-(2,4-Difluorophenyl)-3-{4-[2-(4-tert-butylbenzyl)-2H-tetrazol-5-yl]piperazin-1-yl}-1-(1H-1,2,4-triazol-1yl)butan-2-ol:

The title compound was prepared from the epoxide **IV** and tetrazolylpiperazine **XVI** (Ar=4-*tetrt*-butylphenyl) by following the similar procedure described for example **21**.

After usual workup and column purification the title compound was obtained as a colorless solid in 36% yield.
m.p.: 99-101 °C.
¹H NMR (CDCl₃) δ : 0.94 (d, 3H, CH₃), 1.30 (s, 9H, 3XCH₃), 2.47-2.59 (m, 2H, CH2), 2.91-3.01 (m, 3H, CH2 and CH), 3.50 (m, 4H, 2XCH₂), 4.88 (AB q, 2H, CH₂), 5.12 (br s, 1H, OH), 5.53 (s, 2H, CH2), 6.67-6.81 (m, 2H, Ar-H), 7.29-7.48 (m, 5H, Ar-H), 7.79 (s, 1H, Het-H), 7.95 (s, 1H, Het-H).
FAB-MS: 552.2 (MH⁺); calcd. C₂₈H₃₅F₂N₉O 551.61.

### Example 25

### (2R,3R)-2-(2,4-Difluorophenyl)-1-(1H-1,2,4-triazol-1-yl)-3-{4-[2-(4-trifluoromethylbenzyl)-2H-tetrazol-5-yl]piperazin-1-yl}butan-2-ol:

The title compound was prepared from the epoxide **IV** and tetrazolylpiperazine **XVI** (Ar = 4-(trifluoromethyl)phenyl) by following the similar procedure described for example **21**. After usual workup and column purification the title compound was obtained as a colorless solid in 30% yield. m.p.: 71-72 °C.
¹H NMR (CDCl₃) δ : 0.93 (d, 3H, CH₃), 2.51-2.56 (m, 2H, CH₂), 2.96-3.02 (m, 3H, CH₂ and CH), 3.49 (m, 4H, 2XCH₂), 4.90 (AB q, 2H, CH₂), 5.07 (s, 1H, OH), 5.62 (s, 2H, CH₂), 6.68-6.77 (m, 2H, Ar-H), 7.43-7.65 (m, 5H, Ar-H), 7.78 (s, 1H, Het-H), 7.93 (s, 1H, Het-H).
FAB-MS: 564.1 (MH⁺), calcd. C₂₅H₂₆F₅N₉O 563.54.

### Example 26

### (2R,3R)-2-(2,4-Difluorophenyl)-3-{4-[2-(4-tert-butylbenzyl)-2H-1,2,4-triazol-3- yl]piperazin-1-y}-1-(1H-1,2,4-triazol-1yl)butan-2-ol:

The title compound was prepared from the epoxide **IV** and triazolylpiperazine **XII** (Ar = 4*-tert-*butylphenyl) by following the similar procedure described for example **21.** After usual workup and column purification the title compound was obtained as a colorless solid in 45% yield.
m.p.: 73-75 °C.
¹H NMR (CDCl₃) δ : 0.93 (d, 3H), 1.30 (s, 9H), 2.49-2.59 (m, 2H), 2.95-3.07 (m, 3H), 3.12-3.14 (m, 4H), 4.78-4.95 (m, 2H), 5.04 (s, 1H), 5.15 (s, 2H), 6.66-6.80 (m, 2H), 7.12 (d, 2H, J=8.3 Hz), 7.34 (d, 2H, J=8.3 Hz), 7.35-7.49 (m, 1H), 7.69 (s,1H), 7.76 (s, 1H), 7.92 (s, 1H).
FAB-MS: 551.4 (MH⁺), calcd. C₂₉H₃₆F₂N₈O 550.66

### Example 27

### (2R,3R)-2-(2,4-Difluorophenyl)-3-{4-[1-(4-tert-butylbenzyl)-1H-1,2,4-triazol-3-yl]piperazin-1-yl}-1-(1H-1,2,4-triazol-1yl)butan-2-ol:

The title compound was prepared from the epoxide **IV** and triazolylpiperazine **XIII** (Ar = 4-*tert*-butylphenyl) by following the similar procedure described for example **21.** After usual workup and column purification the title compound was obtained as a colorless solid in 46% yield.
m.p.: 84-86 °C.
¹H NMR (CDCl₃): δ: 0.94 (d, 3H, CH₃), 1.30 (s, 9H, 3XCH₃), 2.45-2.55 (m, 2H), 2.83- 3.01 (m, 3H), 3.42-3.48 (m, 4H), 4.79-4.97 (m, 2H), 5.11 (s, 2H), 5.22 (s, 1H), 6.67-6.81 (m, 2H), 7.16 (d, 2H, J=8.2 Hz), 7.36 (d, 2H, J=8.2 Hz), 7.41-7.53 (m, 1H), 7.66 (s, 1H), 7.78 (s, 1H), 7.97 (s, 1H).
FAB-MS: 551.3 (MH⁺), calcd. C₂₉H₃₆F₂N₈O 550.66.

### Example 28

### (2R,3R)-2-(2,4-Difluorophenyl)-1-(1H-1,2,4-triazol-1yl)-3-{4-[1-(4-trifluoromethylbenzyl)-1H-1,2,4-triazol-3-yl]piperazin-1-y}butan-2-ol:

The title compound was prepared from the epoxide **IV** and triazolylpiperazine **XIII** (Ar = 4-(trifluoromethyl)phenyl) by following the similar procedure described for example **21.** After usual workup and column purification the title compound was obtained as a colorless solid in 18% yield.
m.p.: 69-72 °C
¹H NMR (CDCl₃) δ: 0.96 (d, 3H, CH₃), 2.49-2.54 (m, 2H, CH₂), 2.88-2.99 (m, 3H, CH₂ and CH ), 3.43 (m, 4H, 2XCH₂), 4.93 (AB q, 2H, CH₂), 4.98 (2 s merged , 3H, CH₂ and OH), 6.69-6.80 (m, 2H, Ar-H), 7.32-7.64 (m, 5H, Ar-H), 7.78 (2 s merged, 2H, Het-H), 7.98 (s, 1H, Het-H).
FAB-MS: 562.9 (MH⁺), calcd. C₂₀H₂₇F₅N₈O 562.56.

### Example 29

### (2R,3R)-2-(2,4-Difluorophenyl)-3-(4-phenylpiperazin-1-yl)-1-(1H-1,2,4,triazol-1-yl)butan-2-ol:

The title compound was prepared by opening of the epoxide **IV** with 1-phenylpiperazine in the presence of lithium perchlorate by following similar procedure described to example **21.** After column purification the compound was obtained as a colorless solid.
m.p.: 103-105 °C.
1H NMR (CDCl₃) δ: 0.99 (d, 3H, CH₃), 2.6 (m, 2H, CH₂), 2.9-3.05 (m, 3H, CH₂ and CH), 4.89 (AB q, 2H, CH₂), 5.23 (s, 1H, OH), 6.7-7.0 (complex, 5H, Ar-H), 7.2-7.3 (m, 2H, Ar-H), 7.5 (m, 1H, Ar-H), 7.79 (s, 1H, Ar-H), 7.79 (s, 1H, Het-H), 7.97 (s, 1H, Het-H).
FAB-MS: 414.1 (MH⁺), calcd. C₂₂H₂₅F₂N₅O 413.47.

### Example 30

### (2R,3R)-2-(2,4-Difluorophenyl)-3-[4-(4-nitrophenyl)piperazin-1-yl]-1-(1H-1,2,4,triazol-1-yl)butan-2-ol:

The title compound was prepared similarly to example **21** using the oxirane **IV**(600 mg, 2.4 mmol), 4-nitrophenylpiperazine (660 mg, 3.2 mmol) and lithium perchlorate (383 mg, 3.6 mmol) as starting materials. After usual workup, the crude reaction product was purified on a column of silica gel (EtOAc/hexane) to give the desired compound as light yellow solid (920 mg, 84%).
m.p. 148-150 °C
¹H NMR (CDCl₃) δ : 0.94 (d, 3H, CH₃), 2.58-2.64 (m, 2H,), 3.06-3.13 (m, 3H), 3.4-3.5 (m, 4H), 4.93 (AB q, 2H, CH₂), 5.04 (s, 1H, OH), 6.7-6.85 (m and d merged, 4H, Ar-H), 7.38-7.51 (m, 1H, Ar-H), 7.79 (s, 1H, Het-H), 7.91 (s, 1H, Het-H), 8.13 (d, 2H, Ar-H).
FAB-MS: 459.2 (MH⁺), calcd. C₂₂H₂₄F₂N₆O₃ 458.47

### Example 31

### (2R,3R)-3-[4-(4-Aminophenyl)piperazin-1-yl]-2-(2,4-difluorophenyl]-1-(1H-1,2,4,triazol-1-yl)butan-2-ol:

To a solution of nitrocompound **30** (600 mg, 1.3 mmol) in 50 ml of ethyl acetate, 120 mg of 5% platinum on charcoal was added. The reaction mixture was hydrogenated in Parr hydrogenator at room temperature and 45 psi pressure for 18 h. The catalyst was removed by filtration and the filtrate was concentrated in vacuo. The pure amino compound **31** was obtained by dissolving the product in ethyl acetate and precipitating by addition of hexane (530 mg, 95%).
m.p.: 150-151 °C.
¹H NMR (CDCl₃) δ : 0.98 (d, 3H, CH₃), 2.53-2.59 (m, 2H, CH₂), 2.89-3.04 (complex, 7H, 3XCH₂ and CH), 4.87 (AB q, 2H, CH₂), 6.6-6.9 (2d and m merged, 6H, Ar-H), 7.43-7.55 (m, 1H, Ar-H), 7.79 (s, 1H, Het-H), 7.98 (s, 1H, Het-H).
FAB-MS: 429.2 (MH⁺), calcd. C₂₂H₂₆F₂N₆O 428.487.

### Example 32

### (2R,3R)-2-(2,4-Difluorophenyl)-3-[4-(4-ethoxycarbonylaminophenyl)piperazin-1-yl]-1-(1H-1,2,4-triazol-1-yl)butan-2-ol:

To a cooled (0 °C) mixture of amine **31** (300 mg, 0.7 mmol) and triethylamine (0.4 ml) in dichloromethane (30 ml) was added ethyl chloroformate (160 mg, 1.5 mmol) in dichloromethane (5 ml). The reaction mixture was stirred at 0 °C for 2 h, then diluted with 30 ml of chloroform, washed with water, brine and dried over sodium sulphate. The solvent was removed under reduced pressure, the residue was purified on a column of silica gel (hexane/EtOAc, 1:1 and 1:2) to give the title compound as amorphous solid (330 mg, 94%).
m.p. 90-92 °C.
¹H NMR (CDCl₃)δ: 0.99 (d, 3H, CH₃), 1.29 (t, 3H, CH₃, J=7 Hz), 2.58 (m, 2H, CH₂), 2.95 (m, 3H, CH₂ and CH), 4.2 (q, 2H, CH₂, J=7 Hz), 4.88 (AB q, 2H, CH2), 5.21 (s, 1H, OH), 6.44 (s, 1H, NH), 6.7-6.85 (m, 2H, Ar-H), 6.87 (d, 2H, Ar-H, J=8.9 Hz), 7.26 (d, 2H, Ar-H, J=8.9 Hz), 7.41-7.54 (m, 1H, Ar-H), 7.78 (s, 1H, Het-H), 7.97 (s, 1H, Het-H).
FAB-MS: 501.0 (MH⁺), calcd. C₂₅H₃₀F₂N₆O₃ 500.55.

### Example 33

### (2R,3R)-2-(2,4-Difluorophenyl)-3-[4-(4-phenoxycarbonylaminophenyl)piperazin-1-yl]-1-(1H-1,2,4,triazol-1-yl)butan-2-ol:

The title compound was prepared similarly to the above procedure using the amine 31(428 mg, 1 mmol) and phenyl chloroformate (235 mg, 1.5 mmol) in the presence of triethylamine (0.4 ml). After usual workup and purification on a silica gel column, the product was obtained as a colorless solid (475 mg, 89%).
m.p.: 95-97°C.
¹H NMR (CDCl₃) δ : 0.98 (d, 3H, CH3), 2.58 (m, 2H, CH₂), 2.98 (m, 3H, CH₂ and CH), 3.15 (m, 4H, 2XCH₂), 4.88 (AB q, 2H, CH₂), 5.19 (s, 1H, OH), 6.7-7.0 (d and m merged, 4H, Ar-H), 7.2-7.6 (complex, 8H, Ar-H), 7.78 (s, 1H, Het-H), 7.96 (s, 1H, Het-H).
FAB-MS: 549.3 (MH⁺), calcd. C₂₉H₃₀F₂N₆O₃ 548.594.

### Example 34

### (2R,3R)-2-(2,4-Difluorophenyl)-3-{4-[4-(semicarbazid-4-yl)phenyl]piperazin-1-yl}-1-(1H-1,2,4,triazol-1-yl)butan-2-ol:

To a solution of compound **33** (400 mg, 0.73 mmol) in dimethoxyethane (10 ml) was added hydrazine (1 ml) dropwise at room temperature and stirred for 4 h. The reaction mixture was concentrated under reduced pressure and the residue was treated with crushed ice. The precipitated product was isolated by filtration and washed with water and hexane to give the title compound as a colorless solid (300 mg, 85%).
m.p.: 180-182 °C.
¹H NMR (CDCl₃) δ: 0.99 (d, 3H, CH₃), 2.54-2.60 (m, 2H, CH₂), 2.90-3.0 (m, 3H, CH₂ and CH), 3.10 (m, 4H, 2XCH₂), 3.8 (br s, 2H, NH2), 4.88 (AB q, 2H, CH₂), 5.7 (br s, 1H, OH), 6.09 s, 1H, NH), 6.7-6.9 (d and m merged, 4H, Ar-H), 7.3-7.6 (d and m merged, 3H, Ar-H), 7.8 (s, 1H, Het-H), 7.93 (br s, 1H, NH), 8.0 (s, 1H, Het-H).
FAB-MS: 487.0 (MH⁺), calcd. C₂₃H₂₈F₂N₈O₂ 486.527.

### Example 35

### (2R,3R)-2-(2,4-Difluorophenyl)-1-(1H-1,2,4-triazol-1-yl)-3-{4-[4-(2H-1.2.4-triazol-3-one-4-yl)phenyl]piperazin-1-yl}butan-2-ol.

To a mixture of semicarbazide **34** (486 mg, 1 mmol) and formamidine acetate (416 mg, 4 mmol) in methoxyethanol (5 ml), triethylamine (0.8 ml) was added. The reaction mixture was heated at 110 °C for 18 h. Solvent was removed under reduced pressure and the residue was treated with crushed ice, extracted with ethyl acetate (3 X 30 ml). The combined extract was successively washed with water and brine and dried over sodium sulphate. The solvent was removed under reduced pressure, the crude product was purified on a column of silica gel (EtOAc/MeOH, 9:1) to give the triazolone **35** as a crystaline solid (320 mg, 65%).
m.p.: 223-225 °C.
¹H NMR (CDCl₃) δ: 0.98 (d, 3H, CH₃, J=6.4 Hz), 2.57-2.63 (m, 2H, CH₂), 2.95-3.1 (m, 3H, CH₂ and CH), 4.9 (AB q, 2H, CH2), 5.14 (s, 1H, OH), 6.7-6.82 (m, 2H, Ar-H), 6.98 (d, 2H, Ar-H, J=8.9 Hz), 7.37 (d, 2H, J=8.9, Ar-H), 7.4-7.5 (m, 1H, Ar-H), 7.62 (s, 1H, Het-H), 7.79 (s, 1H, Het-H), 7.95 (s, 1H, Het-H).
FAB-MS: 497.0 (MH⁺), calcd. C₂₄H₂₆F₂N₈O₂ 496.522

### Example 36

### (2R,3R)-2-(2,4-Difluorophenyl)-3-[4-{4-[2-(3-pentyl)-2H-1,2,4-triazol-3-one-4-yl]phenyl]}piperazin-1-yl]-1-(1H-1,2,4-triazol-1-yl)butan-2-ol.

To a mixture of triazolone **35** (248 mg, 0.5 mmol) and cesium carbonate (326 mg, 1 mmol) in DMF, 3-bromopentane (226 mg, 1.5 mmol) was added. The reaction mixture was heated at 80 °C for 18 h and concentrated in vacuo. The residue was treated with crushed ice, extracted with ethyl acetate (3 X 30 ml). The combined extract was washed with brine, dried (Na₂SO₄) and the solvent was removed under rduced pressure. The resulting crude product was purified on a column of silica gel (hexane/EtOAc) to give the title compound **36** as a crystaline solid (240 mg, 85%).

The title compound was also prepared in an alternate method in a convergent approach according to **Scheme-1.** Thus a mixture of (2R,3S)-2-(2,4-difluorophenyl)-3-methyl-2-(1 H-1,2,4-triazol-1-yl)methyloxirane **IV,** piperazino compound **XXIII** (R= 3-pentyl) and lithium perchlorate were heated in acetonitrile for 48 h. After usual workup and chromatographic purification, the title compound was obtained in 75% yield.
m.p.: 143-145 °C.
1H NMR (CDCl₃)δ: 0.88 (t, 6H, 2XCH₃), 0.98 (d, 3H, CH₃), 1.65-1.8 (m, 4H, 2XCH₂), 2.60 (m, 2H, CH₂), 3.1 (m, 3H, CH₂ and CH), 3.2 (m, 4H, 2XCH₂), 4.12 (m, 1H, CH), 4.9 (AB q, 2H, CH2), 5.18 (s, 1H, OH), 6.7-6.8 (m, 2H, Ar-H), 6.97 (d, 2H, Ar-H, J=9 Hz), 7.4-7.6 (d and m merged, 3H, Ar-H), 7.64 (s, 1H, Het-H), 7.79 (s, 1H, Het-H), 7.96 (s, 1H, Het-H).
FAB-MS: 567.3 (MH⁺), calcd. C₂₉H₃₆F₂N₈O₂ 566.657.

### Example 37

### (2S,3R)-2-{2,4-Difluorophenyl)-3-[4-{4-[2-(3-pentyl)-2H-1,2,4-triazol-3-one-4-yl]phenyl}piperazin-1-yl]-1-(1H-1,2,4-triazol-1-yl)butan-2-ol.

The title compound was prepared from (2S,3S)-2-(2,4-difluorophenyl)-3-methyl-2-(1H-1,2,4-triazol-1-yl)methyloxirane and piperazino compound **XXIII** (R = 3-pentyl) by following the similar procedure described for the example **36.**
Yield 70%, colorless solid.
m.p.: 83-85 °C.
¹H NMR (CDCl₃) δ: 0.88 (t, 6H, 2XCH₃), 1.25 (d, 3H, CH₃), 1.65-1.84 (m, 4H, 2XCH₂), 2.46 (m, 2H, CH₂), 2.75 (m, 2H, CH₂), 2.93 (m, 4H, 2XCH₂), 3.19 (q, 1H, CH), 4.05 (m, 1H, CH), 4.44 (d, 1H, J=14 Hz), 4.95 (d, 1H, J=14 Hz), 4.98 (s, 1H, OH), 6.64-6.77 (m, 2H, Ar-H), 6.74 (d, 2H, Ar-H), 7.29-7.43 (m, 3H Ar-H), 7.59 (s, 1H, Het-H), 7.77 (s, 1H, Het-H), 7.93 (s, 1H, Het-H).
FAB-MS: 567.2 (MH⁺), calcd. C₂₉H₃₆F₂N₈O₂ 566.657.

### Example 38

### (2S,3S)-2-(2,4-Difluorophenyl)-3-[4-{4-[2-(3-pentyl)-2H-1,2,4-triazol-3-one-4-yl]phenyl}piperazin-1-yl]-1-(1H-1,2,4-triazol-1-yl)butan-2-ol.

The title compound was prepared from (2S,3R)-2-(2,4-difluorophenyl)-3-methyl-2-(1H-1,2,4-triazol-1-yl)methyloxirane and piperazino compound **XXIII** (R=3-pentyl) by following the similar procedure described for the example **36.**
Yield 87%, off-white solid.
m.p.: 218-220 °C.
1H NMR(CDCl₃) δ : 0.88 (t, 6H, 2XCH₃), 0.97 (d, 3H, CH₃), 1.70-1.84 (m, 4H, 2XCH₂), 2.63 (m, 2H, CH₂), 3.01 (m, 3H, CH₂ and CH), 3.22 (m, 4H, 2XCH₂), 4.12 (m, 1H, CH), 4.90 (AB q, 2H, CH₂), 5.14 (s, 1H, OH), 6.69-6.82 (m, 2H, Ar-H), 6.96 (d, 2H, Ar-H), 7.40-7.49 (m and d merged, 3H, Ar-H), 7.63 (s, 1H, Het-H), 7.79 (s, 1H, Het-H), 7.95 (s, 1H, Het-H).
FAB-MS: 567.4 (MH⁺), calcd. C₂₉H₃₆F₂N₈O₂ 566.657.

### Example 39

### (2R,3S)-2-(2,4-Difluorophenyl)-3-[4-{4-[2-(3-pentyl)-2H-1,2,4-triazol-3-one-4-yl]phenyl}piperazin-1-yl]-1-(1H-1,2,4-triazol-1-yl)butan-2-ol.

The title compound was prepared from (2R,3R)-2-(2,4-difluorophenyl)-3-methyl-2-(1H-1,2,4-triazol-1-yl)methyloxirane and piperazino compound **XXIII** (R= 3-pentyl) by following the similar procedure described for the example **36.**
Yield 60%, colorless solid.
m.p.: 110-113 °C.
¹HNMR (CDCl₃) δ : 0.87 (t, 6H, 2XCH₃), 1.25 (d, 3H, J=7 Hz), 1.69-1.87 (m, 4H, 2XCH₂), 2.46 (m, 2H, CH₂), 2.75-2.95 (m, 6H, 3XCH₂), 3.19 (q, 1H, J=7 Hz), 4.07 (m, 1H, CH), 4.45 (d, 1H, J=15 Hz), 4.94 (d, 1H, J=15 Hz), 5.03 (s, 1H, OH), 6.66-6.76 (m, 2H, Ar-H), 6.87 (d, 2H, Ar-H), 7.34-7.39 (d and m merged, 3H, Ar-H), 7.60 (s, H, Het-H), 7.77 (s, 1H, Het-H), 7.93 (s, 1H, Het-H).
FAB-MS: 567.1 (MH⁺), calcd. C₂₉H₃₆F₂N₈O₂ 566.657.

### Example 40

### (2R,3R)-3-[4-{4-[2-(2-Butyl)-2H-1,2,4-triazol-3-one-4-yl]phenyl}piperazin-1-yl]-2-(2,4-difluorophenyl)-1-(1H-1,2,4-triazol-1-yl)butan-2-ol.

The example **40** was prepared similarly to the above procedure by alkylation of triazolone **35** with 2-bromobutane in the presence of cesium carbonate. After column chromatography the product was obtained as a colorless solid. Yield 88%.
m.p.: 156-157 °C.
¹H NMR (CDCl₃) δ : 0.90 (t, 3H, CH₃), 0.98 (d, 3H, CH₃), 1.6-1.9 (m, 2H, CH₂), 2.60 (m, 2H, CH₂), 3.0 (m, 3H, CH₂ and CH), 3.22 (m, 4H, 2XCH₂), 4.29 (m, 1H, CH), 4.9 (AB q, 2H, CH₂), 5.14 (S, 1H, OH), 6.65-6.85 (m, 2H, Ar-H), 6.96 (d, 2H, Ar-H, J=8.9 Hz), 7.5 (m, 1H, Ar-H), 7.61 (s, 1H, Het-H), 7.79 (s, 1H, Het-H), 7.95 (s, 1H, Het-H).
FAB-MS: 553.1 (MH⁺), calcd. C₂₈H₃₄F₂N₈O₂ 552.573.

### Example 41

### (2R,3R)-2-(2,4-Difluorophenyl)-3-[4-{4-[2-(2-propyl)-2H-1,2,4-triazol-3-one-4-yl]phenyl}piperazin-1-yl]-1-(1H-1,2,4-triazol-1-yl)butan-2-ol.

The title compound **41** was prepared by alkylation of triazolone **35** with 2-bromopropane in the presence of cesium carbonate following the procedure similar to the above described for example **36.**
Yield 80%.
m.p.: 166-167 °C.
¹H NMR (CDCl₃) δ: 0.97 (d, 3H, CH₃), 1.40 (d, 6H, 2XCH₃), 2.60 (m, 2H, CH2), 3.0 (m, 3H, CH₂ and CH), 3.22 (m, 4H, 2XCH₂), 4.55 (m, 1 H, CH), 4.9 (AB q, 2H, CH2), 5.15 (s, 1H, OH), 6.7- 6.85 (m, 2H, Ar-H), 6.96 (d, 2H, Ar-H, J=9 Hz), 7.38 (d, 2H, Ar-H, J=9 Hz), 7.5 (m, 1H, Ar-H), 7.59 (s, 1H, Het-H), 7.79 (s, 1H, Het-H), 7.95 (s, 1H, Het-H).
FAB-MS: 539 (MH⁺), calcd. C₂₇H₃₂F₂N₈O₂ 538.6.

### Example 42

### (2R,3R)-2-(2,4-Difluorophenyl)-3-[4-{4-[2-(2-hydroxypropyl)-2H-1,2,4-triazol-3-one-4-yl]phenyl}piperazin-1-yl]-1-(1H-1,2,4-triazol-1-yl)butan-2-ol.

To a mixture of triazolone **35** (248 mg, 0.5 mmol) and potassium carbonate (138 mg, 1 mmol) in DMF (6 ml), 1,2-epoxypropane (870 mg, 15 mmol) was added. The reaction mixture was heated at 60 °C for 18 h and concentrated in vacuo. The residue was treated with crushed ice, extracted with ethyl acetate (3 X 30 ml). The combined extract was washed with brine, dried (Na₂SO₄) and the solvent was removed under reduced pressure. The resulting crude product was purified on a column of silica gel (EtOAc/MeOH, 9:1) to give the hydoxypropyltriazolone **42** as a colorless solid (200 mg, 72%).
m.p.: 110-112 °C (decomp).
¹H NMR (CDCl₃) δ : 0.97 (d, 3H, CH3), 1.28 (d, 3H, CH₃), 2.60 (m, 2H, CH₂), 2.9-3.1 (m and q merged, 3H, CH₂ and CH), 3.23 (m, 4H, 2XCH₂), 3.7-4.0 (m, 2H, CH₂), 4.8-5.0 (AB q, 2H, CH2), 5.12 (s, 1H, OH), 6.65-6.85 (m, 2H, Ar-H), 6.96 (d, 2H, Ar-H, J=9 Hz), 7.37 (d, 2H, Ar-H, J=9 Hz), 7.5 (m, 1H, Ar-H), 7.63 (s, 1H, Het-H), 7.78 (s, 1H, Het-H), 7.94 (s, 1H, Het-H).
FAB-MS: 555.3 (MH⁺), calcd. C₂₇H₃₂F₂N₈O₃ 554.60

### tert-Butyl 4-(4-nitrophenyl)piperazine-1-carboxylate (XVII: X=H):

To a solution of 1-(nitrophenyl)piperazine (20.7,0.1 mol) and triethylamine (21 ml) in dichloromethane (250 ml) at 0-5 °C was added dropwise a solution of di-*tert*-butyldicarbonate in dichloromethane (50 ml). The resulting mixture was stirred at 0-5 °C for 2 h and at room temperature for 18 h. Then the reaction mixture was diluted with 100 ml of chloroform, washed with water, brine and dried over sodium sulphate. The solvent was removed under reduced pressure and the residue was triturated with hexane and hexanelethyl acetate mixture to give the title compound as a yellow solid (29 g, 94%).
¹H NMR (CDCl₃) δ: 1.5 (s, 9H, 3XCH₃), 3.43 (t, 4H, 2XCH₂), 3.6 (t, 4H, 2XCH₂), 6.8 (d, 2H, Ar-H, J=9 Hz), 8.15 (d, 2H, Ar-H, J=9 Hz).

### tert-Butyl 4-(4-Aminophenyl)piperazine-1-carboxylate (XVIII; X=H):

A solution of butyl 4-(4-nitrophenyl)piperazine (60 g, 0.195 mol) in ethyl acetate (800 ml) was hydrogenated in the presence of 10% palldium on charcoal (6.0) at room temperature and 45 psi pressure in the Parr hydrogenator for 18 h. The catalyst was removed by filtration and the filtrate was concentrated in vacuo to give the title compound as a offwhite solid (50 g, 93%).
¹H NMR (CDCl₃) δ: 1.49 (s, 9H, 3XCH3), 3.0 (t, 4H, 2XCH₂), 3.4 (br s, 2H, NH₂), 3.5 (t, 4H, 2XCH₂), 6.65 (d, 2H, Ar-H), 6.85 (d, 2H, Ar-H).

### tert-Butyl 4-(4-Phenoxycarbonylaminophenyl)piperazine-1-carboxylate (XIX; X=H):

To a solution of *tert*-butyl 4-(4-aminophenyl)piperazine-1-carboxylate (50 g, 0.18 mol) and triethylamine (39 ml, 0.27 mol) in dichloromethane (400 ml) was added dropwise a solution of phenyl chloroformate (36.65 g, 0.23 mol) in dichloromethane (100 ml) at 0 oC. The resulting reaction mixture was stirred for 2 h at 0 °C and additional 3 h at room temperature. Then diluted with chloroform (100 ml), washed with water, brine and dried over sodium sulphate. The solvent was removed under reduced pressure, the crude product was purified on a column of silica gel (hexane/EtOAc, 1:1) to give the tiltle compound as colorless solid (60 g, 84%).
m.p.: 158-160 °C.
¹H NMR (CDCl₃) δ: 1.48 (s, 9H, 3XCH₃), 3.0-3.2 (m, 4H, 2XCH₂), 3.6 (m, 4H, 2XCH₂), 6.8 (brs, 1H, NH), 6.92 (d, 2H, Ar-H), 7.1-7.5 (complex, 7H, Ar-H).

### 4-[4-(4-t-BOC-piperazin-1-yl)phenylsemicarbazide (XX: X=H).

To a solution of *tert*-butyl 4-(phenoxycarbonylaminophenyl)piperazine-1-carboxylate (36g, 90 mmol) in dimethoxyethane (300 ml), anhydrous hydrazine (40 g, 1.25 mol) was added. The resulting reaction mixture was stirred at room temperature for 3-5 h. Solvent was removed under reduced pressure and the residue was treated with crushed ice and left overnight at room temperature. The precipitated solid was collected, washed with water and hexane to give the title compound as a offwhite solid (25 g, 82%).
¹H NMR (CDCl₃) δ: 1.48 (s, 9H, 3XCH₃), 3.06 (t, 4H, 2XCH₂), 3.58 (t, 4H, 2XCH₂), 3.82 (s, 2H, NH2), 5.98 (s, 1H, NH), 6.89 (d, 2H, Ar-H, J=8.9 Hz), 7.36 (d, 2H, Ar-H, J=8.9 Hz), 7.95 (s, 1H, NH).

### 4[4-(4-t-BOC-Piperazin-1-yl)phenyl-2H-1,2,4-triazol-3-one (XXI; X=H):

A mixture of 4-[4-(4-t-BOC-piperazin-1-yl)phenylsemicarbazide (25 g, 75 mmol), formamidine acetate (31.2 g, 300 mmol) and triethylamine (50.5 ml, 360 mmol) in methoxyethanol (250 ml) was heated at 110 °C for 18 h. The solvent was removed under reduced pressure, the residue was treated with crushed ice and extracted with ethyl acetate (3X250 ml). Combined extract was successively washed with water and brine and dried over sodium sulphate. The solvent was removed under reduced pressure and the product was purified on a column of silica gel to give the title compound as a colorless solid (16.7 g, 65%).
m.p.: 195-197 °C.
¹H NMR (CDCl₃) δ: 1.48 (s, (H, 9H, 3XCH₃), 3.16 (t, 4H, 2XCH₂), 3.59 (t, 4H, 2XCH₂), 6.98 (d, Ar-H, J=9 Hz), 7.38 (d, 2H, Ar-H), 7.62 (s, 1H, Het-H), 9.7 (brs, 1H, NH).

### 2-(3-Pentyl)-4[4-(4-t-BOC-piperazin-1-yl)phenyl-2H-1,2,4-triazol-3-on e (XXII; X=H, R= 3-pentyl):

A mixture of 4[4-(4-*t*-BOC-piperazin-1-yl)phenyl-2H-1,2,4-triazol-3-one **XXI** (3.45 g, 10 mmol), 3-bromopentane (4.53 g) and potassium carbonate (2.76 g, 20 mmol) in DMF (30 ml) was heated at 80 °C for 18 h. The solvent was removed under reduced pressure, the residue was diluted with water, extracted with ethyl acetate (3X50 ml). The combined extract was washed with water, brine, dried (Na₂SO₄) and concentrated in vacuo. The resulting product was purified on a column of silica gel (2.9 g, 70%) to give the title compound as a colorless solid.
m.p.: 96-97 °C.
¹H NMR (CDCl₃) δ: 0.88 (t, 6H, 2XCH₃), 1.48 (s, 9H, 3XCH₃), 1.7-1.9 (m, 4H, 2XCH₂), 3.15 (t, 4H, 2XCH₂), 3.59 (t, 4H, 2XCH₂), 4.05 (m, 1H, CH), 6.98 (d, 2H, Ar-H, J=9 Hz), 7.44 (d, 2H, Ar-H, J=9 Hz), 7.63 (s, 1H, Het-H).

### 2-(3-Pentyl)-4-[4-(piperazin-1-yl)phenyl]-2H-1,2,4-triazol-3-one (XXIII: X=H: R=3-pentyl):

To a solution of 2-(3-pentyl)-4-[4-(4-t-BOC-piperazin-1-yl)phenyl-1H-1,2,4-triazol-3-one (2.5 g, 6 mmol) in ethyl acetate (30 ml), 30 ml of 10% hydrochloric acid was added. The resulting heterogenious mixture was stirred at room temperature for 5 h. Solvent was removed under reduced pressure, the residue was diluted with water, basified with potassium carbonate and extracted with chloroform (3X50 ml). The combined extract was washed with water, brine, dried (Na₂SO₄) and concentrated to give the title compound as colorless solid (1.8 g, 95%).
m.p.: 135-137 °C.
¹H NMR (CDCl₃) δ: 0.88 (t, 6H, 2XCH₃), 1.7-1.9 (m, 4H, 2XCH₂), 3.07 (m, 4H, 2XCH₂), 3.18 (m, 4H, 2XCH₂), 4.05 (m, 1H, CH), 6.95 (d, 2H, Ar-H), 7.42 (d, 2H, Ar-H), 7.63 (s, 1H, Het-H).

### 2-(2-Butyl)-4[4-(4-t-BOC-piperazin-1-yl)phenyl]-2H-1,2,4-triazol-3-on e (XXII: X=H, R=2-butyl):

The title compound was obtained by alkylation of triazolone **XXI** with 2-bromobutane in the presence of potassium carbonate.
m.p.: 134-135 °C.
¹H NMR (CDCl₃) δ: 0.90 (t, 3H, CH₃), 1.39 (d, 3H, CH₃), 1.48 (s, 9H, 3XCH₃), 1.8 (m, 2H, CH2), 3.15 (t, 4H, 2XCH₂), 3.59 (t, 4H, 2XCH₂), 4.3 (m, 1H, CH), 6.97 (d, 2H, Ar-H, J=8.9 Hz), 7.42 (d, 2H, Ar-H, J=8.9 Hz), 7.61 (s, 1H, Het-H).

### 2-(2-Butyl)-4-[4-(piperazin-1-yl)phenyl]-2H-1,2,4-triazol-3-one (XXIII; X=H, R=2-butyl):

The title compound was obtained by removal of *t*-BOC group of 2-(2-butyl)-4[4-(4-*t*-BOC-piperazin-1-yl)phenyl]-2H-1,2,4-triazol-3-one with 3M hydrochloric acid.
m.p.: 91-93 °C.
¹H NMR (CDCl₃) δ: 0.90 (t, 3H, CH₃), 1.39 (d, 3H, CH₃), 3.04 (m, 4H, 2XCH₂), 3.17 (m, 4H, 2XCH₂), 4.29 (m, 1H, CH), 6.98 (d, 2H, Ar-H, J=8.9 Hz), 7.4 (d, 2H, Ar-H, J=8.9 Hz), 7.61 (s, 1H, Het-H).

### 4-[4-(4-t-BOC-Piperazin-1-yl)phenyl]-2-(2-propyl)-2H-1,2,4-triazol-3-one (XXII: X=H, R=2-propyl):

The title compound was obtained by alkylation of triazolone **XXI** with 2-bromopropane in the presence of potassium carbonate.
m.p: 166-167 °C.
¹H NMR (CDCl₃) δ: 1.41 (d, 6H, 2XCH₃), 1.49 (s, 9H, 3XCH₃), 3.16 (t, 4H, 2XCH₂), 3.59 (2XCH₂), 4.55 (m, 1H, CH), 6.97 (d, 2H, Ar-H, J=8.9 Hz), 7.40 (d, 2H, Ar-H, J=8.9 Hz), 7.59 (s, 1H, Het-H).

### 4-[4-(Piperazin-1-yl)phenyl]-2-(2-pyropyl)-2H-1,2,4-triazol-3-one (XXIII:X=H, R=2-propyl):

The title compound was obtained by removal of *t*-BOC group of 4-[4-(4-t-BOC-piperazin-1-yl)phenyl]-2-(2-propyl)-2H-1,2,4-triazol-3-one with 3M hydrochloric acid.
m.p.: 120-121 °C.
¹H NMR (CDCl₃) δ : 1.41 (d, 6H, 2XCH₃), 3.04 (m, 4H, 2XCH₂), 3.17 (m, 4H, 2XCH₂), 4.55 (m, 1H, CH), 6.97 (d, 2H, Ar-H, J=9 Hz), 7.38 (d, 2H, Ar-H, J=9 Hz), 7.59 (s, 1H, Het-H).

### 2-(2-Hydroxyrpropyl)-4-[4-(4-t-BOC-piperazin-1-yl)phenyl]-2H-1,2,4-triazol-3-one (XXII: X=H, R=2-hydroxypropyl):

The title compound was obtained by alkylation of triazolone **XXI** with 1,2-epoxypropane in the presence of potassium carbonate
m.p: 168-170 °C.
¹H NMR (CDCl₃) δ: 1.28 (d, 3H, CH₃), 1.48 (s, 9H, 3XCH₃), 3.17 (t, 4H, 2XCH₂), 3.4 (brs, 1H, OH), 3.59 (t, 4H, 2XCH₂), 3.70 (dd, 1H), 3.96 (dd, 1H), 4.2 (m, 1H, CH), 6.98 (d, 2H, Ar-H, J=8.9 Hz), 7.39 (d, 2H, Ar-H, J=8.9 Hz), 7.63 (s, 1H, Het-H).

### 2-(2-Hydroxypropyl)-4-[4-(piperazin-1-yl)phenyl]-2H-1,2,4-triazol-3-one (XXIII; X=H, R=2-hydroxypropyl):

The title compound was obtained by removal of *t*-BOC group of 2-(2-hydroxypropyl)-4-(4-(4-*t*-BOC-piperazin-1-yl)phenyl]-2H-1,2,4-triazol-3-one with 3M hydrochloric acid.
¹H NMR (CDCl₃) δ: 1.28 (d, 3H, CH₃), 3.0 (m, 4H, 2XCH₂), 3.2 (m, 4H, 2XCH₂), 3.8-4.0 (m, 2H, CH2), 4.2 (m, 1H, CH), 6.98 (d, 2H, Ar-H, J=9 Hz), 7.3 (d, 2H, Ar-H, J=9 Hz), 7.62 (s, 1H, Het-H)

### Example 43

### 4-[4-(4-t-BOC-Piperazin-1-yl)phenyl]-2-[(4-trifluoromethyl)benzyl]-2H-1,2,4-triazol-3-one (XXII; X=H, R=4-trifluoromethylbenzyl):

The title compound was prepared by alkylation of triazolone **XXI** (X=H) with 4-(trifluoromethyl)benzyl bromide in the presence of potassium carbonate. After usual workup and purification on a column of silica gel, the title compound was obtained in 95% yield as a colorless solid.
¹H NMR (CDCl₃) δ: 1.49 (s, 9H, 3 x CH₃); 3.16 (t, 4H, 2 x ₂); 3.59 (t, 4H, 2 x CH₂); 5.06 (s, 2H, CH₂); 6.98 (d, 2H, J = 8.9 Hz, Ar-H), 7.39 (d, 2H, J = 8.9 Hz, Ar-H); 7.5-7.60 (m, 6H, Ar-H); 7.64 (s, 1H, Het-H).

### 4-[4-(Piperazin-1-yl)phenyl]-2-[(4-trifluoromethyl)benzyl]-2H-1,2,4-triazol-3-one (XXIII; X=H, R=4-trifluoromethylbenzyl):

The title compound was obtained by removal of *t*-BOC group of 4-[4-(4-*t*-BOC-piperazin-1-yl)phenyl]-2-[(4-trifluoromethyl)benzyl]-2H-1,2,4-triazol-3-one with 3M hydrochloric acid. After usual workup the product was obtained as a colorless solid in 95% yield.
¹H NMR (DMSO-*d*₆) δ : 2.81-2.84 (m, 4H, 2 x CH₂); 3.04-3.07 (m, 4H, 2 x CH₂); 5.05 (s, 2H, CH₂); 7.0 (d, 2H, J = 9 Hz, Ar-H); 7.43-7.53 (m, 4H, Ar-H); 7.73 (d, 2H, J = 8 Hz, Ar-H); 8.38 (s, 1H, Het-H).

### (2R,3R)-2-(2,4-Difluorophenyl) -1-(1H-1,2,4-triazol-1-yl)-3-[4-{4-[2-(4-trifluoromethylbenzyl)-2H-1,2,4-triazol-3-one-4-yl]phenyl]}piperazin-1-yl]butan-2-ol (43):

The title compound was prepared from (2R,3S)-2-(2,4-difluorophenyl)-3-methyl-2-(1H-1,2,4-triazol-1-yl)methyloxirane **IV** and piperazino compound **XXIII** (R= 4-trifluoromethylbenzyl; X=H) by following the similar procedure described for the example **36.**
Colorless solid, 189-191 °C, yield 65%.
¹H NMR (CDCl₃) δ: 0.98 (d, 3H, J = 5.5 Hz, CH₃), 2.57-2.63 (m, 2H, CH₂); 3.0-3.05 (m, 3H); 3.08 -3.23 (m, 4H, 2 x CH₂); 4.81-4.99 (q, 2H, CH₂); 5.07 (s, 2H, CH₂); 5.15 (s, 1H); 6.68-7.64 (m, 12H); 7.79 (s, 1H, Het-H); 7.95 (s, 1H, Het-H).
FAB-MS: 657.3 (MH⁺); calcd. C₃₂H₃₁O₂F₅N₈ 656.64.

### Example 44

### 4-[4-(4-tert-BOC-Piperazin-1-yl)phenyl]-2-(2,2,3,3-tetrafluoropropyl)-2H-1,2,4-triazol-3-one (XXII; X=H, R=2,2,3,3-tetrafluoropropyl):

The title compound was obtained by alkylation of triazolone **XXI** (X=H) with 2,2,3,3-tetrafluropropyl methanesulphonate in the presence of potassium carbonate. The product was obtained as light yellow solid in quantitative yield.
¹H NMR (CDCl₃) δ: 1.48 (s, 9H, 3 x CH₃); 3.18 (t, 4H, 2 x CH₂); 3.59 (t, 4H, 2 x CH₂); 4.34-4.48 (m, 2H) 5.71-6.28 (m, 1H, CHF₂); 6.98 (d, 2H, Ar-H) 7.39 (d, 2H, Ar-H); 7.69 (s, 1H, Het-H).

### 4-[4-(Piperazin-1-yl)phenyl]-2-(2,2,3,3-tetrafluoropropyl)-2H-1,2,4-triazol-3-one (XXIII; X=H, R=2,2,3,3-tetrafluoropropyl):

The title compound was obtained by the deprotection of the BOC group of the above compound with 3N hydrochloric acid. The product was obtianed as light yellow solid in quantitative yield. This was used in the following step without further purification.
¹H NMR (CDCl₃) δ: 3.01-3.21 (m, 8H); 4.33-4.62 (m, 3H, CH₂ and N-H); 5.67-6.29 (m, 1H, CHF₂); 6.97 (d, 2H, Ar-H); 7.37 (d, 2H, Ar-H); 7.70 (s, 1H, Het-H).

### 2R.3R)-2-(2.4-Difluorophenyl)-3-[4-{4-[2-(2,2,3,3-tetrafluoropropyl)-2H-1,2,4-triazol-3-one-4-yl]phenyl}piperazin-1-yl]-1-(1H-1,2,4-triazol-1-yl)butan-2-ol.

The title compound was prepared from (2R,3S)-2-(2,4-difluorophenyl)-3-methyl-2-(1H-1,2,4-triazol-1-yl)methyloxirane **IV** and piperazino compound **XXIII** (R=2,2,3,3,-tetrafluoropropyl; X=H) by following the similar procedure described for the example **36.**
Colorless solid, Yield 55%.
m.p: 155-157 °C.
¹H NMR (CDCl₃) δ: 0.97 (d, 3H, J = 5.4 Hz, CH₃); 2.57-2.65 (m, 2H, CH₂); 3.00-3.23 (m, 7H); 4.35-4.48 (m, 2H, CH₂); 4.82-5.00 (m, 2H, CH₂); 5.13 (s, 1H); 5.71-6.28 (m, 1H, -CHF₂); 6.69-6.83 (m, 2H, Ar-H); 6.96 (d, 2H, J = 9 Hz; Ar-H); 7.35-7.53 (m, 3H, Ar-H); 7.68 (s, 1H, Het-H); 7.79 (s, 1H, Het-H); 7.95 (s, 1H, Het-H).
FAB-MS: 611.2 (MH⁺), calcd. C₂₇H₂₈O₂F₆N₈ 610.56.

### Example 45

### 4-[4-(4-t-BOC-Piperazin-1-yl)phenyl]-2-(2,2,2-trifluoroethyl)-2H-1,2,4-triazol-3-one (XXII: X=H, R=2,2,2-trifluoroethyl):

The title compound was prepared from the reaction of triazolone XXI (X=H) with 2,2,2-trifluroethyl bromide in a sealed vessel in the presence of potassium carbonate. After usual workup and purification on a column of silica gel, the alkylated compound was obtained as a colorless solid in 21% yield.
¹H NMR (CDCl₃) δ: 1.49 (s, 9H, 3 x CH₃); 3.18 (t, 4H, 2 x CH₂); 3.59 (t, 4H, 2 x CH₂); 4.44 (q, 2H, CH₂); 6.98 (d, 2H, J = 7 Hz, Ar-H); 7.38 (d, 2H, J = 7 Hz, Ar-H); 7.69 (s, 1H, Het-H).

### 4-[4-(Piperazin-1-yl)phenyl]-2-(2,2,2-trifluoroethyl)-2H-1,2,4-triazol-3-one (XXIII: X=H, R=2,2,2-trifluoroethyl):

The title compound was obtained by deprotection of BOC group of the above compound with 3M hydrochloric acid.
¹H NMR (CDCl₃) δ: 1.73 (s, 1H, N-H); 3.00-3.20 (m, 8H, 4 x CH₂); 4.44 (q, 2H, CH₂); 6.95-7.00 (m, 2H, Ar-H); 7.34-7.39 (m, 2H, Ar-H); 7.68 (s, 1H, Het-H).

### (2R,3R)-2-(2,4-Difluorophenyl)-1-(1H-1,2,4-triazol-1-yl)-3-[4-{4-[2-(2,2,2-trifluoroethyl)-2H-1,2,4-triazol-3-one-4-yl]phenyl]}piperazin-1-yl]butan-2-ol.

The title compound was prepared from (2R,3S)-2-(2,4-difluorophenyl)-3-methyl-2-(1H-1,2,4-triazol-1-yl)methyloxirane **IV** and piperazino compound **XXIII** (R= 2,2,2-trifluoroethyl, X=H) by following the similar procedure described for the example **36.**
Colorless solid, yield: 59%.
m.p: 95-97 °C.
¹H NMR (CDCl₃) δ: 0.90 (d, 3H, J = 6.3 Hz, CH₃); 2.50-2.56 (m, 2H, CH₂); 2.93-3.16 (m, 7H); 4.37 (q, 2H, CH₂); 4.83 (m, 2H, CH₂); 6.61-6.91 (m, 4H, Ar-H); 7.27-7.46 (m, 3H, Ar-H); 7.61 (s, 1H, Het-H); 7.71 (s, 1H, Het-H); 7.88 (s, 1H, Het-H).
FAB-MS : 579.2 (MH⁺), calcd. C₂₆H₂₇O₂F₅N₈ 578.55.

### Example 46

### 2-(2,4-Difluorobenzyl)-4-[4-(4-tert-BOC-piperazin-1-yl)-2H-1,2,4-triazol-3-one (XXII; X=H, R=2,4-difluorobenzyl):

The title compound was prepared by alkylation of triazolone **XXI** (X=H) with 2,4-difluorobenzyl bromide in the presence of potassium carbonate. After usual workup the product was obtained in quantitative yield as a colorless solid.
¹H NMR (CDCl₃) δ : 1.49 (3, 9H, 3 x CH₃); 3.17 (t, 4H, 2 x CH₂); 3.59 (t, 4H, 2 x CH₂); 5.04 (s, 2H, CH₂); 6.78-7.01 (m, 4H, Ar-H); 7.29-7.45 (m, 3H, Ar-H); 7.63 (s, 1H, Het-H).

### 2-(2,4-Difluorobenzyl)-4-[4-(4-piperazin-1-yl)-2H-1,2,4-triazol-3-one (XXIII: X=H, R=2,4-difluorobenzyl):

The title compound was obtained by deprotection of BOC group of the above compound with 3N hydrochloric acid.
Colorless solid, 70% yield.
¹H NMR (CDCl₃) δ: 1.70 (s, 1H, N-H); 2.92-3.10 (m, 8H, 4 x CH₂); 4.96 (s, 2H, CH₂); 6.71-6.91 (m, 4H, Ar-H); 7.21-7.37 (m, 3H, Ar-H); 7.54 (s, 1H, Het-H).

### (2R,3R)-3-[4-{4-[2-{2,4-Difluorobenzyl)-2H-1,2,4-triazol-3-one-4-yl]phenyl]}piperazin-1-yl]-2-(2,4-difluorophenyl)-1-(1H-1,2,4-triazol-1-yl)butan-2-ol.

The title compound was prepared from (2R,3S)-2-(2,4-difluorophenyl)-3-methyl-2-(1H-1,2,4-triazol-1-yl)methyloxirane and piperazino compound **XXIII** (R= 2,4-difluorobenzyl, X=H) by following the similar procedure described for the example **36**.
Colorless solid, yield 53%
m.p.: 161-163 °C.
¹H NMR (CDCl₃) δ: 0.91 (d, 3H, J = 5.6 Hz, CH₃); 2.49-2.57 (m, 2H, CH₂); 2.91-3.14 (m, 7H); 4.73-4.91 (m, 2H, CH₂); 4.97 (s, 2H, CH₂); 5.09 (s, 1H); 6.60-6.90 (m, 6H, Ar-H); 7.25-7.45 (m, 4H, Ar-H); 7.54 (s, 1H, Het-H); 7.70 (s, 1H, Het-H); 7.87 (s, 1H, Het-H).
FAB-MS: 623.1 (MH⁺); calcd. C₃₀H₃₀ F₄O₂N₈ 622.63.

### Example 47

### 4-[4-(4-tert-BOC-Piperazin-1-yl)phenyl-2-(4-trifluoromethoxy)benzyl-2H-1,2,4-triazol-3-one (XXII; X=H, R=4-trifluoromethoxybenzyl):

The title compound was prepared by alkylation of triazolone **XXI** (X=H) with 4-(trifluoromethoxy)benzyl bromide in the presence of potassium carbonate. After usual workup the product was obtained in 90% yield as a colorless solid.
¹H NMR (CDCl₃) δ: 1.49 (s, 9H, 3 x CH₃), 3.13-3.18 (m, 4H, 2 x CH₂); 3.56-3.61 (m, 4H, 2 x CH₂); 5.00 (s, 2H, CH₂); 6.94-6.99 (m, 2H, Ar-H); 7.17-7.22 (m, 2H, Ar-H) 7.38-7.47 (m, 4H, Ar-H); 7.63 (s, 1H, Het-H).

### 4-[4-(Piperazin-1-yl)phenyl-2-(4-trifluoromethoxy)benzyl-2H-1,2,4-triazol-3-one (XXIII; X=H, R=4-trifluoromethoxybenzyl):

The title compound was obtained by removal of *t*-BOC group of the aboove compound with 3M hydrochloric acid.
Colorless solid, quantitative yield.
¹H NMR (CDCl₃) δ: 1.64 (s, 1H, N-H); 3.01-3.20 (m, 8H, 4 x CH₂); 5.01 (s, 2H, CH₂); 6.95-6.99 (m, 2H, Ar-H); 7.18-7.22 (m, 2H, Ar-H), 7.34-7.47 (m, 4H, Ar-H); 7.61 (s, 1H, Het-H).

### (2R,3R)-2-(2,4-Difluorophenyl)-3-[4-{4-[2-(4-trifluoromethoxybenzyl)-2H-1,2,4-triazol-3-one-4-yl]phenyl}piperazin-1-yl]-1-(1H-1,2,4-triazol-1-yl)butan-2-ol:

The title compound was prepared from (2R,3S)-2-(2,4-difluorophenyl)-3-methyl-2-(1H-1,2,4-triazol-1-yl)methyloxirane and piperazino compound **XXIII** [R=4-(trifluoromethoxy)benzyl; X=H] by following the similar procedure described for the example **36.**
Colorless solid, 91% yield
m.p.: 175-177 °C.
¹H NMR (CDCl₃) δ: 0.98 (d, 3H, J = 5.7 Hz, CH₃); 2.57-2.65 (m, 2H, CH₂); 2.99-3.22 (m, 7H); 4.82-4.92 (m, 2H, CH₂) 5.01 (s, 2H, CH₂); 5.15 (s, 1H); 6.68-7.54 (m, 11H, Ar-H); 7.62 (s, 1H, Het-H); 7.79 (s, 1H, Het-H); 7.95 (s, 1H, Het-H).
FAB-MS: 671.3 (MH⁺), calcd. C₃₂H₃₁O₃F₅N₈ 670.64.

### Example 48

### 2-(4-Methoxybenzyl)-4-[4-(4-tert-BOC-piperazin-1-yl)phenyl-2H-1,2,4-triazol-3-one (XXII; X=H, R=4-methoxybenzyl):

The title compound was prepared by alkylation of triazolone **XXI** (X=H) with 4-methoxybenzyl bromide in DMF in the presence of potassium carbonate.
Colorless solid, 96% yield.
¹H NMR (CDCl₃) δ: 1.49 (s, 9H, 3 x CH₃); 3.12-3.17 (m, 4H, 2 x CH₂); 3.55-3.61 (m, 4H, 2 x CH₂); 3.77 (s, 3H, OCH₃); 4.94 (s, 2H, CH₂); 6.83-6.99 (m, 4H, Ar-H); 7.32-7.41 (m, 4H, Ar-H); 7.59 (s, 1H, Het-H).

### 2-(4-Methoxybenzyl)-4-[4-(piperazin-1-yl)phenyl-2H-1,2,4-triazol-3-one (XXIII; X=H. R=4-methoxybenzyl):

The title compound was obtained by deprotection of *t*-BOC group of 2-(4-methoxybenzyl)-4-[4-(4-*tert*-BOC-piperazin-1-yl)phenyl-2H-1,2,4-triazol-3-one with 3M hydrochloric acid.
Colorless solid, 94% yield.
¹H NMR (CDCl₃) δ: 1.62 (s, 1H, N-H); 3.01-3.19 (m, 8H, 4 x CH₂); 3.79 (s, 3H, OCH₃); 4.95 (s, 2H, CH₂); 6.86-6.99 (m, 4H, Ar-H); 7.35-7.39 (m, 4H, Ar-H); 7.57 (s, 1 H, Het-H).

### (2R,3R)-2-(2,4-Difluorophenyl)-3-[4-{4-[2-(4-methoxybenzyl)-2H-1,2,4 - triazol-3-one-4-yl]phenyl}piperazin-1-yl]-1-(1H-1,2,4-triazol-1-yl)butan-2-ol:

The title compound was prepared from (2R,3S)-2-(2,4-difluorophenyl)-3-methyl-2-(1H-1,2,4-triazol-1-yl)methyloxirane **IV** and piperazino compound **XXIII** (R= 4-methoxybenzyt; X=H) by following the similar procedure described for the example **36**.
Colorless solid, yield 69%.
m.p. 178-179 °C
¹H NMR (CDCl₃) δ: 0.96 (d, 3H, J = 6.8 Hz, CH₃); 2.56-2.62 (m, 2H CH₂); 2.99-3.21 (m, 7H); 3.79 (s, 3H, OCH₃); 4.89-4.99 (m, 2H, CH₂); 4.95 (s, 2H, CH₂); 5.14 (s, 1H); 6.68-6.97 (m, 6H, Ar-H); 7.35-7.53 (m, 5H, Ar-H), 7.58 (s, 1H, Het-H); 7.78 (s, 1H, Het-H); 7.95 (s, 1H, Het-H).
FAB-MS: 617.0 (MH⁺), calcd. C₃₂H₃₄O₃F₂N₈ 616.67.

### Example 49

### 2-(2,4-Bis-trifluoromethyl)benzyl-4-[4-(4-tert-BOC-piperazin-1-yl)phenyl]-2H-1,2,4-triazol-3-one (XXII; X=H, R=2,4-bis-trifluoromethylbenzyl):

The title compound was prepared by alkylation of triazolone **XXI** (X=H) with 2,4-bis(trifluoromethyl)benzyl bromide in the presence of potassium carbonate. After usual workup the product was obtained in 76% yield as a colorless solid.
¹H NMR (CDCl₃) δ: 1.49 (s, 9H, 3 x CH₃); 3.15-3.20 (m, 4H, 2 x CH₂); 3.57-3.63 (m, 4H, 2 x CH₂); 5.31 (s, 2H, CH₂); 6.98-7.02 (m, 2H, Ar-H); 7.40-7.53 (m, 3H, Ar-H); 7.72 (s, 1H, Het-H); 7.77-7.95 (m, 2H, Ar-H).

### 2-(2,4-Bis-trifluoromethyl)benzyl-4-[4-(piperazin-1-yl)phenyl]-1H-1,2,4-triazol-3-one (XXIII: X=H, R=2,4-bis-trifluoromethylbenzyl):

The title compound was obtained by deprotection of *t*-BOC group of 4[-4-(4-tert-BOC-piperazin-1-yl)phenyl]-2-(2,4*-bis*-trifluoromethyl)benzyl-2H-1,2,4-triazol-3-one with 3M hydrochloric acid.
Colorless solid, 91% yield.
¹H NMR (CDCl₃) δ: 1.88 (s, 1H, NH); 3.02-3.21 (m, 8H, 4 x CH₂); 5.31 (s, 2H, CH₂); 6.96-7.01 (m, 2H, Ar-H); 7.39-7.95 (m, 5H, Ar-H); 7.70 (s, 1H, Het-H).

### (2R,3R)-3-[4-{4-[2-(2,4-Bis-trifluoromethylbenzyl)-2H-1,2,4-triazol-3-one-4-yl]phenyl}piperazin-1-yl]-2-(2.4-difluorophenyl)-1-(1H-1,2,4-triazol-1-yl)-butan-2-ol:

The title compound was prepared from (2R,3S)-2-(2,4-difluorophenyl)-3-methyl-2-(1H-1,2,4-triazol-1-yl)methyloxirane **IV** and piperazino compound **XXIII** [R= 2,4-bis(trifluromethyl)benzyl; X=H] by following the similar procedure described for the example **36.**
Colorless solid, yield: 70%.
m.p.: 146-148 °C.
¹H NMR (CDCl₃) δ: 0.99 (d, 3H, J = 6.8 Hz, CH₃); 2.58-2.66 (m, 2H, CH₂); 3.01-3.24 (m, 7H); 4.82-5.00 (m, 2H, CH₂); 5.14 (s, 1H); 5.31 (s, 2H, CH₂), 6.69-6.83 (m, 2H, Ar-H); 6.96-7.00 (m, 2H, Ar-H) 7.40-7.53 (m, 4H, Ar-H); 7.72 (s, 1H, Het-H) 7.77-7.81 (m, 2H, Ar-H & Het-H); 7.95 (s, 2H, Ar-H & Het-H).
FAB-MS: 723 (MH⁺), calcd. 722.64.

### Example 50

### 4-[4-(4-tert-BOC-Piperazin-1-yl)phenyl]-2-[4-(2,2,3,3-tetrafluoropropoxy)benzyl]-2H-1,2,4-triazol-3-one [XXII; X=H, R=4-(2,2,3,3-tetrafluoropropoxy)benzyl]:

The title compound was prepared by alkylation of triazolone **XXI** (X=H) with 4-(2,2,3,3-tetrafluoropropoxy)benzyl bromide in the presence of potassium carbonate. After usual workup the product was obtained in 81% yield as a colorless solid.
¹H NMR (CDCl₃) δ: 1.49 (s, 9H, 3XCH₃), 3.12-3.17 (m, 4H, 2XCH2), 3.55-3.60 (m, 4H, 2XCH₂), 4.26-4.37 (m, 2H, OCH₂), 4.94 (s, 2H, CH₂), 5.76-6.36 (m, 1H, CF₂H), 6.86-6.98 (m, 4H, Ar-H), 7.37 (m, 4H, Ar-H), 7.60 (s, 1H, Het-H).

### 4-[4-(Piperazin-1-yl)phenyl]-2-[4-(2,2,3,3-tetrafluoropropoxy)benzyl]-2H-1,2,4-triazol-3-one [XXIII; X=H, R=4-(2,2,3,3-tetrafluoropropoxy)benzyl]:

The title compound was obtained by deprotection of *t*-BOC group of the above compound with 3M hydrochloric acid. After usual workup the product was obtained in 98% yield as colorless solid.
¹H NMR (CDCl₃) δ: 1.87 (s, 1H, NH), 3.01-3.19 (m, 8H, 4XCH₂), 4.27-4.39 (m, 2H, OCH₂), 4.96 (s, 2H, CH₂), 5.77-6.35 (m, H, CF₂H), 6.89-6.99 (m, 4H, Ar-H), 7.33-7.42 (m, 4H, Ar-H), 7.58 (s, 1H, Het-H).

### (2R,3R)-2-(2,4-Difluorophenyl)-3-[4-{4-[2-{(4-{2,2,3,3-tetrafluoropropoxy)benzyl)}-2H-1,2,4-triazol-3-one-4-yl]phenyl}piperazin-1-yl]-1-(1H-1,2,4-triazol-1-yl)butan-2-ol:

The title compound was prepared from (2R,3S)-2-(2,4-difluorophenyl)-3-methyl-2-(1H-1,2,4-triazol-1-yl)methyloxirane **IV** and piperazino compound **XXIII** [R=4-(2,2,3,3-tetrafluoropropoxy)benzyl; X=H] by following the similar procedure described for the example **36.**
Yield 86%, coloriess prisms.
m.p.: 83-85 °C.
¹H NMR (CDCl₃) δ: 0.97 (d, 3H, CH₃), 2.57-2.62 (m, 2H, CH₂), 2.99-3.22 (m, 7H), 4.27-4.38 (m, 2H, CH₂), 4.81-4.99 (m, 2H, CH₂), 4.96 (s, 2H, CH₂), 5.15 (s, 1H, OH), 5.77-6.35 (m, 1H, CF₂H), 6.68-6.98 (m, 6H, Ar-H), 7.35-7.53 (m, 5H, Ar-H), 7.59 (s, 1H, Het-H), 7.78 (s, 1H, Het-H), 7.95 (s, 1H, Het-H).
FAB-MS: 717.3 (MH⁺), calcd C₃₄H₃₄F₆O₃N₈ 716.69.

### Example 51

### 4-[3-Fluoro-4-(4-tert-BOC-piperazin-1-yl)phenyl]-2-[4-(trifluoromethyl)benzyl]-2H-1,2,4-triazol-3-one (XXII; X=F, R=4-trifluoromethylbenzyl):

The title compound was prepared by alkylation of triazolone **XXI** (X=F) with (trifluoromethyl)benzyl bromide in the presence of potassium carbonate. After usual workup and purification on a column of silica gel, the title compound was obtained in 95% yield as a colorless solid.
¹H NMR (CDCl₃) δ: 1.48 (s, 9H, 3XCH₃), 3.04 (m, 4H, 2XCH₂), 3.60 (m, 4H, 2XCH₂), 5.06 (s, 2H, CH₂), 6.99 (m, 1H, Ar-H), 7.2-7.4 (m, 2H, Ar-H), 7.49-7.62 (m, 4H, Ar-H), 7.64 (s, 1H, Het-H).

### 4-[3-Fluoro-4-(piperazin-1-yl)phenyl]-2-[4-(trifluoromethyl)benzyl]-2H-1,2,4-triazol-3-one (XXIII: X=F, R=4-trifluoromethylbenzyl):

The title compound was obtained in 96% yield by deprotection of BOC group of the above compound with 3M hydrochloric acid.
¹H NMR (CDCl₃) δ: 3.0 (s, 8H, 4XCH₂), 5.05 (s, 2H, CH₂), 7.0 (m, 1H, Ar-H), 7.2-7.38 (m, 2H, Ar-H), 7.48-7.62 (m, 4H, Ar-H), 7.65 (s, 1H, Het-H).

### (2R,3R)-2-(2,4-Difluorophenyl)-3-[4-{3-fluoro-4-[2-(4-trifluoromethyl)benzyl-2H-1,2,4-triazol-3-one-4-yl]phenyl}piperazin-1-yl]-1-(1H-1,2,4-triazol-1-yl)butan-2-ol:

The title compound was prepared from (2R,3S)-2-(2,4-difluorophenyl)-3-methyl-2-(1H-1,2,4-triazol-1-yl)methyloxirane **IV** and piperazino compound **XXIII** [R = 4-(trifluoromethyl)benzyl; X=F] by following the similar procedure described for the example **36**.
Colorless solid, Yield 69%.
m.p.: 189-191 °C.
¹H NMR (CDCl₃) δ: 0.99 (d, 3H, CH₃), 2.65 (m, 2H, CH₂), 2.95-3.2 (m, 7H, 3XCH₂ and CH), 4.90 (AB q, 2H, CH₂), 5.07 (s, 2H, CH₂), 5.15 (s, 1H, OH), 6.7-6.85 (m, 2H, Ar-H), 7.04 (m, 1H, Ar-H), 7.2-7.62 (m, 7H, Ar-H), 7.64 (s, 1H, Het-H), 7.79 (s, 1H, Het-H), 7.95 (s, 1H, Het-H).
FAB-MS: 673.8 (MH⁺), calcd. C₃₂H₃₀F₆N₈O₂ 672.634

## Claims

1. A compound of formula I, or an optical isomer or pharmaceutically acceptable salt thereof, 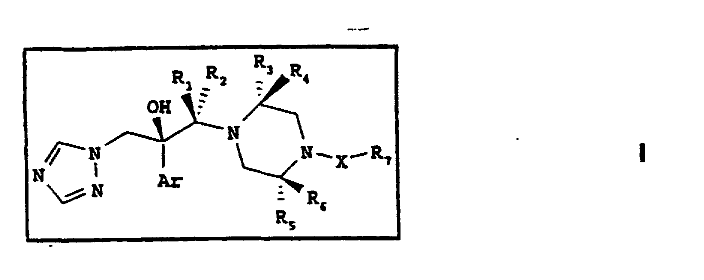 wherein:
Ar is a phenyl group which is unsubstituted or substituted by 1-3 substituents each independently selected from the group consisting of halogen, CF₃ and OCF₃;
R₁ and R₂ are each independently hydrogen or C₁-C₄ alkyl group which is unsubstituted or substituted by 1-3 substituents each independently selected from the group consisting of halogen, hydroxy, C₁-C₄ alkoxy and amino, with the proviso that where R₁ is hydrogen, R₂ is other than hydrogen, and vice versa;
R₃ and R₄ are each independently hydrogen or C₁-C₄ alkyl group which is unsubstituted or substituted by 1-3 substituents each independently selected from the group consisting of halogen, hydroxy, C₁-C₄ alkoxy and amino, or R₃ and R₄ together form =S;
R₅ and R₆ are each independenly hydrogen or C₁-C₄ alkyl group which is unsubstituted or Substituted by 1-3 substituents each independently selected from the group consisting of halogen, hydroxy, C₁-C₄ alkoxy and amino, or R₅ and R₆ together form =S;
X is selected from the group consisting of a direct bond, CO, CS, SO₂ and -N=N-;
R₇ is selected from the group consisting of
(i) phenyl which is unsubstituted or substituted with 1-3 substituents each independently selected from the group consisting of (1) C₁-C₄ alkyl which is unsubstituted or substituted with 1-3 substituents each independently selected from the group consisting of halogen, hydroxy, C₁-C₄ alkoxy and amino, (2) C₁-C₄ alkoxy, (3) halogen, (4) formyl, (5) carboxyl, (6) C₁-C₄ acyloxy, (7) C₁-C₄ alkoxycarbonylamino, (8) phenyl- or naphthyl-oxycarbonylamino, (9) semicarbazido, (10) formamido, (11) thioformamido, (12) hydroxy, (13) nitro, (14) amino, (15) furyl, (16) triazolyl, (17) thienyl, (18) oxazolyl, (19) imidazolyl and (20) triazolone-yl,
(ii) a 5- or 6-membered monocyclic or 8- to 10-membered bicyclic heterocycle having 1-4 heteroatoms each independently selected from the group consisting of N, O and S, which heterocycle is unsubstituted or ring-substituted with 1-3 substituents each independently selected from the group consisting of (1) C₁-C₄ alkyl which is unsubstituted or substituted with 1-3 substituents each independently selected from the group consisting of halogen, hydroxy, C₁-C₄ alkoxy and amino, (2) benzyl which is unsubstituted or substituted with 1-3 substituents selected from the group consisting of C₁-C₄ alkyl, CF₃, halogen and OCF₃, (3) halogen, (4) hydroxy, (5) nitro, (6) amino, (7) C₁-C₄ acylamino, (8) formyl, (9) formamido, (10) thioformamido, (11) C₁-C₄ alkoxycarbonylamino, (12) phenyl- or naphthyl-oxycarbonylamino and (13) semicarbazido,
(iii) NHR₈ wherein R₈ is selected from the group consisting of (1) C₁-C₄ alkyl which is unsubstituted or substituted with 1-3 substituents each independently selected from the group consisting of halogen, hydroxy, C₁-C₄ alkoxy and amino, (2) phenyl which is unsubstituted or substituted with 1-3 substituents each independently selected from the group consisting of (a) C₁-C₄ alkyl which is unsubstituted or substituted with 1-3 substituents each independently selected from the group consisting of halogen, hydroxy, C₁-C₄ alkoxy and amino, (b) C₁-C₄ alkoxy, (c) halogen, (d) formyl, (e) carboxyl, (f) C₁-C₄ acyloxy, (g) C₁-C₄ alkoxycarbonylamino, (h) phenyl- or naphthyl-oxycarbonylamino, (i) semicarbazido, (j) formamido, (k) thioformamido, (I) hydroxy, (m) nitro, (n) amino, (o) furyl, (p) triazotyl, (q) thienyl, (r) oxazolyl, (s) imidazolyl and (t) triazolone-yl, and (3) a 5- or 6-membered monocyclic or 8-to 10-membered bicyclic heterocycle having 1-3 heteroatoms each independently selected from the group consisting of N, O and S, which is unsubstituted or substituted with 1-3 substituents each independently selected from the group consisting of hydroxy, halogen, amino and carboxyl,
(iv) OR₉ wherein R₉ is selected from the group consisting of (1) C₁-C₄ alkyl which is unsubstituted or substituted with 1-3 substituents each independently selected from the group consisting of halogen, hydroxy, C₁-C₄ alkoxy and amino, (2) phenyl which is unsubstituted or substituted with 1-3 substituents each independently selected from the group consisting of (a) C₁-C₄ alkyl which is unsubstituted or substituted with 1-3 substituents each independently selected from the group consisting of halogen, hydroxy, C₁-C₄ alkoxy and amino, (b) C₁-C₄ alkoxy, (c) halogen, (d) formyl, (e) carboxyl, (f) C₁-C₄ acyloxy, (g) C₁-C₄ alkoxycarbonylamino, (h) phenyl- or naphthyl-oxycarbonylamino, (i) semicarbazido, (j) formamido, (k) thioformamido, (I) hydroxy, (m) nitro, (n) amino, (o) furyl, (p) triazolyl, (q) thienyl, (r) oxazolyl, (s) imidazolyl and (t) triazolone-yl and (3) a 5- or 6-membered monocyclic or 8-to 10-membered bicyclic heterocycle having 1-3 heteroatoms each independently selected from the group consisting of N, O and S, which is unsubstituted or substituted with 1-3 substituents each independently selected from the group consisting of (a) C₁-C₄ alkyl which is unsubstituted or substituted with 1-3 substituents each independently selected from the group consisting of halogen, hydroxy, C₁-C₄ alkoxy and amino, (b) phenyl which is unsubstituted or substituted with 1-3 substituents each independently selected from the group consisting of (A) C₁-C₄ alkyl which is unsubstituted or substituted with 1-3 substituents each independently selected from the group consisting of halogen, hydroxy, C₁-C₄ alkoxy and amino, (B) C₁-C₄ alkoxy, (C) halogen, (D) formyl, (E) carboxyl, (F) C₁-C₄ acyloxy, (G) C₁-C₄ alkoxycarbonylamino, (H) phenyl- or naphthyl-oxycarbonylamino, (I) semicarbazido, (J) formamido, (K) thioformamido, (L) hydroxy, (M) nitro, (N) amino, (O) furyl, (P) triazolyl, (Q) thienyl, (R) oxazolyl, (S) imidazolyl and (T) triazolone-yl, (c) naphthyl which is unsubstituted or substituted with 1-3 substituents each independently selected from the group consisting of (A) C₁-C₄ alkyl which is unsubstituted or substituted with 1-3 substituents each independently selected from the group consisting of halogen, hydroxy, C₁-C₄ alkoxy and amino, (B) C₁-C₄ alkoxy, (C) halogen, (D) formyl, (E) carboxyl, (F) C₁-C₄ acyloxy, (G) C₁-C₄ alkoxycarbonylamino, (H) phenyl- or naphthyl-oxycarbonylamino, (I) semicarbazido, (J) formamido, (K) thioformamido, (L) hydroxy, (M) nitro, (N) amino, (O) furyl, (P) triazolyl, (Q) thienyl, (R) oxazolyl, (S) imidazolyl and (T) triazolone-yl, (d) a 5- or 6-membered monocyclic or 8-to 10-membered bicyclic heterocycle having 1-3 heteroatoms each independently selected from the group consisting of N, O and S, (e) (C₁-C₄ alkyl)phenyl, (f) (C₁-C₄ alkyl)naphthyl, (g) hydroxy, (h) halogen, (i) amino and (j) carboxyl, and
v ) a group of the formula 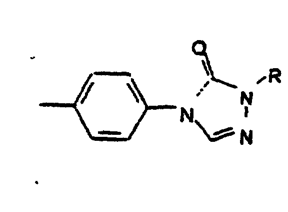 wherein R is selected from the group consisting of (1) hydrogen, (2) C₁-C₁₀ alkyl which Is unsubstituted or substituted by 1-5 substituents each independently selected from the group consisting of halogen, hydroxy, C₁-C₄ alkoxy and amino, (3) phenyl which is unsubstituted or substituted with 1-3 substituents each independently selected from the group consisting of (a) C₁-C₄ alkyl which is unsubstituted OF substituted with 1-3 substituents each independently selected from the group consisting of halogen, hydroxy, C₁-C₄ alkoxy and amino, (b) C₁-C₄ alkoxy, (c) halogen, (d) formyl, (e) carboxyl, (f) C₁-C₄ acyloxy, (g) C₁-C₄ alkoxycarbonylamino, (h) phenyl- or naphthyl-oxycarbonylamino, (i) semicarbazido, (j) formamido, (k) thioformamido, (I) hydroxy, (m) nitro, (n) amino, (o) furyl, (p) triazolyl, (q) thienyl, (r) oxazolyl, (s) imidazolyl, (t) triazolone-yl, (u) CF₃ and (v) OCF₃, (4) a 5- or 6-membered monocyclic or 8- to 10-membered bicyclic heterocycle having 1-3 heteroatoms each independently selected from the group consisting of N, O and S, which is unsubstituted or substituted with 1-3 substituents each independently selected from the group consisting of (a) C₁-C₄ alkyl which is unsubstituted or substituted with 1-3 substituents each independently selected from the group consisting of halogen, hydroxy, C₁-C₄ alkoxy and amino, (b) phenyl which is unsubstituted or substituted with 1-3 substituents each independently selected from the group consisting of (A) C₁-C₄ alkyl which is unsubstituted or substituted with 1-3 substituents each independently selected from the group consisting of halogen, hydroxy, C₁-C₄ alkoxy and amino, (B) C₁-C₄ alkoxy, (C) halogen, (D) formyl, (E) carboxyl, (F). C₁-C₄ acyloxy, (G) C₁-C₄ alkoxycarbonylamino, (H) phenyl- or naphthyl-oxycarbonylamino. (I) semicarbazido, (J) formamido, (K) thioformamido, (L) hydroxy, (M) nitro, (N) amino, (O) furyl, (P) triazolyl, (Q) thienyl, (R) oxazolyl, (S) imidazolyl and (T) triazolone-yl, (c) naphthyl which is unsubstituted or substituted with 1-3 substituents each independently selected from the group consisting of (A) C₁-C₄ alkyl which is unsubstituted or substituted with 1-3 substituents each independently selected from the group consisting of halogen, hydroxy, C₁-C₄ alkoxy and amino, (B) C₁-C₄ alkoxy, (C) halogen, (D) formyl, (E) carboxyl, (F) C₁-C₄ acyloxy, (G) C₁-C₄ alkoxycarbonylamino, (H) phenyl- or naphthyl-oxycarbonylamino, (I) semicarbazido, (J) formamido, (K) thioformamido, (L) hydroxy, (M) nitro, (N) amino, (O) furyl, (P) triazolyl, (Q) thienyl, (R) oxazolyl, (S) imidazolyl and (T) triazolone-yl, (d) a 5- or 6-membered monocyclic or 8- to 10-membered bicyclic heterocycle having 1-3 heteroatoms each independently selected from the group consisting of N, O and S, (e) (C₁-C₄ alkyl)phenyl, (f) (C₁-C₄ alkyl)naphthyl, (g) hydroxy, (h) halogen, (i) amino and (j) carboxyl, (5) phenyl(C₁-C₄ alkyl) which is unsubstituted or ring-substituted with 1-3 substituents each independently selected from the group consisting of (a) C₁-C₅ alkyl which is unsubstituted or substituted with 1-3 substituents each independently selected from the group consisting of halogen, hydroxy, C₁-C₄ alkoxy and amino, (b) halogen, (c) halo(C₁-C₄ alkyl), (d) C₁-C₄ alkoxy, (e) hydroxy, (f) amino, (g) carboxyl, (h) trifluormethoxyl, (i) trifluoromethyl, (j) tetrafluoroethyl, (k) tetrafluoroethoxyl, (I) tetrafluoropropyl and (m) tetrafluoropropoxyl, (6) naphthyl(C₁-C₄ alkyl) which may be substituted with 1-6 substituents selected from (a) C₁-C₅ alkyl which is unsubstituted or substituted with 1-3 substituents each independently selected from the group consisting of halogen, hydroxy, C₁-C₄ alkoxy and amino, (b) halogen, (c) (C₁-C₄ alkyl)halo, (d) C₁-C₄ alkoxy, (e) hydroxy, (f) amino, (g) carboxyl, (h) trifluormethoxyl, (i) trifluoromethyl, (j) tetrafluoroethyl, (k) tetrafluoroethoxyl, (I) tetrafluoropropyl and (m) tetrafluoropropoxyl, (7) methoxyl, (8) trifluormethoxyl, (9) trifluoromethyl, (10) trifluoroethyl, (11) tetrafluoroethyl, (12) tetrafluoroethoxyl, (13) tetrafluoropropyl and (14) tetrafluoropropoxyl.

2. The compound according to claim 1, wherein Ar is selected from the group consisting of 4-fluorophenyl, 2-4-difluorophenyl, 2,4,6-trifluorophenyl, 4-chlorophenyl, 2,4-dichlorophenyl, 2,4,6-trichlorophenyl, 4-trifluoromethylphenyl and 4-trifluoromethoxyphenyl.

3. The compound according to claim 1, wherein Ar is a phenyl group having 1 to 2 substituents each independently selected from the group consisting of fluorine, chlorine, trifluoromethyl and trifluromethoxy.

4. The compound according to claim 1, wherein Ar is selected from the group consisting of 2,4-difluorophenyl, 2,4-dichlorophenyl, 4-trifluromethylphenyl and 4-trifluoromethoxyphenyl.

5. The compound according to claim 1, wherein Ar is 2,4-diflourophenyl.

6. The compound according to claim 1, wherein R₁ is alkyl.

7. The compound according to claim 1, wherein R₁ is methyl.

8. The compound according to claim 1, wherein R₂ is hydrogen.

9. The compound according to claim 1, wherein R₁ and R₂ are the same, and the compound has a 2R isomeric configuration.

10. The compound according to claim 1, wherein R₁ and R₂ are different, and the compound has a 2R, 3R isomeric configuration.

11. The compound according to claim 1, wherein X is a direct bond and R₇ is a group of the formula

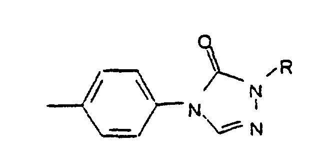

12. The compound according to claim 11, wherein Ar is 2,4-difluorophenyl, R₁ is methyl, and R₂ through R₆ are each hydrogen.

13. The compound according to claim 1, wherein R, is hydrogen and R₂ is methyl.

14. The compound according to claim 1, wherein R₃ through R₆ are each hydrogen.

15. The compound according to claim 1, wherein R₃ and R₅ are each methyl and R₄ and R₆ are each hydrogen.

16. The compound according to claim 1, wherein X is a direct bond.

17. The compound according to daim 1, wherein R₇ is group of the formula 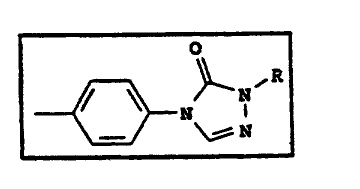 wherein R is selected from the group consisting of 2-propyl, 2-butyl, 3-pentyl, 2-hydroxypropyl, 4-trifluoromethylbenzyl, tetrafluoropropyl, trifluoroethyl, 2,4-difluorobenzyl, 4-methoxybenzyl, 4-trifluoromethoxybenzyl and 2,4-bis(trifluoromethyl)benzyl.

18. The compound according to claim 1, wherein the compound is selected from the group consisting of:
(2R,3R)-2-(2,4-Difluorophenyl)-3-(2,5-dimethylpiperazin-1-yl)-1-(1H-1,2,4-triazol-1-yl)butan-2-ol,
(2R,3R)-2-(2,4-Difluorophenyl)-3-(2,5-dimethyl-4-ethoxycarbonylpiperazin-1-yl)-1-(1H-1,2,4-triazol-1-yl)butan-2-ol,
(2R,3R)-3-(Anilinocarbonylpiperazin-1-yl)-2-(2,4-difluorophenyl)-1-(1H-1,2,4-triazol-1-yl)butan-2-ol,
(2R,3R)-2-(2,4-Difluorophenyl)-3-(4-ethylaminocarbonylpiperazin-1-yl)-1-(1H-1,2,4-triazol-1-yl)butan-2-ol,
(2R,3R)-3-(Anilinothiocarbonylpiperazin-1-yl)-2-(2,4-difluorophenyl)-1-(1H-1,2,4-triazol-1-yl)butan-2-ol,
(2R,3R)-2-(2,4-Difluorophenyl)-3-(4-ethylaminothiocarbonylpiperazin-1-yl)-1-(1H-1,2,4-triazol-1-yl)butan-2-ol,
(2R,3R)-3-[4-(2,4-Difluorobenzoyl)piperazin-1-yl]-2-(2,4-diflurophenyl)-1-(1 H-1,2,4-triazol-1-yl)butan-2-ol,
(2R,3R)-2-(2,4-Difluorophenyl)-1-(1H-1,2,4-triazol-1-yl)-3-[4-(4-trifluoromethylbenzoyl)piperazin-1-yl]butan-2-ol,
(2R,3R)-2-(2,4-Difluorophenyl)-3-[4-(4-nitrobenzoyl)piperazin-1-yl]-1-(1 H-1,2,4-triazol-1-yl)butan-2-ol,
(2R,3R)-3-[4-(4-Aminobenzoyl)piperazin-1-yl]-2-(2,4-diflurophenyl)-1-(1H-1,2,4-triazol-1-yl)butan-2-ol,
(2R,3R)-2-(2,4-Difluorophenyl)-3-[4-(*p*-toluenesulphonyl)piperazin-1-yl]-1-(1H-1,2,4-triazol-1-yl)butan-2-ol,
(2R,3R)-2-(2,4-Difluorophenyl)-3-[4-(methanesulphonyl)piperazin-1-yl]-1-(1 H-1,2,4-triazol-1-yl)butan-2-ol,
(2R,3R)-2-(2,4-Dtfluorophenyl)-3-[4-(4-nitrophenylsulphonyl)piperazin-1-yl]-1-(1H-1,2,4-triazol-1-yl)butan-2-ol,
(2R,3R)-3-[4-(4-Aminophenylsulphonyl)piperazin-1-yl]- 2-(2,4-difluorophenyl)-1-(1H-1,2,4-triazol-1-yl)butan-2-ol,
(2R,3R)-2-(2,4-Difluorophenyl)-3-[4-(3-azo-1H-1,2,4-triazol-3-yl))piperazin-1-yl]-1-(1H-1,2,4-triazol-1-yl)butan-2-ol,
(2R,3R)-2-(2,4-Difluorophenyl)-3-[4-(2-thiazolyl)piperazin-1-yl]-1-(1H-1,2,4-triazol-1-yl)butan-2-ol,
(2R,3R)-2-(2,4-Difluorophenyi)-3-[4-(1H-1,2,4-triazol-3-yl)piperazin-1-yl]-1-(1H-1,2,4-triazol-1-yl)butan-2-ol,
(2R,3R)-2-(2,4-Difluorophenyl)-3-[4-(1H-5-tetrazolyl)piperazin-1-yl]-1-(1 H-1,2,4-triazo(-1-yl)butan-2-ol,
(2R,3R)-2-(2,4-Difluorophenyl)-3-{4-[2-(4-*tert*-butylbenzyl)-2H-tetrazol-5-yl]piperazin-1-yl}-1-(1H-1,2,4-triazol-1-yl)butan-2-ol,
(2R,3R)-2-(2,4-Difluorophenyl)-1-(1H-1,2,4-triazol-1-yl)-3-{4-[2-(4-trifluoromethylbenzyl)-2H-tetrazol-5-yl]piperazin-1-yl}butan-2-ol,
(2R,3R)-2-(2,4-Difluorophenyl)-3-{4-{2-(4-*tert*-butylbenzyl)-2H-1,2,4-triazol-3-yl]piperazin-1-yl}-1-(1H-1,2,4-triazol-1-yl)butan-2-ol,
(2R,3R)-2-(2,4-Difluorophenyl)-3-{4-[1-(4-*tert*-butylbenzyl)-1H-1,2;4-triazol-3-yl]piperazin-1-yl}-1-(1 H-1,2,4-triazol-1-yl)butan-2-ol,
(2R,3R)-2-(2,4-Difluorophenyl)-1-(1H-1,2,4-triazot-1-yl)-3-{4-(1-(4-trifluoromethylbenzyl)-1H-1,2,4-triazol-3-yl]piperazin-1-yl}butan-2-ol,
(2R,3R)-2-(2,4-Difluorophenyl)-3-(4-phenylpiperazin-1-yl)-1-(1 H-1,2,4-triazol-1-yl)butan-2-ol,
(2R,3R)-2-(2,4-Difluorophenyl)-3-[4-(4-nitrophenyl)piperazin-1-yl]-1-(1 H-1,2,4-triazol-1-yl)butan-2-ol,
(2R,3R)-3-[4-(4-Aminophenyl)piperazin-1-yl]-2-(2,4-difluorophenyl)-1-(1 H-1,2,4-triazol-1-yl)butan-2-ol,
(2R,3R)-2-(2,4-Difluorophenyl)-3-[4-(4-ethoxycarbonylaminophenyl)piperazin-1-yl]-1-(1H-1,2,4-triazol-1-yl)butan-2-ol;
(2R,3R)-2-(2,4-Difluorophenyl)-3-[4-(4-phenoxycarbonylaminophenyl)piperazin-1-yl]-1-(1 H-1,2,4-triazol-1-yl)butan-2-ol,
(2R,3R)-2-(2,4-Difluorophenyl)-3-{4-[4-(semicarbazid-4-yl)phenyl]piperazin-1-yl}-1-(1H-1,2,4-triazol-1-yl)butan-2-ol, and
(2R,3R)-2-(2,4-Difluorophenyl)-1-(1H-1,2,4-triazol-1-yl)-3-{4-[4-(2H-1,2,4-triazol-3-one-4-yl)phenyl]piperazin-1-yl}butan-2-ol.

19. The compound according to claim 1, wherein the compound is selected from the group consisting of:
(2R,3R)-2-(2,4-Difluorophenyl)-3-[4-{4-[2-(3-pentyl)-2H-1,2,4-triazol-3-one-4-yl]phenyl}piperazin-1-yl]-1-(1H-1,2,4-triazol-1-yl)butan-2-ol,
(2S,3R)-2-(2,4-Difluorophenyl)-3-[4-{4-[2-(3-pentyl)-2H-1,2,4-triazol-3-one-4-yl]phenyl}piperazin-1-yl]-1-(1H-1,2,4-triazol-1-yl)butan-2-ol,
(2S,3S)-2-(2,4-Difluorophenyl)-3-[4-{4-[2-(3-pentyl)-2H-1,2,4-triazol-3-one-4-yl]phenyl}piperazin-1-yl]-1-(1H-1,2,4-triazol-1-yl)butan-2-ol,
(2R,3S)-2-(2,4-Difluorophenyl)-3-[4-{4-[2-(3-pentyl)-2H-1,2,4-triazol-3-one-4-yl]phenyl}piperazin-1-yl]-1-(1H-1,2,4-triazol-1-yl)butan-2-ol,
(2R,3R)-3-[4-{4-[2-(2-Butyl)-2H-1,2,4-triazol-3-one-4 - yl]phenyl}piperazin-1-yl]-2-(2,4-Difluorophenyl)-1-(1H-1,2,4-triazol-1-yl)butan-2-ol,
(2R,3R)-2-(2,4-Difluorophenyl)-3-[4-{4-[2-(2-propyl)-2H-1,2,4-triazol-3-one-4-yl]phenyl}piperazin-1-yl]-1-(1H-1,2,4-triazol-1-yl)butan-2-ol,
(2R,3R)-2-(2,4-Difluorophenyl)-3-[4-{4-[2-(2-hydroxypropyl)-2H-1,2,4-triazol-3-one-4-yl]phenyl}piperazin-1-yl]-1-(1 H-1,2,4-triazol-1-yl)butan-2-ol,
(2R,3R)-2-(2,4-Difluorophenyl)-1-(1H-1,2,4-triazol-1-yl)-3-[4-{4-[2-(4-trifluoromethyl)benzyl-2H-1,2,4-triazol-3-one-4-yl]phenyl}piperazin-1-yl]butan-2-ol,
(2R,3R)-2-(2,4-Difluorophenyl)-3-[4-{4-[2-(2,2,3,3-tetrafluoro)propyl-2H-1,2,4-triazol-3-one-4-yl]phenyl}piperazin-1-yl]-1-(1H-1,2,4-triazol-1-yl)- butan-2-ol,
(2R,3R}-2-(2,4-Difluorophenyl)-1-(1H-1,2,4-triazol-1-yl)-3-[4-{4-[2-(2,2,2-trifluoro)ethyl-2H-1,2,4-triazol-3-one-4-yl]phenyl}piperazin-1-yl]butan-2-ol,
(2R,3R)-3-[4-{4-[2-(2,4-Difluorobenzyl)-2H-1,2,4-triazol-3-one-4-yl]phenyl}piperazin-1-yl]-2-(2,4-Difluorophenyl)-1-(1H-1,2,4-triazol-1-yl)butan-2-ol,
(2R,3R)-2-(2,4-Difiuorophenyl)-3-[4-{4-[2-(4-trifluoromethoxy)benzyl-2H-1,2,4-triazol-3-one-4-yl]phenyl}piperazin-1-yl]-1-(1H-1,2,4-triazol-1-yl)-butan-2-ol,
(2R,3R)-2-(2,4-Difluorophenyl)-3-[4-{4-[2-(4-methoxylbenzyl)-2H-1,2,4-triazol-3-one-4-yl]phenyl}piperazin-1-yl]-1-(1 H-1,2,4-triazol-1-yl)-butan-2-ol,
(2R, 3R)-3-[4-{4-[2-(2,4-Bis-trifluoromethylbenzyl)-2H-1,2,4-triazol-3-one-4-yl]phenyl}piperazin-1-yl]-2-(2,4-difluorophenyl)-1-(1H-1,2,4-triazol-1-yl)-butan-2-ol,
(2R,3R)-2-(2,4-Difluorophenyl)-3-[4-{4-[2-((4-(2,2,3,3-tetrafluoropropoxy)benzyl))-2H-1,2,4-triazol-3-one-4-yl]phenyl}piperazin-1-yl]-1-(1H-1,2,4-triazol-1-yl)butan-2-ol, and
(2R,3R)-2-(2,4-Difluorophenyl)-3-[4-{3-fluoro-4-[2-(4-trifluoromethyl)benzyl-2H-1,2,4-triazol-3-one-4-yl]phenyl}piperazin-1-yl]-1-(1H-1,2,4-triazol-1-yl)butan-2-ol.

20. The compound according to claim 19, wherein the compound is selected from the group consisting of:
(2R,3R)-2-(2,4-Difluorophenyl)-1-(1H-1,2,4-triazol-1-yl)-3-[4-{4-[2-(4-trifluoromethyl)benzyl-2H-1,2,4-triazol-3-one-4-yl]phenyl}piperazin-1-yl]butan-2-ol,
(2R,3R)-2-(2,4-Difluorophenyl)-3-[4-{4-[2-(4-trifluoromethoxy)benzyl-2H-1,2,4-triazol-3-one-4-yl]phenyl}piperazin-1-yl]-1-(1H-1,2,4-triazol-1-yl)-butan-2-ol,
(2R,3R)-2-(2,4-Difluorophenyl)-3-[4-{4-[2-((4-(2,2,3,3-tetrafluoropropoxy)benzyl))-2H-1,2,4-triazol-3-one-4-yl]phenyl}piperazin-1-yl]-1-(1H-1,2,4-triazol-1-yl)butan-2-ol, and
(2R,3R)-2-(2,4-Difluorophenyl)-3-[4-{3-fluoro-4-[2-(4-trifluoromethyl)benzyl-2H-1,2,4-triazol-3-one-4-yl]phenyl}piperazin-1-yl]-1-(1H-1,2,4-triazol-1-yl)butan-2-ol.

21. A compound of formula **III**, 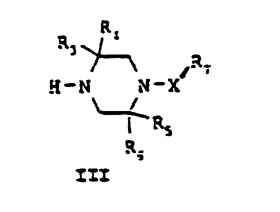 wherein:
R₃ and R₄ are each independently hydrogen or C₁-C₄ alkyl group which is unsubstituted or substituted by 1-3 substituents each independently selected from the group consisting of halogen, hydroxy, C₁-C₄ alkoxy and amino, or R₃ and R₄ together form =Z, wherein Z is oxygen or sulphur,
R₅ and R₆ are each independenly hydrogen or C₁-C₄ alkyl group which is unsubstituted or substituted by 1-3 substituents each independently selected from the group consisting of halogen, hydroxy, C₁-C₄ alkoxy and amino, or R₅ and R₆ together form =Z, wherein Z is oxygen or sulphur;
X is a direct bond;
R₇ is selected from the group consisting of a group of the formula 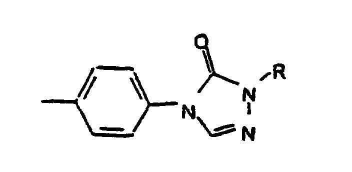 wherein R is selected from the group consisting of (1) hydrogen, (2) C₁-C₁₀ alkyl which is unsubstituted or substituted by 1-5 substituents each independently selected from the group consisting of halogen, hydroxy, C₁-C₄ alkoxy and amino, (3) phenyl which is unsubstituted or substituted with 1-3 substituents each independently selected from the group consisting of (a) C₁-C₄ alkyl which is unsubstituted or substituted with 1-3 substituents each independently selected from the group consisting of halogen, hydroxy, C₁-C₄ alkoxy and amino, (b) C₁-C₄ alkoxy, (c) halogen, (d) formyl, (e) carboxyl, (f) C₁-C₄ acyloxy, (g) C₁-C₄ alkoxycarbonylamino, (h) phenyl- or naphthyl-oxycarbonylamino, (i) semicarbazido, (j) formamido, (k) thioformamido, (I) hydroxy, (m) nitro, (n) amino, (o) furyl, (p) triazolyl, (q) thienyl, (r) oxazolyl, (s) imidazolyl, (t) triazolone-yl, (u) CF₃ and (v) OCF₃, (4) a 5- or 6-membered monocyclic or 8- to 10-membered bicyclic heterocycle having 1-3 heteroatoms each independently selected from the group consisting of N, O and S, which is unsubstituted or substituted with 1-3 substituents each independently selected from the group consisting of (a) C₁-C₄ alkyl which is unsubstituted or substituted with 1-3 substituents each independently selected from the group consisting of halogen, hydroxy, C₁-C₄ alkoxy and amino, (b) phenyl which is unsubstituted or substituted with 1-3 substituents each independently selected from the group consisting of (A) C₁-C₄ alkyl which is unsubstituted or substituted with 1-3 substituents each independently selected from the group consisting of halogen, hydroxy, C₁-C₄ alkoxy and amino, (B) C₁-C₄ alkoxy, (C) halogen, (D) formyl, (E) carboxyl, (F) C₁-C₄ acyloxy, (G) C₁-C₄ alkoxycarbonylamino, (H) phenyl- or naphthyl-oxycarbonylamino, (I) semicarbazido, (J) formamido, (K) thioformamido, (L) hydroxy, (M) nitro, (N) amino, (O) furyl, (P) triazolyl, (Q) thienyl, (R) oxazolyl, (S) imidazolyl and (T) triazolone-yl, (c) naphthyl which is unsubstituted or substituted with 1-3 substituents each independently selected from the group consisting of (A) C₁-C₄ alkyl which is unsubstituted or substituted with 1-3 substituents each independently selected from the group consisting of halogen, hydroxy, C₁-C₄ alkoxy and amino, (B) C₁-C₄ alkoxy, (C) halogen, (D) formyl, (E) carboxyl, (F) C₁-C₄ acyloxy, (G) C₁-C₄ alkoxycarbonylamino, (H) phenyl- or naphthyl-oxycarbonylamino, (I) semicarbazido, (J) formamido, (K) thioformamido, (L) hydroxy, (M) nitro, (N) amino, (O) furyl, (P) triazolyl, (Q) thienyl, (R) oxazolyl, (S) imidazolyl and (T) triazolone-yl, (d) a 5- or 6-membered monocyclic or 8- to 10-membered bicyclic heterocycle having 1-3 heteroatoms each independently selected from the group consisting of N, O and S, (e) (C₁-C₄ alkyl)phenyl, (f) (C₁-C₄ alkyl)naphthyl, (g) hydroxy, (h) halogen, (i) amino and (j) carboxyl, (5) phenyl(C₁-C₄ alkyl) which is unsubstituted or ring-substituted with 1-3 substituents each independently selected from the group consisting of (a) C₁-C₅ alkyl which is unsubstituted or substituted with 1-3 substituents each independently selected from the group consisting of halogen, hydroxy, C₁-C₄ alkoxy and amino, (b) halogen, (c) halo(C₁-C₄ alkyl), (d) C₁-C₄ alkoxy, (e) hydroxy, (f) amino, (g) carboxyl, (h) trifluormethoxyl, (i) trifluoromethyl, (j) tetrafluoroethyl, (k) tetrafluoroethoxyl, (l) tetrafluoropropyl and (m) tetrafluoropropoxyl, (6) naphthyl(C₁-C₄ alkyl) which may be substituted with 1-6 substituents selected from (a) C₁-C₅ alkyl which is unsubstituted or substituted with 1-3 substituents each independently selected from the group consisting of halogen, hydroxy, C₁-C₄ alkoxy and amino, (b) halogen, (c) (C₁-C₄ alkyl)halo, (d) C₁-C₄ alkoxy, (e) hydroxy, (f) amino, (g) carboxyl, (h) trifluormethoxyl, (i) trifluoromethyl, (j) tetrafluoroethyl, (k) tetrafluoroethoxyl, (I) tetrafluoropropyl and (m) tetrafluoropropoxyl, (7) methoxyl, (8) trifluormethoxyl, (9) trifluoromethyl, (10) trifluoroethyl, (11) tetrafluoroethyl, (12) tetrafluoroethoxyl, (13) tetrafluoropropyl and (14) tetrafluoropropoxyl.

22. The compound according to claim 21, wherein the compound is selected from the group consisting of:
2-(3-pentyl)-4-[4-(piperazin-1-yl)phenyl]-2H-1,2,4-triazol-3-one,
2-(2-butyl)-4-[4-(piperazin-1-yl)phenyl]-2H-1,2,4-triazol-3-one,
4-[4-(piperazin-1-yl)phenyl]-2-(2-propyl)-2H-1,2,4-triazol-3-one,
2-(2-hydroxypropyl)-4-[4-(piperazin-1-yl)phenyl]-2H-1,2,4-triazol-3-one,
4-[4-(piperazin-1-yl)phenyl]-2-[2-(4-trifluoromethyl)benzyl]-2H-1,2,4-triazol-3-one,
4-[4-(piperazin-1-yl)phenyl]-2-[2-(2,2,3,3-tetrafluoro)propyl]-2H-1,2,4-triazol-3-one,
4-[4-(piperazin-1-yl)phenyl]-2-[2-(3,3,3-trifluoroethyl)]-2H-1,2,4-triazol-3-one,
2-(2,4-difluorobenzyl)-4-[4-(piperazin-1-yl)phenyl]-2H-1,2,4-triazol-3-one,
4-[4-(piperazin-1-yl)phenyl]-2-(4-trifluoromethoxybenzyl)-2H-1,2,4-triazol-3-one,
2-(4-methoxybenzyl)-4-[4-(piperazin-1-yl)phenyl]-2H-1,2,4-triazol-3-one,
2-(2,4-bis-trifluoromethylbenzyl)-4-[4-(piperazin-1-yl)phenyl]-2H-1,2,4-triazol-3-one,
4-[4-(piperazin-1-yl)phenyl]-2-[4-(2,2,3,3-tetrafluoropropoxy)benzyl]-2H-1,2,4-triazol-3-one, and
4-[3-fluoro-4-(piperazln-1-yl)phenyl]-2-(4-trifluoromethylbenzyl)-2H-1,2,4-triazol-3-one.

23. A composition for treating a fungal infection comprising a pharmaceutically effective amount of a compound according to any of claims 1-22 in combination with a pharmaceutically acceptable carrier or diluent.

24. The pharmaceutical composition according to claim 23, wherein the pharmaceutical composition is an oral formulation and the compound is present in an amount of 1 to 25% (w/w).

25. The pharmaceutical composition according to claim 23, wherein the pharmaceutical composition is an injectable formulation and the compound is present in an amount of 0.1 to 5% (w/w).

26. The pharmaceutical composition according to claim 23, wherein the pharmaceutical composition is a topical or rectal formulation and the compound is present in an amount of 1 to 10% (w/w).

27. Use of a compound in accordance with any of claims 1-26 for the manufacture of a medicament for treating or preventing a fungal infection in a patient in need of such treatment or prevention.

28. Use of a compound according to claim 27, wherein the compound is formulated to be administered in a daily dose of 0.01 to 20 mg/kg patient.

29. Use of a compound according to claim 28, wherein the compound is formulated to be administered in a daily dose which is divided into a plurality of individual doses.

30. Use of a compound according to any of claims 27-29, wherein the fungal infection is a topical infection.

31. Use of a compound according to any of claims 27-30, wherein the fungal infection is a systemic infection.

32. Use of a compound according to any of claims 27-31, wherein the fungal infection is a mucosal infection.

33. Use of a compound according to any of claims 27-32, wherein the fungal infection is a lung-invasive infection.

34. A composition according to any of claims 1-22 for use as a pharmaceutical.

## Patentansprüche

1. Verbindung mit der Formel I, oder ein optisches Isomer oder ein pharmazeutisch akzeptables Salz davon, 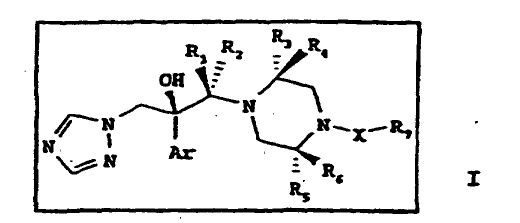 wobei:
Ar eine Phenylgruppe ist, die unsubstituiert oder mit 1-3 Substituenten, die jeder unabhängig aus der Gruppe bestehend aus Halogen, CF₃ und OCF₃ ausgewählt werden, substituiert ist;
R₁ und R₂ jeder unabhängig Wasserstoff oder eine C₁-C₄-Alkylgruppe ist, die unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, mit der Bedingung, dass wenn R₁ Wasserstoff ist, R₂ von Wasserstoff verschieden ist, und umgekehrt;
R₃ und R₄ jeder unabhängig Wasserstoff oder eine C₁-C₄-Alkylgruppe ist, die unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, oder R₃ und R₄ zusammen =S bilden;
R₅ und R₆ jeder unabhängig Wasserstoff oder eine C₁-C₄-Alkylgruppe ist, die unsubstituiert oder mit 1-3 Substituenten, die jeder unabhängig aus der Gruppe bestehend aus Halogen, Hydroxy, C₁-C₄ Alkoxy-und Amino ausgewählt werden, substituiert ist, oder R₅ und R₆ zusammen =S bilden;
X aus der Gruppe bestehend aus einer direkten Bindung, CO, CS, SO₂ und -N=N- ausgewählt wird;
R₇ ausgewählt wird aus der Gruppe bestehend aus:
(i) Phenyl, das unsubstituiert oder mit 1-3 Substituenten substituiert ist, die jeder unabhängig ausgewählt werden aus der Gruppe bestehend aus (1) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (2) C₁-C₄-Alkoxy, (3) Halogen, (4) Formyl, (5) Carboxyl, (6) C₁-C₄-Acyloxy, (7) C₁-C₄-Alkoxycarbonylamino, (8) Phenyl- oder Naphthyl-oxycarbonylamino, (9) Semicarbazido, (10) Formamido, (11) Thioformamido, (12) Hydroxy, (13) Nitro, (14) Amino, (15). Furyl, (16) Triazolyl, (17) Thienyl, (18) Oxazolyl, (19) Imidazolyl und (20) Triazolon-yl,
(ii) einem 5- oder 6-gliedrigen monocyclischen oder einem 8- bis 10-gliedrigen bicyclischen Heterocyclus mit 1-4 Heteroatomen, die jedes unabhängig aus der Gruppe bestehend aus N, O und S ausgewählt werden, wobei der Heterocyclus unsubstituiert oder mit 1-3 Substituenten ringsubstituiert ist, die jeder unabhängig ausgewählt werden aus der Gruppe bestehend aus (1) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (2) Benzyl, das unsubstituiert oder mit 1-3 Substituenten ausgewählt aus der Gruppe bestehend aus C₁-C₄-Alkyl, CF₃, Halogen und OCF₃, substituiert ist, (3) Halogen, (4) Hydroxy (5) Nitro, (6) Amino, (7) C₁-C₄-Acylamino, (8) Formyl, (9) Formamido, (10) Thioformamido, (11) C₁-C₄-Alkoxycarbonylamino, (12) Phenyl- oder Naphthyl-oxycarbonylamino und (13) Semicarbazido
(iii) NHR₈, wobei R₈ aus der Gruppe ausgewählt wird bestehend aus (1) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (2) Phenyl, das unsubstituiert oder mit 1-3 Substituenten substituiert ist, die jeder unabhängig ausgewählt werden aus der Gruppe bestehend aus (a) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (b) C₁-C₄-Alkoxy, (c) Halogen, (d) Formyl, (e) Carboxyl, (f) C₁-C₄-Acyloxy, (g) C₁-C₄-Alkoxycarbonylamino, (h) Phenyl- oder Naphthyl-oxycarbonylamino, (i) Semicarbazido, (j) Formamido, (k) Thioformamido, (1) Hydroxy, (m) Nitro, (n) Amino, (o) Furyl, (p) Triazolyl, (q) Thienyl, (r) Oxazolyl, (s) Imidazolyl und (t) Triazolon-yl und (3) einem 5- oder 6-gliedrigen monocyclischen oder einem 8- bis 10-gliedrigen bicyclischen Heterocyclus mit 1-3 Heteroatomen, die jedes unabhängig aus der Gruppe bestehend aus N, O und S ausgewählt werden, der unsubstituiert oder mit 1-3 Substituenten substituiert ist, die jeder unabhängig ausgewählt werden aus der Gruppe bestehend aus Hydroxy, Halogen, Amino und Carboxyl,
(iv) OR₉, wobei OR₉ ausgewählt wird aus der Gruppe bestehend aus (1) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (2) Phenyl, das unsubstituiert oder mit 1-3 Substituenten substituiert ist, die jeder unabhängig ausgewählt werden aus der Gruppe bestehend aus (a) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (b) C₁-C₄-Alkoxy, (c) Halogen, (d) Formyl, (e) Carboxyl, (f) C₁-C₄-Acyloxy, (g) C₁-C₄-Alkoxycarbonylamino, (h) Phenyl- oder Naphthyl-oxycarbonylamino, (i) Semicarbazido, (j) Formamido, (k) Thioformamido, (l) Hydroxy, (m) Nitro, (n) Amino, (o) Furyl, (p) Triazolyl, (q) Thienyl, (r) Oxazolyl, (s) Imidazolyl und (t) Triazolon-yl und (3) einem 5- oder 6-gliedrigen monocyclischen oder einem 8- bis 10-gliedrigen bicyclischen Heterocyclus mit 1-3 Heteroatomen, die jedes unabhängig aus der Gruppe bestehend aus N, O und S ausgewählt werden, der unsubstituiert oder mit 1-3 Substituenten substituiert ist, die jeder unabhängig ausgewählt werden aus der Gruppe bestehend aus (a) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (b) Phenyl, das unsubstituiert oder mit 1-3 Substituenten substituiert ist, die jeder unabhängig ausgewählt werden aus der Gruppe bestehend aus (A) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (B) C₁-C₄-Alkoxy, (C) Halogen, (D) Formyl, (E) Carboxyl, (F) C₁-C₄-Acyloxy, (G) C₁-C₄-Alkoxycarbonylamino, (H) Phenyl- oder Naphthyl-oxycarbonylamino, (I) Semicarbazido, (J) Formamido, (K) Thioformamido, (L) Hydroxy, (M) Nitro, (N) Amino, (O) Furyl, (P) Triazolyl, (Q) Thienyl, (R) Oxazolyl, (S) Imidazolyl und (T) Triazolon-yl, (c) Naphthyl, das unsubstituiert oder mit 1-3 Substituenten substituiert ist, die jeder unabhängig ausgewählt werden aus der Gruppe bestehend aus (A) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (B) C₁-C₄-Alkoxy, (C) Halogen, (D) Formyl, (E) Carboxyl, (F) C₁-C₄-Acyloxy, (G) C₁-C₄-Alkoxycarbonylamino, (H) Phenyl- oder Naphthyl-oxycarbonylamino, (I) Semicarbazido, (J) Formamido, (K) Thioformamido, (L) Hydroxy, (M) Nitro, (N) Amino, (O) Furyl, (P) Triazolyl, (Q) Thienyl, (R) Oxazolyl, (S) Imidazolyl und (T) Triazolon-yl, (d) einem 5- oder 6-gliedrigen monocyclischen oder einem 8- bis 10-gliedrigen bicyclischen Heterocyclus mit 1-3 Heteroatomen, die jedes unabhängig aus der Gruppe bestehend aus N, O und S ausgewählt werden, (e) (C₁-C₄-Alkyl)phenyl, (f) (C₁-C₄-Alkyl)naphthyl, (g) Hydroxy, (h) Halogen, (i) Amino und (j) Carboxyl, und
(v) einer Gruppe mit der Formel 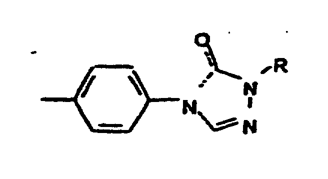 wobei R ausgewählt wird aus der Gruppe bestehend aus (1) Wasserstoff, (2) C₁-C₁₀-Alkyl, das unsubstituiert oder mit 1-5 Substituenten, die jeder unabhängig aus der Gruppe bestehend aus Halogen, Hydroxy, C₁-C₄-Alkoxy und Amino ausgewählt werden, substituiert ist, (3) Phenyl, das unsubstituiert oder mit 1-3 Substituenten substituiert ist, die jeder unabhängig ausgewählt werden aus der Gruppe bestehend aus (a) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (b) C₁-C₄-Alkoxy, (c) Halogen, (d) Formyl, (e) Carboxyl, (f) C₁-C₄-Acyloxy, (g) C₁-C₄-Alkoxycarbonylamino, (h) Phenyl- oder Naphthyl-oxycarbonylamino, (i) Semicarbazido, (j) Formamido, (k) Thioformamido, (l) Hydroxy, (m) Nitro, (n) Amino, (o) Furyl, (p) Triazolyl, (q) Thienyl, (r) Oxazolyl, (s) Imidazolyl, (t) Triazolon-yl, (u) CF₃ und (v) OCF₃, (4) einem 5- oder 6-gliedrigen monocyclischen oder einem 8-bis 10-gliedrigen bicyclischen Heterocyclus mit 1-3 Heteroatomen, die jedes unabhängig aus der Gruppe bestehend aus N, O und S ausgewählt werden, der unsubstituiert oder mit 1-3 Substituenten substituiert ist, die jeder unabhängig ausgewählt werden aus der Gruppe bestehend aus (a) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (b) Phenyl, das unsubstituiert oder mit 1-3 Substituenten substituiert ist, die jeder unabhängig ausgewählt werden aus der Gruppe bestehend aus (A) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (B) C₁-C₄-Alkoxy, (C) Halogen, (D) Formyl, (E) Carboxyl, (F) C₁-C₄ Acyloxy, (G) C₁-C₄-Alkoxycarbonylamino, (H) Phenyl- oder Naphthyl-oxycarbonylamino, (I) Semicarbazido, (J) Formamido, (K) Thioformamido, (L) Hydroxy, (M) Nitro, (N) Amino, (O) Furyl, (P) Triazolyl, (Q) Thienyl, (R) Oxazolyl, (S) Imidazolyl und (T) Triazolon-yl, (c) Naphthyl, das unsubstituiert oder mit 1-3 Substituenten substituiert ist, die jeder unabhängig ausgewählt werden aus der Gruppe bestehend aus (A) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (B) C₁-C₄-Alkoxy, (C) Halogen, (D) Formyl, (E) Carboxyl, (F) C₁-C₄-Acyloxy, (G) C₁-C₄-Alkoxycarbonylamino, (H) Phenyl- oder Naphthyl-oxycarbonylamino, (I) Semicarbazido, (J) Formamido, (K) Thioformamido, (L) Hydroxy, (M) Nitro, (N) Amino, (O) Furyl, (P) Triazolyl, (Q) Thienyl, (R) Oxazolyl, (S) Imidazolyl und (T) Triazolon-yl, (d) einem 5- oder 6-gliedrigen monocyclischen oder einem 8- bis 10-gliedrigen bicyclischen Heterocyclus mit 1-3 Heteroatomen, die jedes unabhängig aus der Gruppe bestehend aus N, O und S ausgewählt werden, (e) (C₁-C₄-Alkyl)phenyl, (f) (C₁-C₄-Alkyl)naphthyl, (g) Hydroxy, (h) Halogen, (i) Amino und (j) Carboxyl, (5) Phenyl(C₁-C₄alkyl), das unsubstituiert oder mit 1-3 Substituenten ringsubstituiert ist, die jeder unabhängig ausgewählt werden aus der Gruppe bestehend aus (a) C₁-C₅-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (b) Halogen, (c) Halogen (C₁-C₄-alkyl), (d) C₁-C₄ Alkoxy, (e) Hydroxy, (f) Amino, (g) Carboxyl, (h) Trifluormethoxyl, (i) Trifluoromethyl, (j) Tetrafluoroethyl, (k) Tetrafluoroethoxyl, (1) Tetrafluoropropyl und (m) Tetrafluoropropoxyl, (6) Naphthyl(C₁-C₄-alkyl), das unsubstituiert oder mit 1-6 Substituenten substituiert ist ausgewählt aus (a) C₁-C₅ Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (b) Halogen, (c) (C₁-C₄-Alkyl) halogen, (d) C₁-C₄-Alkoxy, (e) Hydroxy, (f) Amino, (g) Carboxyl, (h) Trifluormethoxyl, (i) Trifluoromethyl, (j) Tetrafluoroethyl, (k) Tetrafluoroethoxyl, (1) Tetrafluoropropyl und (m) Tetrafluoropropoxyl, (7) Methoxyl, (8) Trifluormethoxyl, (9) Trifluoromethyl, (10) Trifluoroethyl, (11) Tetrafluoroethyl, (12) Tetrafluoroethoxyl, (13) Tetrafluoropropyl und (14) Tetrafluoropropoxyl.

2. Verbindung gemäß Anspruch 1, wobei Ar aus der Gruppe bestehend aus 4-Fluorophenyl, 2,4-Difluorophenyl, 2,4,6-Trifluorophenyl, 4-Chlorophenyl, 2,4-Dichlorophenyl, 2,4,6-Trichlorophenyl, 4-Trifluoromethylphenyl und 4-Trifluoromethoxyphenyl ausgewählt wird.

3. Verbindung gemäß Anspruch 1, wobei Ar eine Phenylgruppe mit 1 bis 2 Substituenten ist, die jeder unabhängig aus der Gruppe bestehend aus Fluor, Chlor, Trifluoromethyl und Trifluoromethoxy ausgewählt werden.

4. Verbindung gemäß Anspruch 1, wobei Ar aus der Gruppe bestehend aus 2,4-Difluorophenyl, 2,4-Dichlorophenyl, 4-Trifluoromethylphenyl und 4-Trifluoromethoxyphenyl ausgewählt wird.

5. Verbindung gemäß Anspruch 1, wobei Ar 2,4-Difluorophenyl ist.

6. Verbindung gemäß Anspruch 1, wobei R₁ ein Alkyl ist.

7. Verbindung gemäß Anspruch 1, wobei R₁ Methyl ist.

8. Verbindung gemäß Anspruch 1, wobei R₂ Wasserstoff ist.

9. Verbindung gemäß Anspruch 1, wobei R₁ und R₂ gleich sind, und die Verbindung eine 2R isomere Konfiguration hat.

10. Verbindung gemäß Anspruch 1, wobei R₁ und R₂ verschieden sind, und die Verbindung eine 2R,3R isomere Konfiguration hat.

11. Verbindung gemäß Anspruch 1, wobei X eine direkte Bindung ist und R₇ eine Gruppe ist mit der Formel

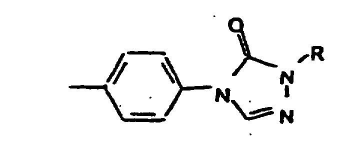

12. Verbindung gemäß Anspruch 11, wobei Ar 2,4-Difluorophenyl ist, R₁ Methyl ist und R₂ bis R₆ alle Wasserstoff sind.

13. Verbindung gemäß Anspruch 1, wobei R₁ Wasserstoff ist und R₂ Methyl ist.

14. Verbindung gemäß Anspruch 1, wobei R₃ bis R₆ alle Wasserstoff sind.

15. Verbindung gemäß Anspruch 1, wobei R₃ und R₅ jeweils Methyl sind und R₄ und R₆ jeweils Wasserstoff sind.

16. Verbindung gemäß Anspruch 1, wobei X eine direkte Bindung ist.

17. Verbindung gemäß Anspruch 1, wobei R₇ eine Gruppe ist mit der Formel 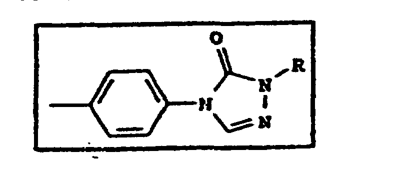 wobei R ausgewählt wird aus der Gruppe bestehend aus 2-Propyl, 2-Butyl, 3-Pentyl, 2-Hydroxypropyl, 4-Trifluoromethylbenzyl, Tetrafluoropropyl, Trifluoroethyl, 2,4-Difluorobenzyl, 4-Methoxybenzyl, 4-Trifluoromethoxybenzyl und 2,4-Bis(trifluoromethyl)benzyl.

18. Verbindung gemäß Anspruch 1, wobei die Verbindung ausgewählt wird aus der Gruppe bestehend aus:
(2R,3R)-2-(2,4-Difluorophenyl)-3-(2,5-dimethylpiperazin-1-yl)-1-(1H-1,2,4-triazol-1-yl)butan-2-ol,
(2R,3R)-2-(2,4-Difluorophenyl)-3-(2,5-dimethyl-4-ethoxycarbonylpiperazin-1-yl)-1-(1H-1,2,4-triazol-1yl)butan-2-ol,
(2R,3R)-3-(Anilinocarbonylpiperazin-1-yl)-2- (2,4-difluorophenyl)-1-(1H-1,2,4-triazol-1-yl)butan-2-ol,
(2R,3R)-2-(2,4-Difluorophenyl)-3-(4-ethylaminocarbonylpiperazin-1-yl)-1-(1H-1,2,4-triazol-1-yl)butan-2-ol,
(2R,3R)-3-(Anilinothiocarbonylpiperazin-1-yl)-2-(2,4-difluorophenyl)-1-(1H-1,2,4-triazol-1-yl)butan-2-ol,
(2R,3R)-2-(2,4-Difluorophenyl)-3-(4-ethylaminothiocarbonylpiperazin-1-yl)-1-(1H-1,2,4-triazol-1-yl)butan-2-ol,
(2R,3R)-3-[4-(2,4-Difluorobenzoyl)piperazin-1-yl]-2-(2,4-difluorophenyl)-1-(1H-1,2,4-triazol-1-yl)butan-2-ol,
(2R,3R)-2-(2,4-Difluorophenyl)-1-(1H-1,2,4-triazoll-yl)-3-[4-(4-trifluoromethylbenzoyl)piperazin-1-yl]butan-2-ol,
(2R,3R)-2-(2,4-Difluorophenyl)-3-[4-(4-nitrobenzoyl)piperazin-1-yl]-1-(1H-1,2,4-triazol-1-yl)butan-2-ol,
(2R,3R)-3-[4-(4-Aminobenzoyl)piperazin-1-yl]-2-(2,4-difluorophenyl)-1-(1H-1,2,4-triazol-1-yl)butan-2-ol,
(2R,3R)-2-(2,4-Difluorophenyl)-3-[4-(*p*toluolsulfonyl)piperazin-1-yl]-1-(1H-1,2,4-triazol-1-yl)butan-2-ol,
(2R,3R)-2-(2,4-Difluorophenyl)-3-[4-(methansulfonyl)piperazin-1-yl]-1-(1H-1,2,4-triazol-1-yl)butan-2-ol,
(2R,3R)-2-(2,4-Difluorophenyl)-3-[4-(4-nitrophenylsulfonyl)piperazin-1-yl]-1-(1H-1,2,4-triazol-1-yl)butan-2-ol,
(2R,3R)-3-[4-(4-Aminophenylsulfonyl)piperazin-1-yl]-2-(2,4-difluorophenyl)-1-(1H-1,2,4-triazol-1-yl)butan-2-ol,
(2R,3R)-2-(2,4-Difluorophenyl)-3-[4-(3-azo-lH-1,2,4-triazol-3-yl)piperazin-1-yl]-1-(1H-1,2,4-triazol-1-yl)butan-2-ol,
(2R,3R)-2-(2,4-Difluorophenyl)-3-[4-(2-thiazolyl)piperazin-1-yl]-1-(1H-1,2,4-triazol-1-yl)butan-2-ol,
(2R,3R)-2-(2,4-Difluorophenyl)-3-[4-(1H-1,2,4-triazol-3-yl)piperazin-1-yl]-1-(1H-1,2,4-triazol-1-yl)butan-2-ol,
(2R,3R)-2-(2,4-Difluorophenyl)-3-[4-(1H-5-tetrazolyl)piperazin-1-yl] -1-(1H-1,2,4-triazol-1-yl)butan-2-ol,
(2R,3R)-2-(2,4-Difluorophenyl)-3-{4-[2-(4-tertbutylbenzyl)-2H-tetrazol-5-yl]piperazin-1-yl}-1-(1H-1,2,4-triazol-1-yl)butan-2-ol,
(2R,3R)-2-(2,4-Difluorophenyl)-1-(1H-1,2,4-triazol-1-yl)-3-{4-[2-(4-trifluoromethylbenzyl)-2H-tetrazol-5-yl]piperazin-1-yl}butan-2-ol,
(2R,3R)-2- (2,4-Difluorophenyl)-3-(4- [2-(4-*tert*butylbenzyl)-2H-1,2,4-triazol-3-yl]piperazin-1-yl}-1-(1H-1,2,4-triazol-1-yl)butan-2-ol,
(2R,3R)-2-(2,4-Difluorophenyl)-3-{4-[1-(4-tertbutylbenzyl)-1H-1,2,4-triazol-3-yl]piperazin-1-yl}-1-(1H-1,2,4-triazol-1-yl)butan-2-ol,
(2R,3R)-2-(2,4-Difluorophenyl)-1-(1H-1,2,4-triazol-1-yl)-3-(4-[1-(4-trifluoromethylbenzyl)-1H-1,2,4-triazol-3-yl]piperazin-1-yl}butan-2-ol,
(2R,3R)-2-(2,4-Difluorophenyl)-3-(4-phenylpiperazin-1-yl)-1-(1H-1,2,4-triazol-1-yl)butan-2-ol,
(2R,3R)-2-(2,4-Difluorophenyl)-3-[4-(4-nitrophenyl)piperazin-1-yl]-1-(1H-1,2,4-triazol-1-yl)butan-2-ol,
(2R, 3R)-3-[4-(4-Aminophenyl)piperazin-1-yl]-2-(2,4-difluorophenyl)-1- (1H-1,2,4-triazol-1-yl)butan-2-ol,
(2R,3R)-2-(2,4-Difluorophenyl)-3-[4-(4-ethoxycarbonylaminophenyl)piperazin-1-yl]-1-(1H-1,2,4-triazol-1-yl)butan-2-ol,
(2R,3R)-2-(2,4-Difluorophenyl)-3-[4-(4-phenoxycarbonylaminophenyl)piperazin-1-yl]-1-(1H-1,2,4-triazol-1-yl)butan-2-ol,
(2R,3R)-2-(2,4-Difluorophenyl)-3-{4-[4-(semicarbazid-4-yl)phenyl]piperazin-1-yl}-1-(1H-1,2,4-triazol-1-yl)butan-2-ol, und
(2R,3R)-2-(2,4-Difluorophenyl)-1-(1H-1,2,4-triazol-1-yl)-3-{4-[4-(2H-1,2,4-triazol-3-on-4-yl)phenyl]piperazin-1-yl}butan-2-ol.

19. Verbindung gemäß Anspruch 1, wobei die Verbindung ausgewählt wird aus der Gruppe bestehend aus:
(2R,3R)-2-(2,4-Difluorophenyl)-3-[4-{4-[2-(3-pentyl}-2H-1,2,4-triazol-3-on-4-yl]phenyl}piperazin-1-yl]-1-(1H-1,2,4-triazol-1-yl)butan-2-ol,
(2S,3R)-2-(2,4-Difluorophenyl)-3-[4-{4-[2-(3-pentyl)-2H-1,2,4-triazol-3-on-4-yl]phenyl}piperazin-1-yl]-1-(1H-1,2,4-triazol-1-yl)butan-2-ol,
(2S,3S)-2-(2,4-Difluorophenyl)-3-[4-{4-[2-(3-pentyl)-2H-1,2,4-triazol-3-on-4-yl]phenyl}piperazin-1-yl]-1-(1H-1,2,4-triazol-1-yl)butan-2-ol,
(2R,3S)-2-(2,4-Difluorophenyl)-3-[4-{4-[2-(3-pentyl)-2H-1,2,4-triazol-3-on-4-yl]phenyl}piperazin-1-yl]-1-(1H-1,2,4-triazol-1-yl)butan-2-ol,
(2R,3R)-3-[4-{4-[2-(2-Butyl)-2H-1,2,4-triazol-3-on-4-yl]phenyl}piperazin-1-yl]-2-(2,4-Difluorophenyl)-1-(1H-1,2,4-triazol-1-yl)butan-2-ol,
(2R,3R)-2-(2,4-Difluorophenyl)-3-[4-{4-[2-(2-propyl)-2H-1,2,4-triazol-3-on-4-yl]phenyl)piperazin-1-yl]-1-(1H-1,2,4-triazol-1-yl)butan-2-ol,
(2R,3R)-2-(2,4-Difluorophenyl)-3-[4-{4-[2-(2-hydroxypropyl)-2H-1,2,4-triazol-3-on-4-yl]phenyl}piperazin-1-yl]-1-(1H-1,2,4-triazol-1-yl)butan-2-ol,
(2R,3R)-2-(2,4-Difluorophenyl)-1-(1H-1,2,4-triazol-1-yl)-3-[4-{4-[2-(4-trifluoromethyl)benzyl-2H-1,2,4-triazol-3-on-4-yl]phenyl}piperazin-1-yl]butan-2-ol,
(2R,3R)-2-(2,4-Difluorophenyl)-3-[4-{4-[2-(2,2,3,3-tetrafluoro)propyl-2H-1,2,4-triazol-3-on-4-yl]phenyl}piperazin-1-yl]-1-(1H-1,2,4-triazol-1-yl)butan-2-ol,
(2R,3R)-2-(2,4-Difluorophenyl)-1-(1H-1,2,4-triazol-1-yl)-3-[4-{4-[2-(2,2,2-trifluoro)ethyl-2H-1,2,4-triazol-3-on-4-yl]phenyl}piperazin-1-yl]butan-2-ol,
(2R,3R)-3-[4-{4-[2-(2,4-Difluorobenzyl)-2H-1,2,4-triazol-3-on-4-yl]phenyl}piperazin-1-yl]-2-(2,4-Difluorophenyl)-1-(1H-1,2,4-triazol-1-yl)butan-2-ol,
(2R,3R)-2-(2,4-Difluorophenyl)-3-[4-{4-[2-(4-trifluoromethoxy)benzyl-2H-1,2,4-triazol-3-on-4-yl]phenyl}piperazin-1-yl]-1-(1H-1,2,4-triazol-1-yl)butan-2-ol,
(2R,3R)-2-(2,4-Difluorophenyl)-3-[4-{4-[2-(4-methoxylbenzyl)-2H-1,2,4-triazol-3-on-4-yl]phenyl}piperazin-1-yl)-1-(1H-1,2,4-triazol-1-yl)butan-2-ol,
(2R,3R)-3-[4-{4-[2-(2,4-Bis-trifluoromethylbenzyl)-2H-1,2,4-triazol-3-on-4-yl]phenyl}piperazin-1-yl]-2-(2,4-difluorophenyl)-1-(1H-1,2,4-triazol-1-yl)butan-2-ol,
(2R,3R)-2-(2,4-Difluorophenyl)-3-[4-{4-[2-((4-(2,2,3,3-tetrafluoropropoxy)benzyl)-2H,-1,2,4-triazol-3-on-4-yl]phenyl}piperazin-1-yl]-1-(1H-1,2,4-triazol-1-yl)butan-2-ol, und
(2R,3R)-2-(2,4-Difluorophenyl)-3-[4-{3-fluoro-4-[2-(4-trifluoromethyl)benzyl-2H-1,2,4-triazol-3-on-4-yl]phenyl}piperazin-1-yl]-1-(1H-1,2,4-triazol-1-yl)butan-2-ol.

20. Verbindung gemäß Anspruch 19, wobei die Verbindung ausgewählt wird aus der Gruppe-bestehend aus:
(2R,3R)-2-(2,4-Difluorophenyl)-1-(1H-1,2,4-triazoll-yl)-3-[4-{4-[2-(4-trifluoromethyl)benzyl-2H-1,2,4-triazol-3-on-4-yl]phenyl}piperazin-1-yl]butan-2-ol,
(2R,3R)-2-(2,4-Difluorophenyl)-3-[4-{4-[2-(4-trifluoromethoxy)benzyl-2H-1,2,4-triazol-3-on-4-yl]phenyl}piperazin-1-yl]-1-(1H-1,2,4-triazol-1-yl)butan-2-ol,
(2R,3R)-2-(2,4-Difluorophenyl)-3-[4-{4-[2-((4-(2,2,3,3-tetrafluoropropoxy)benzyl)-2H-1,2,4-triazol-3-on-4-yl]phenyl}piperazin-1-yl]-1-(1H-1,2,4-triazol-1-yl)butan-2-ol, und
(2R,3R)-2-(2,4-Difluorophenyl)-3-[4-{3-fluoro-4-[2-(4-trifluoromethyl)benzyl-2H-1,2,4-triazol-3-on-4-yl]phenyl}piperazin-1-yl]-1-(1H-1,2,4-triazol-1-yl)butan-2-ol.

21. Verbindung mit der Formel III, 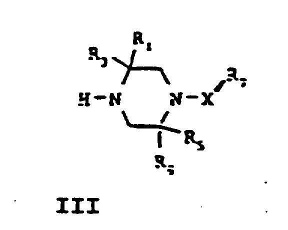 wobei:
R₃ und R₄ jeder unabhängig Wasserstoff oder eine C₁-C₄-Alkylgruppe ist, die unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, oder R₃ und R₄ zusammen =Z bilden, wobei Z Sauerstoff oder Schwefel ist;
R₅ und R₆ jeder unabhängig Wasserstoff oder eine C₁-C₄-Alkylgruppe ist, die unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, oder R₅ und R₆ zusammen =Z bilden, wobei Z Sauerstoff oder Schwefel ist;
X eine direkte Bindung ist;
R₇ ausgewählt wird aus der Gruppe bestehend aus einer Gruppe mit der Formel 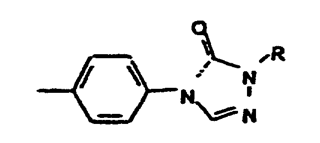 wobei R ausgewählt wird aus der Gruppe bestehend aus (1) Wasserstoff, (2) C₁-C₁₀-Alkyl, das unsubstituiert oder mit 1-5 Substituenten, die jeder unabhängig aus der Gruppe bestehend aus Halogen, Hydroxy, C₁-C₄-Alkoxy und Amino ausgewählt wird, substituiert ist, (3) Phenyl, das unsubstituiert oder mit 1-3 Substituenten substituiert ist, die jeder unabhängig ausgewählt werden aus der Gruppe bestehend aus (a) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (b) C₁-C₄-Alkoxy, (c) Halogen, (d) Formyl, (e) Carboxyl, (f) C₁-C₄-Acyloxy, (g) C₁-C₄-Alkoxycarbonylamino, (h) Phenyl- oder Naphthyl-oxycarbonylamino, (i) Semicarbazido, (j) Formamido, (k) Thioformamido, (1) Hydroxy, (m) Nitro, (n) Amino, (o) Furyl, (p) Triazolyl, (q) Thienyl, (r) Oxazolyl, (s) Imidazolyl, (t) Triazolon-yl, (u) CF₃ und (v) OCF₃, (4) einem 5- oder 6-gliedrigen monocyclischen oder einem 8- bis 10-gliedrigen bicyclischen Heterocyclus mit 1-3 Heteroatomen, die jedes unabhängig aus der Gruppe bestehend aus N, O und S ausgewählt werden, der unsubstituiert oder mit 1-3 Substituenten substituiert ist, die jeder unabhängig ausgewählt werden aus der Gruppe bestehend aus (a) C₁-C₄-Alkyl, das unsubstitutiert oder mit 1-3 Substituenten, die jeder unabhängig aus der Gruppe bestehend aus Halogen, Hydroxy, C₁-C₄-Alkoxy und Amino ausgewählt werden, substituiert ist, (b) Phenyl, das unsubstituiert oder mit 1-3 Substituenten substituiert ist, die jeder unabhängig ausgewählt werden aus der Gruppe bestehend aus (A) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (B) C₁-C₄-Alkoxy, (C) Halogen, (D) Formyl, (E) Carboxyl, (F) C₁-C₄-Acyloxy, (G) C₁-C₄-Alkoxycarbonylamino, (H) Phenyl- oder Naphthyl-oxycarbonylamino, (I) Semicarbazido, (J) Formamido, (K) Thioformamido, (L) Hydroxy, (M) Nitro, (N) Amino, (O) Furyl, (P) Triazolyl, (Q) Thienyl, (R) Oxazolyl, (S) Imidazolyl und (T) Triazolon-yl, (c) Naphthyl, das unsubstituiert oder mit 1-3 Substituenten substituiert ist, die jeder unabhängig ausgewählt werden aus der Gruppe bestehend aus (A) C₁-C₄-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (B) C₁-C₄-Alkoxy, (C) Halogen, (D) Formyl, (E) Carboxyl, (F) C₁-C₄-Acyloxy, (G) C₁-C₄-Alkoxycarbonylamino, (H) Phenyl- oder Naphthyl-oxycarbonylamino, (I) Semicarbazido, (J) Formamido, (K) Thioformamido, (L) Hydroxy, (M) Nitro, (N) Amino, (O) Furyl, (P) Triazolyl, (Q) Thienyl, (R) Oxazolyl, (S) Imidazolyl und (T) Triazolon-yl, (d) einem 5- oder 6-gliedrigen monocyclischen oder einem 8- bis 10-gliedrigen bicyclischen Heterocyclus mit 1-3 Heteroatomen, die jedes unabhängig aus der Gruppe bestehend aus N, O und S ausgewählt werden, (e) (C₁-C₄-Alkyl)phenyl, (f) (C₁-C₄-Alkyl)naphthyl, (g) Hydroxy, (h) Halogen, (i) Amino und (j) Carboxyl, (5) Phenyl(C₁-C₄alkyl), das unsubstituiert oder mit 1-3 Substituenten ringsubstituiert ist, die jeder unabhängig ausgewählt werden aus der Gruppe bestehend aus (a) C₁-C₅-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (b) Halogen, (c) Halogen(C₁-C₄-alkyl), (d) C₁-C₄-Alkoxy, (e) Hydroxy, (f) Amino, (g) Carboxyl, (h) Trifluormethoxyl, (i) Trifluoromethyl, (j) Tetrafluoroethyl, (k) Tetrafluoroethoxyl, (l) Tetrafluoropropyl und (m) Tetrafluoropropoxyl, (6) Naphthyl(C₁-C₄-alkyl), das unsubstituiert oder mit 1-6 Substituenten substituiert ist ausgewählt aus (a) C₁-C₅-Alkyl, das unsubstituiert oder mit 1-3 Substituenten, die jeder unabhängig aus der Gruppe bestehend aus Halogen, Hydroxy, C₁-C₄-Alkoxy und Amino ausgewählt werden, substituiert ist, (b) Halogen, (c) (C₁-C₄-Alkyl)halogen, (d) C₁-C₄-Alkoxy, (e) Hydroxy, (f) Amino, (g) Carboxyl, (h) Trifluormethoxyl, (i) Trifluoromethyl, (j) Tetrafluoroethyl, (k) Tetrafluoroethoxyl, (1) Tetrafluoropropyl und (m) Tetrafluoropropoxyl, (7) Methoxyl, (8) Trifluormethoxyl, (9) Trifluoromethyl, (10) Trifluoroethyl, (11) Tetrafluoroethyl, (12) Tetrafluoroethoxyl, (13) Tetrafluoropropyl und (14) Tetrafluoropropoxyl.

22. Verbindung gemäß Anspruch 21, wobei die Verbindung ausgewählt wird aus der Gruppe bestehend aus:
2-(3-Pentyl)-4-[4-(piperazin-1-yl)phenyl]-2H-1,2,4-triazol-3-on,
2-(2-Butyl)-4-[4-(piperazin-1-yl)phenyl]-2H-1,2,4-triazol-3-on,
4-[4-(Piperazin-1-yl)phenyl]-2-(2-propyl)-2H-1,2,4-triazol-3-on,
2-(2-Hydroxypropyl)-4-[4-(piperazin-1-yl)phenyl]-2H-1,2,4-triazol-3-on,
4-[4-(Piperazin-1-yl)phenyl]-2-[2-(4-trifluoromethyl)benzyl]-2H-1,2,4-triazol-3-on,
4-[4-(Piperazin-1-yl)phenyl]-2-[2-(2,2,3,3-tetrafluoro)propyl]-2H-1,2,4-triazol-3-on,
4-[4-(Piperazin-1-yl)phenyl]-2-[2-(3,3,3-trifluoroethyl)]-2H-1,2,4-triazol-3-on,
2-(2,4-Difluorobenzyl)-4-[4-(piperazin-1-yl)phenyl]-2H-1,2,4-triazol-3-on,
4-[4-(Piperazin-1-yl)phenyl]-2-(4-trifluoromethoxybenzyl)-2H-1,2,4-triazol-3-on,
2-(4-Methoxybenzyl)-4-[4-(piperazin-1-yl)phenyl]-2H-1,2,4-triazol-3-on,
2-(2,4-Bis-trifluoromethylbenzyl)-4-[4-(piperazin-1-yl)phenyl]-2H-1,2,4-triazol-3-on,
4-[4-(Piperazin-1-yl)phenyl]-2-[4-(2,2,3,3-tetrafluoropropoxy)benzyl]-2H-1,2,4-triazol-3-on, und
4-[3-Fluoro-4-(piperazin-1-yl)phenyl]-2-(4-trifluoromethylbenzyl)-2H-1,2,4-triazol-3-on.

23. Zusammensetzung zur Behandlung einer Pilzinfektion umfassend eine pharmazeutisch wirksame Menge einer Verbindung gemäß einem der Ansprüche 1-22 in Kombination mit einem pharmazeutisch akzeptablen Träger oder Verdünnungsmittel.

24. Pharmazeutische Zusammensetzung gemäß Anspruch 23, wobei die pharmazeutische Zusammensetzung eine orale Verabreichungsform ist und die Verbindung in einer Menge von 1 bis 25% (w/w) vorliegt.

25. Pharmazeutische Zusammensetzung gemäß Anspruch 23, wobei die pharmazeutische Zusammensetzung eine injizierbare Verabreichungsform ist und die Verbindung in einer Menge von 0,1 bis 5% (w/w) vorliegt.

26. Pharmazeutische Zusammensetzung gemäß Anspruch 23, wobei die pharmazeutische Zusammensetzung eine topische oder rektale Verabreichungsform ist und die Verbindung in einer Menge von 1 bis 10% (w/w) vorliegt.

27. Verwendung einer Verbindung in Übereinstimmung mit einem der Ansprüche 1-26 für die Herstellung eines Medikaments zur Behandlung oder zum Schutz vor einer Pilzinfektion in einem Patienten, der eine solche Behandlung oder einen solchen Schutz benötigt.

28. Verwendung einer Verbindung gemäß Anspruch 27, wobei die Verbindung so zubereitet ist, um in einer täglichen Dosis von 0,01 bis 20 mg pro kg Patientengewicht verabreicht zu werden.

29. Verwendung einer Verbindung gemäß Anspruch 28, wobei die Verbindung so zubereitet ist, um in einer täglichen Dosis verabreicht zu werden, die in eine Vielzahl individueller Dosen aufgeteilt ist.

30. Verwendung einer Verbindung gemäß einem der Ansprüche 27-29, wobei die Pilzinfektion eine topische Infektion ist.

31. Verwendung einer Verbindung gemäß einem der Ansprüche 27-30, wobei die Pilzinfektion eine systemische Infektion ist.

32. Verwendung einer Verbindung gemäß einem der Ansprüche . 27-31, wobei die Pilzinfektion eine Infektion der Schleimhäute ist.

33. Verwendung einer Verbindung gemäß eine der Ansprüche 27-32, wobei die Pilzinfektion eine die Lunge angreifende Infektion ist.

34. Zusammensetzung gemäß einem der Ansprüche 1-22 zur Verwendung als ein Arzneimittel.

## Revendications

1. Composé de la formule I, ou un isomère optique ou un sel pharmaceutiquement acceptable de celui-ci : 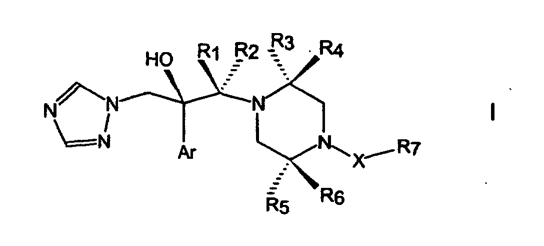 où :
Ar est un radical phényle qui est non substitué ou substitué par 1 à 3 substituants, chacun choisi indépendamment parmi le groupe consistant en halogène, CF₃ et OCF₃ ;
R₁ et R₂ sont chacun indépendamment,. hydrogène ou un radical alkyle en C₁-C₄, qui est non substitué ou substitué par 1 à 3 substituants, chacun choisi indépendamment parmi le groupe consistant en halogène, hydroxy, alcoxy en C₁-C₄ et amino, avec la condition que lorsque R₁ est hydrogène, R₂ est autre que hydrogène et vice versa ;
R₃ et R₄ sont chacun indépendamment, hydrogène ou un groupe alkyle en C₁-C₄, qui est non substitué ou substitué par 1 à 3 substituants, chacun choisi indépendamment parmi le groupe consistant en halogène, hydroxy, alcoxy en C₁-C₄ et amino, ou R₃ et R₄ forment ensemble =S ;
R₅ et R₆ sont chacun indépendamment, hydrogène ou un groupe alkyle en C₁-C₄, qui est non substitué ou substitué par 1 à 3 substituants, chacun choisi indépendamment parmi le groupe consistant en halogène, hydroxy, alcoxy en C₁-C₄ et amino, ou R₅ et R₆ forment ensemble =S ;
X est choisi parmi le groupe consistant en une liaison directe, CO, CS, SO₂ et -N=N- ;
R₇ est choisi parmi le groupe consistant en :
(1) phényle qui est non substitué ou substitué par 1 à 3 substituants, chacun indépendamment choisi parmi le groupe consistant (1) alkyle en C₁-C₄, qui est non substitué ou substitué par 1-3 substituants, chacun indépendamment choisi parmi le groupe consistant en halogène, hydroxy, alcoxy en C₁-C₄ et amino, (2) alkoxy en C₁-C₄, (3) halogène, (4) formyle, (5) carboxyle, (6) acyloxy en C₁-C₄, (7) C₁-C₄ alkoxycarbonylamino; (8) phényl- ou naphtyloxycarbonylamino, (9) semicarbazido, (10) formamido, (11) thioformamido, (12) hydroxy, (13) nitro, (14) amino, (15) furyle, (16) triazolyle, (17) thiényle, (18) oxazolyle, (19) imidazolyle et (20) triazolonyle ;
(ii) hétérocycle monocyclique à 5 ou 6 membres ou un hétérocycle bicyclique à 8 à 10 membres, ledit hétérocycle ayant 1 à 4 hétéroatomes, chacun indépendamment choisi parmi le groupe consistant en N, O et S, ledit hétérocycle étant non substitué ou substitué sur le cycle par 1 à 3 substituants, chacun indépendamment choisi parmi le groupe consistant en (1) alkyle en C₁-C₄, qui est non substitué ou substitué par 1 à 3 substituants, chacun indépendamment choisi parmi le groupe consistant en halogène, hydroxy, alcoxy en C₁-C₄ et amino, (2) benzyle qui est non substitué ou substitué par 1 à 3 substituants, choisi(s) parmi le groupe consistant en alkyle en C₁-C₄, CF₃, halogène et OCF₃, (3) halogène, (4) hydroxy, (5) nitro, (6) amino, (7) C₁-C₄ acylamino, (8) formyle, (9) formamido, (10) thioformamido, (11) C₁-C₄ alkoxycarbonylamino, (12) phényl- ou naphtyl-oxycarbonylamino et (13) semicarbazido ;
(iii) NHR₈ où R₈ est choisi parmi le groupe consistant en (1) alkyle en C₁-C₄, qui est non substitué ou substitué par 1 à 3 substituants, chacun indépendamment choisi parmi le groupe consistant en halogène, hydroxy, alcoxy en C₁-C₄ et amino, (2) phényle qui est non substitué ou substitué par 1 à 3 substituants, chacun indépendamment choisi parmi le groupe consistant en (a) alkyle en C₁-C₄, qui est non substitué ou substitué par 1 à 3 substituants, chacun indépendamment choisi parmi le groupe consistant en halogène, hydroxy, alcoxy en C₁-C₄ et amino, (b) alcoxy en C₁-C₄, (c) halogène, (d) formyle, (e) carboxyle, (f) acyloxy en C₁-C₄, (g) C₁-C₄ alkoxycarbonylamino, (h) phényl- ou naphtyloxycarbonylamino, (i) semicarbazido, (j) formamido, (k) thioformamido, (1) hydroxy, (m) nitro, (n) amino, (o) furyle, (p) triazolyle, (q) thiényle, (r) oxazolyle, (s) imidazolyle et (t) triazolonyle, et (3) hétérocycle monocyclique à 5 ou 6 membres ou bicyclique à 8 à 10 membres, ledit hétérocycle ayant 1 à 3 hétéroatomes, chacun indépendamment choisi parmi le groupe consistant en N, O et S, qui est non substitué ou substitué par 1 à 3 substituants, chacun indépendamment choisi parmi le groupe consistant en hydroxy, halogène, amino et carboxyle ;
(iv) OR₉ où R₉ est choisi parmi le groupe consistant en (1) alkyle en C₁-C₄, qui est non substitué ou substitué par 1 à 3 substituants, chacun indépendamment choisi parmi le groupe consistant en halogène, hydroxy, alcoxy en C₁-C₄ et amino, (2) phényle qui est non substitué ou substitué par 1 à 3 substituants, chacun indépendamment choisi parmi le groupe consistant en (a) alkyle en C₁-C₄, qui est non substitué ou substitué par 1 à 3 substituants, chacun indépendamment choisi parmi le groupe consistant en halogène, hydroxy, alcoxy en C₁-C₄ et amino, (b) alcoxy en C₁-C₄, (c) halogène, (d) formyle, (e) carboxyle, (f) acyloxy en C₁-C₄, (g) C₁-C₄ alkoxycarbonylamino, (h) phényl- ou naphtyloxycarbonylamino, (i) semicarbazido, (j) formamido, (k) thioformamido, (l) hydroxy, (m) nitro, (n) amino, (o) furyle, (p) triazolyle, (q) thiényle, (r) oxazolyle, (s) imidazolyle et (t) triazolonyle, et, (3) hétérocycle monocyclique à 5 ou 6 membres ou bicyclique à 8 à 10 membres, ledit hétérocycle ayant 1 à 3 hétéroatomes., chacun indépendamment choisi parmi le groupe consistant en N, O et S, qui est non substitué ou substitué par 1 à 3 substituants, chacun indépendamment choisi parmi le groupe consistant en (a) alkyle en C₁-C₄, qui est non substitué ou substitué par 1 à 3 substituants, chacun indépendamment choisi parmi le groupe consistant en halogène, hydroxy, alcoxy en C₁-C₄ et amino, (b) phényle qui est non substitué ou substitué par 1 à 3 substituants, chacun indépendamment choisi parmi le groupe consistant en (A) alkyle en C₁-C₄, qui est non substitué ou substitué par 1-3 substituants, chacun indépendamment choisi parmi le groupe consistant en halogène, hydroxy, alcoxy en C₁-C₄ et amino, (B) alcoxy en C₁-C₄, (C) halogène, (D) formyle, (E) carboxyle, (F) acyloxy en C₁-C₄, (G) C₁-C₄ alkoxycarbonylamino, (H) phényl- ou naphtyl-oxycarbonylamino, (I) semicarbazido, (J) formamido, (K) thioformamido, (L) hydroxy, (M) nitro, (N) amino, (O) furyle, (P) triazolyle, (Q) thiényle, (R) oxazolyle, (S) imidazolyle et (T) triazolonyle, (c) naphtyle qui non substitué ou substitué par 1 à 3 substituants, chacun indépendamment choisi parmi le groupe consistant en (A) alkyle en C₁-C₄, qui est non substitué ou substitué par 1 à 3 substituants, chacun indépendamment choisi parmi le groupe consistant en halogène, hydroxy, alcoxy en C₁-C₄ et amino, (B) alcoxy en C₁-C₄, (C) halogène, (D) formyle, (E) carboxyle, (F) acyloxy en C₁-C₄, (G) C₁-C₄ alkoxycarbonylamino, (H) phényl- ou naphtyloxycarbonylamino, (I) semicarbazido, (J) formamido, (K) thioformamido, (L) hydroxy, (M) nitro, (N) amino, (O) furyle, (P) triazolyle, (Q) thiényle, (R) oxazolyle, (S) imidazolyle et (T) triazolonyle, (d) hétérocycle monocyclique à 5 ou 6 membres ou bicyclique à 8 à 10 membres, ledit hétérocycle ayant 1 à 3 hétéroatomes, chacun indépendamment choisi parmi le groupe consistant en N, O et S, (e) (alkyl en C₁-C₄)phényle, (f) (alkyl en C₁-C₄)naphtyle, (g) hydroxy, (h) halogène, (i) amino et (j) carboxyle, et
(v) un groupe de formule : 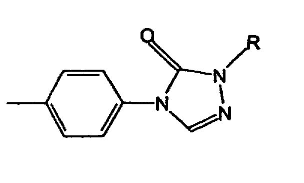 où R est choisi parmi le groupe consistant en (1) hydrogène, (2) alkyle en C₁-C₁₀, qui est non substitué ou substitué par 1 à 5 substituants, chacun indépendamment choisi parmi le groupe consistant en halogène, hydroxy, alcoxy en C₁-C₄ et amino, (3) phényle qui est non substitué ou substitué par 1 à 3 substituants, chacun indépendamment choisi parmi le groupe consistant en (a) alkyle en C₁-C₄, qui est non substitué ou substitué par 1 à 3 substituants, chacun indépendamment choisi parmi le groupe consistant en halogène, hydroxy, alcoxy en C₁-C₄ et amino, (b) alcoxy en C₁-C₄, (c) halogène, (d) formyle, (e) carboxyle, (f) acyloxy en C₁-C₄, (g) C₁-C₄ alkoxycarbonylamino, (h) phényl- ou naphtyloxycarbonylamino, (i) semicarbazido, (j) formamido, (k) thioformamido, (1) hydroxy, (m) nitro, (n) amino, (o) furyle, (p) triazolyle, (q) thiényle, (r) oxazolyle, (s) imidazolyle et (t) triazolonyle, (u) CF₃ et (v) OCF₃, (4) hétérocycle monocyclique à 5 ou 6 membres ou bicyclique à 8 à 10 membres, ledit hétérocyle ayant 1 à 3 hétéroatomes, chacun indépendamment choisi parmi le groupe consistant en N, O et S, et étant non substitué ou substitué par 1 à 3 substituants, chacun indépendamment choisi parmi le groupe consistant en (a) alkyle en C₁-C₄, qui est non substitué ou substitué par 1 à 3 substituants, chacun indépendamment choisi parmi le groupe consistant en halogène, hydroxy, alcoxy en C₁-C₄ et amino, (b) phényle qui est non substitué ou substitué par 1 à 3 substituants, chacun indépendamment choisi parmi le groupe consistant en (A) alkyle en C₁-C₄, qui est non substitué ou substitué par 1 à 3 substituants, chacun indépendamment choisi parmi le groupe consistant en halogène, hydroxy, alcoxy en C₁-C₄ et amino, (B) alcoxy en C₁-C₄, (C) halogène, (D) formyle, (E) carboxyle, (F) acyloxy en C₁-C₄, (G) C₁-C₄ alkoxycarbonylamino, (H) phényl- ou naphtyl-oxycarbonylamino, (I) semicarbazido, (J) formamido, (K) thioformamido, (L) hydroxy, (M) nitro, (N) amino, (O) furyle, (P) triazolyle, (Q) thiényle, (R) oxazolyle, (S) imidazolyle et (T) triazolonyle, (c) naphtyle qui est non substitué ou substitué par 1 à 3 substituants, chacun indépendamment choisi parmi le groupe consistant en (A) alkyle en C₁-C₄, qui est non substitué ou substitué par 1 à 3 substituants, chacun indépendamment choisi parmi le groupe consistant en halogène, hydroxy, alcoxy en C₁-C₄ et amino, (B) alcoxy en C₁-C₄, (C) halogène, (D) formyle, (E) carboxyle, (F) acyloxy en C₁-C₄, (G) C₁-C₄ alkoxycarbonylamino, (H) phényl- ou naphtyloxycarbonylamino, (I) semicarbazido, (J) formamido, (K) thioformamido, (L) hydroxy, (M) nitro, (N) amino, (O) furyle, (P) triazolyle, (Q) thiényle, (R) oxazolyle, (S) imidazolyle et (T) triazolonyle, (d) hétérocycle monocyclique à 5 ou 6 membres ou bicyclique à 8 à 10 membres, ledit hétérocycle ayant 1 à 3 hétéroatomes, chacun indépendamment choisi parmi le groupe consistant en N, O et S, (e) C₁-C₄ alkylphényle, (f) (alkyl en C₁-C₄)naphtyle, (g) hydroxy, (h) halogène, (i) amino et (j) carboxyle, (5) phényl(alkyle en C₁-C₄), qui est non substitué ou substitué sur le cycle par 1 à 3 substituants, chacun indépendamment choisi parmi le groupe consistant en (a) alkyle en C₁-C₄, qui est non substitué ou substitué par 1 à 3 substituants, chacun indépendamment choisi parmi le groupe consistant en halogène, hydroxy, alcoxy en C₁-C₄ et amino, (b) halogène, (c) halo(alkyle en C₁-C₄), (d) alkoxy en C₁-C₄, (e) hydroxy, (f) amino, (g) carboxyle, (h) trifluorométhoxyle, (i) trifluorométhyle, (j) tétrafluoroéthyle, (k) tétrafluoroéthoxyle, (1) tétrafluoropropyle et (m) tétrafluoropropoxyle, (6) naphtyl(alkyle en C₁-C₄), qui peut être substitué par 1 à 6 substituants choisis parmi (a) alkyle en C₁-C₄, qui est non substitué ou substitué par 1 à 3 substituants, chacun indépendamment choisi parmi le groupe consistant en halogène, hydroxy, alcoxy en C₁-C₄ et amino, (b) halogène, (c) halo(alkyle en C₁-C₄), (d) alkoxy en C₁-C₄, (e) hydroxy, (f) amino, (g) carboxyle, (h) trifluorométhoxyle, (i) trifluorométhyle, (j) tétrafluoroéthyle, (k) tétrafluoroéthoxyle, (1) tétrafluoropropyle et (m) tétrafluoropropoxyle, (7) méthoxyle, (8) trifluorométhoxyle, (9) trifluorométhyle, (10) trifluoroéthyle, (11) tétrafluoroéthyle, (12) tétrafluoroéthoxyle, (13) tétrafluoropropyle et (14) tétrafluoropropoxyle.

2. Composé selon la revendication 1, dans lequel Ar est choisi parmi le groupe consistant en 4-fluorophényle, 2,4-difluorophényle, 2,4,6-trifluorophényle, 4-chlorophényle, 2,4-dichlorophényle, 2,4,6-trichlorophényle, 4-trifluorométhylphényle et 4-trifluorométhoxyphényle.

3. Composé selon la revendication 1, dans lequel Ar est un radical phényle ayant 1 à 2 substituants, chacun indépendamment choisi parmi le groupe consistant en fluor, chlore, trifluorométhyle et trifluorométhoxy.

4. Composé selon la revendication 1, dans lequel Ar est choisi parmi le groupe consistant en 2,4-difluorophényle, 2,4-dichlorophényle, 4-trifluorométhylphényle et 4-trifluorométhoxyphényle.

5. Composé selon la revendication 1, dans lequel Ar est 2,4-difluorophényle.

6. Composé selon la revendication 1, dans lequel R₁ est alkyle.

7. Composé selon la revendication 1, dans lequel R₁ est méthyle.

8. Composé selon la revendication 1, dans lequel R₂ est hydrogène.

9. Composé selon la revendication 1, dans lequel R₁ et R₂ sont identiques, et le composé à une configuration isomérique 2R.

10. Composé selon la revendication 1, dans lequel R₁ et R₂ sont différents et le composé à une configuration isomérique 2R, 3R.

11. Composé selon la revendication 1, dans lequel X est une liaison directe et R₇ est un radical de la formule :

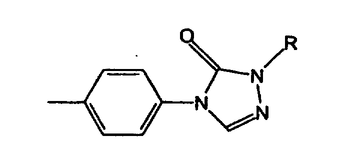

12. Composé selon la revendication 11, dans lequel Ar est 2,4-difluorophényle, R₁ est méthyle, et les groupes R₂ à R₆ sont chacun hydrogène.

13. Composé selon la revendication 1, dans lequel R₁ est hydrogène et R₂ est méthyle.

14. Composé selon la revendication 1, dans lequel les groupes R₃ à R₆ sont chacun hydrogène.

15. Composé selon la revendication 1, dans lequel R₃ et R₅ sont chacun méthyle et R₄ et R₆ sont chacun hydrogène.

16. Composé selon la revendication 1, dans lequel X est une liaison directe.

17. Composé selon la revendication 1, dans lequel R₇ est un radical de la formule : 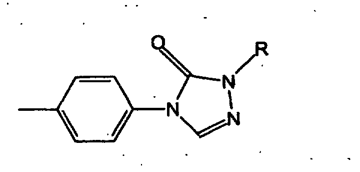 dans lequel R est choisi parmi le groupe consistant en 2-propyle, 2-butyle, 3-pentyle, 2-hydroxypropyle, 4-trifluorométhylbenzyle, tétrafluoropropyle, trifluoroéthyle, 2,4-difluorobenzyle, 4-méthoxybenzyle, 4-trifluorométhoxybenzyle et 2,4-bis(trifluorométhyl)benzyle.

18. Composé selon la revendication 1, dans lequel le composé est choisi parmi le groupe consistant en :
le (2R,3R)-2-(2,4-difluorophényl)-3-(2,5-diméthylpipérazin-1-yl)-1-(1H-1,2,4-triazol-1-yl)butan-2-ol,
le (2R,3R)-2-(2,4-difluorophényl)-3-(2,5-diméthyl-4-éthoxycarbonylpipérazin-1-yl)-1-(1H-1,2,4-triazol-1-yl)butan-2-ol,
le (2R,3R)-3-(anilinocarbonylpipérazin-1-yl)-2-(2,4-difluorophényl)-1-(1H-1,2,4-triazol-1-yl)butan-2-ol,
le (2R,3R)-2-(2,4-difluorophényl)-3-(4-éthylaminocarbonylpipérazin-1-yl)-1-(1H-1,2,4-triazol-1-yl)butan-2-ol,
le (2R,3R)-3-(anilinothiocarbonylpipérazin-1-yl)-2-(2,4-difluorophényl)-1-(1H-1,2,4-triazol-1-yl)butan-2-ol,
le (2R,3R)-2-(2,4-difluorophényl)-3-(4-éthylaminothiocarbonylpipérazin-1-yl)-1-(1H-1,2,4-triazol-1-yl)butan-2-ol,
le (2R,3R)-3-[4-(2,4-difluorobenzoyl)pipérazin-1-yl]-2-(2,4-difluorophényl)-1-(1H-1,2,4-triazol-1-yl)butan-2-ol,
le (2R,3R)-2-(2,4-difluorophényl)-1-(1H-1,2,4-triazol-1-yl) -3-[4- (4-trifluorométhylbenzoyl)pipérazin-1-yl]butan-2-ol,
le (2R,3R)-2-(2,4-difluorophényl)-3-[4-(4-nitrobenzoyl)pipérazin-1-yl]-1-(1H-1,2,4-triazol-1-yl)butan-2-ol,
le (2R,3R)-3-[4-(4-aminobenzoyl)pipérazin-1-yl]-2-(2,4-difluorophényl)-1-(1H-1,2,4-triazol-1-yl)butan-2-ol,
le (2R,3R)-2-(2,4-difluorophényl)-3-[4-(p-toluènesulfonyl)pipérazin-1-yl]-1-(1H-1,2,4-triazol-1-yl)butan-2-ol,
le (2R,3R)-2-(2,4-difluorophényl)-3-[4-(méthanesulfonyl)pipérazin-1-yl]-1-(1H-1,2,4-triazol-1-yl)butan-2-ol,
le (2R,3R)-2-(2,4-difluorophényl)-3-[4- (4-nitrophénylsulfonyl)pipérazin-1-yl]-1-(1H-1,2,4-triazol-1-yl)butan-2-ol,
le (2R,3R) -3-[4-(4-aminophénylsulfonyl)pipérazin-1-yl]-2-(2,4-difluorophényl)-1-(1H-1,2,4-triazol-1-yl)butan-2-ol,
le (2R,3R)-2-(2,4-difluorophényl)-3-[4-(3-azo-1H-1,2,4-triazol-3-yl)pipérazin-1-yl]-1-(1H-1,2,4-triazol-1-yl)butan-2-ol,
le (2R,3R)-2-(2,4-difluorophényl)-3-[4-(2-thiazolyl)pipérazin-1-yl]-1-(1H-1,2,4-triazol-1-yl)butan-2-ol,
le (2R,3R)-2-(2,4-difluorophényl)-3-[4-(1H-1,2,4-triazol-3-yl)pipérazin-1-yl]-1-(1H-1,2,4-triazol-1-yl)butan-2-ol,
le (2R,3R)-2-(2,4-difluorophényl)-3-[4-(1H-5-tétrazolyl)pipérazin-1-yl]-1-(1H-1,2,4-triazol-1-yl)butan-2-ol,
le (2R,3R)-2-(2,4-difluorophényl)-3-[4-[2-(4-t-butylbenzyl)-2H-tétrazol-5-yl]pipérazin-1-yl]-1-(1H-1,2,4-triazol-1-yl)butan-2-ol,
le (2R,3R)-2-(2,4-difluorophényl)-1-(1H-1,2,4-triazol-1-yl) -3-[4-[2- (4-trifluorométhylbenzyl)-2H-tétrazol-5-yl]pipérazin-1-yl]butan-2-ol,
le (2R,3R)-2-(2,4-difluorophényl)-3-[4-[2-(4-t-butylbenzyl)-2H-1,2,4-triazol-3-yl]pipérazin-1-yl]-1-(1H-1,2,4-triazol-1-yl)butan-2-ol,
le (2R,3R)-2- (2,4-difluorophényl) -3-[4-[1- (4-t-butylbenzyl)-1H-1,2,4-triazol-3-yl]pipérazin-1-yl]-1-(1H-1,2,4-triazol-1-yl)butan-2-ol,
le (2R,3R)-2-(2,4-difluorophényl)-1-(1H-1,2,4-triazol-1-yl)-3-[4-[1-(4-trifluorométhylbenzyl)-1H-1,2,4-triazol-3-yl]pipérazin-1-yl]butan-2-ol,
le (2R,3R)-2-(2,4-difluorophényl)-3-(4-phénylpipérazin-1-yl)-1-(1H-1,2,4-triazol-1-yl)butan-2-ol,
le (2R,3R)-2-(2,4-difluorophényl)-3-[4-(4-nitrophényl)- pipérazin-1-yl]-1-(1H-1,2,4-triazol-1-yl)butan-2-ol,
le (2R,3R)-3-[4-(4-aminophényl)pipérazin-1-yl]-2-(2,4-difluorophényl)-1-(1H-1,2,4-triazol-1-yl)butan-2-ol,
le (2R,3R)-2-(2,4-difluorophényl)-3-[4-(4-éthoxycarbonylaminophényl)pipérazin-1-yl]-1-(1H-1,2,4-triazol-1-yl)butan-2-ol,
le (2R,3R)-2-(2,4-difluorophényl)-3-[4-(4-phénoxycarbonylaminophényl)pipérazin-1-yl]-1-(1H-1,2,4-triazol-1-yl)butan-2-ol,
le (2R,3R)-2-(2,4-difluorophényl)-3-[4-[4-(semicarbazide-4-yl)phényl]pipérazin-1-yl]-1-(1H-1,2,4-triazol-1-yl)butan-2-ol, et
le (2R,3R)-2-(2,4-difluorophényl)-1-(1H-1,2,4-triazol-1-yl)-3-[4-[4-(2H-1,2,4-triazol-3-one-4-yl)phényl]pipérazin-1-yl]butan-2-ol.

19. Composé selon la revendication 1, dans lequel le composé est choisi parmi le groupe consistant en :
le (2R,3R)-2-(2,4-difluorophényl)-3-[4-[4-[2-(3-pentyl)-2H-1,2,4-triazol-3-one-4-yl]phényl]pipérazin-1-yl]-1-(1H-1,2,4-triazol-1-yl)butan-2-ol,
le (2S,3R)-2-(2,4-difluorophényl)-3-[4-[4-[2-(3-pentyl)-2H-1,2,4-triazol-3-one-4-yl]phényl]pipérazin-1-yl]-1-(1H-1,2,4-triazol-1-yl)butan-2-ol,
le (2S,3S)-2-(2,4-difluorophényl)-3-[4-[4-[2-(3-pentyl)-2H-1,2,4-triazol-3-one-4-yl]phényl]pipérazin-1-yl]-1-(1H-1,2,4-triazol-1-yl)butan-2-ol,
le (2R,3S)-2-(2,4-difluorophényl)-3-[4-[4-[2-(3-pentyl)-2H-1,2,4-triazol-3-one-4-yl]phényl]pipérazin-1-yl]-1-(1H-1,2,4-triazol-1-yl)butan-2-ol,
le (2R,3R)-3-[4-[4-[2-(2-butyl)-2H-1,2,4-triazol-3-one-4-yl]phényl]pipérazin-1-yl]-2-(2,4-difluorophényl)-1-(1H-1,2,4-triazol-1-yl)butan-2-ol,
le (2R,3R)-2-(2,4-difluorophényl)-3-[4-[4-[2-(2-propyl)-2H-1,2,4-triazol-3-one-4-yl]phényl]pipérazin-1-yl]-1-(1H-1,2,4-triazol-1-yl)butan-2-ol,
le (2R,3R)-2-(2,4-difluorophényl)-3-[4-[4-[2-(2-hydroxypropyl)-2H-1,2,4-triazol-3-one-4-yl]phényl]pipérazin-1-yl]-1-(1H-1,2,4-triazol-1-yl)butan-2-ol,
le (2R,3R)-2-(2,4-difluorophényl)-1-(1H-1,2,4-triazol-1-yl)-3-[4-[4-[2-(4-trifluorométhyl)benzyl-2H-1,2,4-triazol-3-one-4-yl]phényl]pipérazin-1-yl]butan-2-ol,
le (2R,3R)-2-(2,4-difluorophényl)-3-[4-[4-[2-(2,2,3,3-tétrafluoro)propyl-2H-1,2,4-triazol-3-one-4-yl]phényl]pipérazin-1-yl]-1-(1H-1,2,4-triazol-1-yl)butan-2-ol,
le (2R,3R)-2-(2,4-difluorophényl)-1-(1H-1,2,4-triazol-1-yl)-3-[4-[4-[2-(2,2,2-trifluoro)éthyl-2H-1,2,4-triazol-3-one-4-yl]phényl]pipérazin-1-yl]butan-2-ol,
le (2R,3R)-3-[4-[4-[2-(2,4-difluorobenzyl)-2H-1,2,4-triazol-3-one-4-yl]phényl]pipérazin-1-yl]-2- (2,4-difluorophényl)-1-(1H-1,2,4-triazol-1-yl)butan-2-ol,
le (2R,3R)-2-(2,4-difluorophényl)-3-[4-[4-[2-(4-trifluorométhoxy)benzyl-2H-1,2,4-triazol-3-one-4-yl]phényl]pipérazin-1-yl]-1-(1H-1,2,4-triazol-1-yl)butan-2-ol,
le (2R,3R)-2-(2,4-difluorophényl)-3-[4-[4-[2- (4-méthoxybenzyl)-2H-1,2,4-triazol-3-one-4-yl]phényl]pipérazin-l-yl]-1-(1H-1,2,4-triazol-1-yl)butan-2-ol,
le (2R,3R)-3-[4-[4-[2-(2,4-bis-trifluorométhylbenzyl)-2H-1,2,4-triazol-3-one-4-yl]phényl]pipérazin-1-yl]-2-(2,4-difluorophényl)-1-(1H-1,2,4-triazol-1-yl)butan-2-ol,
le (2R,3R)-2-(2,4-difluorophényl)-3-[4-[4-[2-((4-(2,2,3,3-tétrafluoropropoxy)benzyl))-2H-1,2,4-triazol-3-one-4-yl]phényl]pipérazin-1-yl]-1-(1H-1,2,4-triazol-1-yl)-butan-2-ol, et
le (2R,3R)-2-(2,4-difluorophényl)-3-[4-[3-fluoro-4-[2-(4-trifluorométhyl)benzyl-2H-1,2,4-triazol-3-one-4-yl]phényl]pipérazin-1-yl]-1-(1H-1,2,4-triazol-1-yl)butan-2-ol.

20. Composé selon la revendication 19, dans lequel le composé est choisi parmi le groupe consistant en :
le (2R,3R)-2-(2,4-difluorophényl)-1-(1H-1,2,4-triazol-1-yl)-3-[4-[4-[2-(4-trifluorométhyl)benzyl-2H-1,2,4-triazol-3-one-4-yl]phényl]pipérazin-1-yl]butan-2-ol,
le (2R,3R)-2-(2,4-difluorophényl)-3-[4-[4-[2-(4-trifluorométhoxy)benzyl-2H-1,2,4-triazol-3-one-4-yl]phényl]pipérazin-1-yl]-1-(1H-1,2,4-triazol-1-yl)butan-2-ol,
le (2R,3R)-2-(2,4-difluorophényl)-3-[4-[4-[2-((4-(2,2,3,3-tétrafluoropropoxy)benzyl))-2H-1,2,4-triazol-3-one-4-yl]phényl]pipérazin-1-yl]-1-(1H-1,2,4-triazol-1-yl)-butan-2-ol, et
le (2R,3R)-2-(2,4-difluprophényl)-3-[4-[3-fluoro-4-[2-(4-trifluorométhyl)benzyl-2H-1,2,4-triazol-3-one-4-yl]phényl]pipérazin-1-yl]-1-(1H-1,2,4-triazol-1-yl)butan-2-ol.

21. Composé de la formule III : 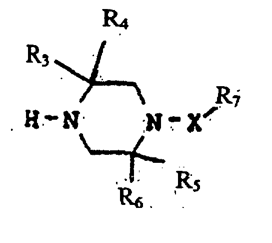 dans laquelle :
R₃ et R₄ sont chacun indépendamment, hydrogène ou un groupe alkyle en C₁-C₄, qui est. non substitué ou substitué par 1 à 3 substituants, chacun choisi indépendamment parmi le groupe consistant en halogène, hydroxy, alcoxy en C₁-C₄ et amino, ou R₃ et R₄ forment ensemble =Z, où Z est oxygène ou soufre ;
R₅ et R₆ sont chacun indépendamment, hydrogène ou un groupe alkyle en C₁-C₄, qui est non substitué ou substitué par 1 à 3 substituants, chacun choisi indépendamment parmi le groupe consistant en halogène, hydroxy, alcoxy en C₁-C₄ et amino, ou R₅ et R₆ forment ensemble =Z, où Z est oxygène ou soufre ;
X est une liaison directe ;
R₇ est choisi parmi le groupe consistant en un groupe de formule : 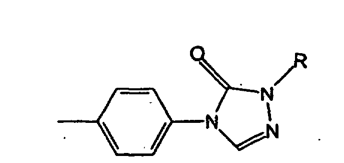
où R est choisi parmi le groupe consistant en (1) hydrogène, (2) alkyle en C₁-C₁₀, qui est non substitué ou substitué par 1 à 5 substituants, chacun indépendamment choisi parmi le groupe consistant en halogène, hydroxy, alcoxy en C₁-C₄ et amino, (3) phényle qui est non substitué ou substitué par 1 à 3 substituants, chacun indépendamment choisi parmi le groupe consistant en (a) alkyle en C₁-C₄, qui est non substitué ou substitué par 1 à 3 substituants, chacun indépendamment choisi parmi le groupe consistant en halogène, hydroxy, alcoxy en C₁-C₄ et amino, (b) alcoxy en C₁-C₄, (c) halogène, (d) formyle, (e) carboxyle, (f) acyloxy en C₁-C₄, (g) C₁-C₄ alkoxycarbonylamino, (h) phényl- ou naphtyl-oxycarbonylamino, (i) semicarbazido, (j) formamido, (k) thioformamido, (1) hydroxy, (m) nitro, (n) amino, (o) furyle, (p) triazolyle, (q) thiényle, (r) oxazolyle, (s) imidazolyle et (t) triazolonyle, (u) CF₃ et (v) OCF₃, (4) hétérocycle monocyclique à 5 ou 6 membres ou bicyclique à 8 à 10 membres, ledit hétérocycle ayant 1 à 3 hétéroatomes, chacun indépendamment choisi parmi le groupe consistant en N, O et S, l'hétérocycle étant non substitué ou substitué par 1 à 3 substituants, chacun indépendamment choisi parmi le groupe consistant en (a) alkyle en C₁-C₄, qui est non substitué ou substitué par 1 à 3 substituants, chacun indépendamment choisi parmi le groupe consistant en halogène, hydroxy, alcoxy en C₁-C₄ et amino, (b) phényle qui est non substitué ou substitué par 1 à 3 substituants, chacun indépendamment choisi parmi le groupe consistant en (A) alkyle en C₁-C₄, qui est non substitué ou substitué par 1 à 3 substituants, chacun indépendamment choisi parmi le groupe consistant en halogène, hydroxy, alcoxy en C₁-C₄ et amino, (B) alcoxy en C₁-C₄, (C) halogène, (D) formyle, (E) carboxyle, (F) acyloxy en C₁-C₄, (G) C₁-C₄ alkoxycarbonylamino, (H) phényl- ou naphtyl-oxycarbonylamino, (I) semicarbazido, (J) formamido, (K) thioformamido, (L) hydroxy, (M) nitro, (N) amino, (O) furyle, (P) triazolyle, (Q) thiényle, (R) oxazolyle, (S) imidazolyle et (T) triazolonyle, (c) naphtyle qui est non substitué ou substitué par 1 à 3 substituants, chacun indépendamment choisi parmi le groupe consistant en (A) alkyle en C₁-C₄, qui est non substitué ou substitué par 1 à 3 substituants, chacun indépendamment choisi parmi le groupe consistant en halogène, hydroxy, alcoxy en C₁-C₄ et amino, (B) alcoxy en C₁-C₄, (C) halogène, (D) formyle, (E) carboxyle, (F) acyloxy en C₁-C₄, (G) (alkoxy en C₁-C₄)carbonylamino, (H) phényl- ou naphtyl-oxycarbonylamino, (I) semicarbazido, (J) formamido, (K) thioformamido, (L) hydroxy, (M) nitro, (N) amino, (O) furyle, (P) triazolyle, (Q) thiényle, (R) oxazolyle, (S) imidazolyle et (T) triazolonyle, (d) un hétérocycle monocyclique à 5 ou 6 membres ou bicyclique à 8 à 10 membres, ledit hétérocycle ayant 1 à 3 hétéroatomes, chacun indépendamment choisi parmi le groupe consistant en N, O et S, (e) (alkyl en C₁-C₄)phényle, (f) (alkyl en C₁-C₄)naphtyle, (g) hydroxy, (h) halogène, (i) amino et (j) carboxyle, (5) phényl(alkyle en C₁-C₄), qui est non substitué ou substitué sur le cycle par 1 à 3 substituants, chacun indépendamment choisi parmi le groupe consistant en (a) alkyle en C₁-C₅, qui est non substitué ou substitué par 1 à 3 substituants, chacun indépendamment choisi parmi le groupe consistant en halogène, hydroxy, alcoxy en C₁-C₄ et amino, (b) halogène, (c) halo(alkyle en C₁-C₄), (d) alkoxy en C₁-C₄, (e) hydroxy, (f) amino, (g) carboxyle, (h) trifluorométhoxyle, (i) trifluorométhyle, (j) tétrafluoroéthyle, (k) tétrafiuoroéthoxyle, (1) tétrafluoropropyle et (m) tétrafluoropropoxyle, (6) naphtyl (alkyle en C₁-C₄), qui peut être substitué par 1 à 6 substituants choisis parmi le groupe consistant en (a) alkyle en C₁-C₅, qui est non substitué ou substitué par 1 à 3 substituants, chacun indépendamment choisi parmi le groupe consistant en halogène, hydroxy, alcoxy en C₁-C₄ et amino, (b) halogène, (c) halo(alkyle en C₁-C₄), (d) alkoxy en C₁-C₄, (e) hydroxy, (f) amino, (g) carboxyle, (h) trifluorométhoxyle, (i) trifluorométhyle, (j) tétrafluoroéthyle, (k) tétrafluoroéthoxyle, (1) tétrafluoropropyle et (m) tétrafluoropropoxyle, (7) méthoxyle, (8) trifluorométhoxyle, (9) trifluorométhyle, (10) trifluoroéthyle, (11) tétrafluoroéthyle, (12) tétrafluoroéthoxyle, (13) tétrafluoropropyle et (14) tétrafluoropropoxyle.

22. Composé selon la revendication 21, dans lequel le composé est choisi parmi le groupe consistant en :
la 2- (3-pentyl) -4-[4- (pipérazin-1-yl)phényl]-2H-1,2,4-triazol-3-one,
la 2- (2-butyl) -4-[4- (pipérazin-1-yl)phényl]-2H-1,2,4-triazol-3-one,
la 4-[4-(pipérazin-1-yl)phényl]-2-(2-propyl) -2H-1,2,4-triazol-3-one,
la 2- (2-hydroxypropyl) -4-[4-(pipérazin-1-yl)phényl]-2H-1,2,4-triazol-3-one,
la 4-[4-(pipérazin-1-yl)phényl]-2-[2- (4-trifluorométhyl)benzyl]-2H-1,2,4-triazol-3-one,
la 4-[4-(pipérazin-1-yl)phényl]-2-[2-(2,2,3,3-tétrafluoro)propyl]-2H-1,2,4-triazol-3-one,
la 4-[4-(pipérazin-1-yl)phényl]-2-[2-(3,3,3-trifluoroéthyl)]-2H-1,2,4-triazol-3-one,
la -2-(2,4-difluorobenzyl)-4-[4-(pipérazin-1-yl)phényl]-2H-1,2,4-triazol-3-one,
la 4-[4-(pipérazin-1-yl)phéhyl]-2-(4-trifluorométhoxybenzyl)-2H-1,2,4-triazol-3-one,
la 2-(4-méthoxybenzyl)-4-[4-(pipérazin-1-yl)phényl]-2H-1,2,4-triazol-3-one,
la 2- (2,4-bis-trifluorométhylbenzyl)-4-[4-(pipérazin-1-yl)phényl]-2H-1,2,4-triazol-3-one,
la 4-[4-(pipérazin-1-yl)phényl]-2-[4-(2,2,3,3-tétrafluoropropoxy)benzyl]-2H-1,2,4-triazol-3-one, et
la 4-[3-fluoro-4-(pipérazin-1-yl)phényl]-2-(4-trifluorométhylbenzyl)-2H-1,2,4-triazol-3-one.

23. Composition pour traiter une infection fongique comprenant une quantité pharmaceutiquement efficace d'un composé selon l'une quelconque des revendications 1 à 22, en combinaison avec un support ou diluant pharmaceutiquement acceptable.

24. Composition pharmaceutique selon la revendication 23, dans laquelle la composition pharmaceutique est une formulation orale et le composé est présent en une quantité allant de 1 à 25% (poids/poids).

25. Composition pharmaceutique selon la revendication 23, dans laquelle la composition pharmaceutique est une formulation injectable et le composé est présent en une quantité allant de 0,1 à 5% (poids/poids).

26. Composition pharmaceutique selon la revendication 23, dans laquelle la composition pharmaceutique est une formulation topique ou rectale et le composé est présent en une quantité allant de 1 à 10% (poids/poids).

27. Utilisation d'un composé selon l'une quelconque des revendications 1 à 26, pour la fabrication d'un médicament pour le traitement ou la prévention d'une infection fongique chez un patient nécessitant un tel traitement ou prévention.

28. Utilisation d'un composé selon la revendication 27, dans laquelle le composé est formulé pour être administré à une dose quotidienne allant de 0,01 à 20 mg/kg de patient.

29. Utilisation d'un composé selon la revendication 28, dans laquelle le composé est formulé pour être administré à une dose quotidienne qui est divisée en une série de doses individuelles.

30. Utilisation d'un composé selon l'une quelconque des revendications 27 à 29, dans laquelle l'infection fongique est une infection topique.

31. Utilisation d'un composé selon l'une quelconque des revendications 27 à 30, dans laquelle, l'infection fongique est une infection systémique.

32. Utilisation d'un composé selon l'une quelconque des revendications 27 à 31, dans laquelle l'infection fongique est une infection des muqueuses.

33. Utilisation d'un composé selon l'une quelconque des revendications 27 à 32, dans laquelle l'infection fongique est une infection invasive des poumons.

34. Composition selon l'une quelconque des revendications 1 à 22, à utiliser comme produit pharmaceutique.
